# EUROPEAN PATENT APPLICATION

(11) **EP 2 053 395 A1**
(43) Date of publication of application: **29.04.2009**
(21) Application number: 07791194.9
(22) Date of filing: 23.07.2007
(51) Int. Cl.: G01N 33/50, G01N 33/15

(54) **METABOLIC SYNDROME EVALUATION METHOD, METABOLIC SYNDROME EVALUATION APPARATUS, METABOLIC SYNDROME EVALUATION METHOD, METABOLIC SYNDROME EVALUATION SYSTEM, METABOLIC SYNDROME EVALUATION PROGRAM AND RECORDING MEDIUM, AND METHOD FOR SEARCH FOR PROPHYLACTIC/AMELIORATING SUBSTANCE FOR METABOLIC SYNDROME**

(30) Priority: 04.08.2006 JP 2006213920
(71) Applicant: Ajinomoto Co., Inc., Tokyo 104-8315 (JP)
(72) Inventor: TAKAHASHI, Mitsuo, Kawasaki-shi Kanagawa 210-8681 (JP); ONO, Nobukazu, Kawasaki-shi Kanagawa 210-8681 (JP); ANDO, Toshihiko, Kawasaki-shi Kanagawa 210-8681 (JP); OKANO, Akira, Kawasaki-shi Kanagawa 210-8681 (JP); YAMAKADO, Minoru, Tokyo 101-8643 (JP)
(74) Representative: Johnson, Richard Alan
(86) International application number: PCT/JP2007/064462
(87) International publication number: WO 2008/015929

(57) **Abstract**

Provided are a method of evaluating metabolic syndrome, a metabolic syndrome-evaluating apparatus, a metabolic syndrome-evaluating method, a metabolic syndrome-evaluating system, a metabolic syndrome-evaluating program, recording medium and the like, which are capable of evaluating the state of Metabolic syndrome accurately by utilizing the concentration of amino acids in blood. According to the method of evaluating metabolic syndrome of the present invention, amino acid concentration data on the concentration value of amino acid in blood collected from a subject to be evaluated is measured, and the state of metabolic syndrome in the subject is evaluated based on the measured amino acid concentration data of the subject.

## Description

### TECHNICAL FIELD

The present invention relates to a method of evaluating metabolic syndrome, a metabolic syndrome-evaluating apparatus, a metabolic syndrome-evaluating method, a metabolic syndrome-evaluating system, a metabolic syndrome-evaluating program and a recording medium, which utilize the concentration of amino acids in blood (plasma).
The present invention also relates to a method of searching for prophylactic/ameliorating substance for metabolic syndrome, wherein a substance for preventing or ameliorating metabolic syndrome is searched.

In recent years, people with a plurality of symptoms resulting from hyperglycemia, hypertension and hyperlipidemia based on obesity and insulin resistance are increasing due to lifestyle under the background of high-fat diet and insufficient exercise, and such symptoms develop arteriosclerosis with age, and eventually people leading to cardiovascular disturbances such as myocardial infarction and cerebrovascular accidents such as cerebral infarction are increasing, thus raising a urgent issue of health insurance at present.

Metabolic syndrome refers to a disorder with high risk of cardiovascular disturbances and cerebrovascular accidents with a plurality of symptoms resulting from hyperglycemia, hypertension and hyperlipidemia. In this disorder, accumulation of visceral fat causes hypertension, diabetes and hyperlipidemia and develops arteriosclerosis, and thus metabolic syndrome is also called visceral fat syndrome. Metabolic syndrome is considered attributable to hyperinsulinemia accompanying visceral fat accumulation and to disruption of the balance of adipocytokines secreted from fat tissues. If there is a lot of visceral fat, the risk of myocardial infarction and cerebral infarction is increased even if hypertension, diabetes and hyperlipidemia are not severe.

In the 102^{nd} general meeting of Japanese Society of Internal Medicine in April, 2005, 8 related societies including Japanese Society of Internal Medicine (8 societies: Japan Society for the Study of Obesity, Japan Atherosclerosis Society, Japan Diabetes Society, Japanese Society of Hypertension, Japanese Circulation Society, Japan Society of Nephrology, The Japanese Society of Thrombosis and Hemostasis, and Japanese Society of Internal Medicine) jointly established diagnostic criteria of metabolic syndrome for Japanese (see Nonpatent Literature 1). The diagnostic criteria of metabolic syndrome are as follows: (1) waist circumference (abdominal circumference) is 85 cm or more for men or 90 cm or more for women, (2) systolic blood pressure is 130 mmHg or more, or diastolic blood pressure is 85 mmHg or more, (3) fasting blood glucose level is not less than 110 mg/dl, and (4) triglyceride is not less than 150 mg/dl, or HDL-cholesterol is less than 40 mg/dL. When a subject meets (1) and 2 or more of (2) to (4), the subject is diagnosed as having metabolic syndrome. When a subject meets (1) and 1 of (2) to (4), the subject is regarded as premetabolic syndrome.

According to the "Summary of Results of National Health and Nutrition Examination Survey" (2004) published by Ministry of Health, Labour and Welfare, Japan, in May, 2006, the number of those who have metabolic syndrome or premetabolic syndrome reaches about 20,000,000 (about 1/3 of the population) at 40 to 74 years of age, wherein the percentage of those who are strongly suspected of having metabolic syndrome is 25.7% for men and 10.0% for women, and the percentage of premetabolic syndrome is 26.0% for men and 9.6% for women. Piecing these results together, those who are strongly suspected of having metabolic syndrome and premetabolic syndrome are at high percentages, that is, 1 of 2 men and 1 of 5 women at 40 to 74 years of age.

As to the diagnostic criteria for metabolic syndrome, there is also a clinical need for establishing more clinically useful diagnostic criteria by reevaluating the existing diagnostic criteria (see Nonpatent Literature 2). For the reasons of necessity for the reevaluation, it is noted that the criteria of WHO, that are the existing and worldwide published diagnostic criteria for metabolic syndrome according to WHO (see Nonpatent Literature 3), and criteria of NCEP (National Cholesterol Education Program) according to NCEP (see Nonpatent Literature 4) are inconsistent in some of criterion items, the present diagnostic criteria in Japan do not sufficiently reflect underlying predisposing factors, cardiovascular disturbance and cerebrovascular accident as risk factors of metabolic syndrome cannot be said to have priority over risk factors of individual diseases (diabetes, hypertension and hyperlipidemia), and when a therapeutic strategy for metabolic syndrome is drawn up, a difference thereof from a therapeutic strategy of individual diseases is unestablished.

From these viewpoints, biomarkers that are more suitable as risk factors reflecting underlying predisposing factors of metabolic syndrome are being searched. The candidate biomarkers studied at present include inflammatory factors such as high-sensitive CRP (C-reactive protein; hs-CRP) (see Nonpatent Literature 5), adipokines that are adipocytic secreted factors such as adiponectin (see Nonpatent Literature 6), resistin (see Nonpatent Literature 7), leptin and a leptin/adiponectin ratio (see Nonpatent Literature 8), and other biochemical factors such as alanine transaminase (ALT) (see Nonpatent Literature 9), albuminuria (see Nonpatent Literature 10), uric acid (see Nonpatent Literature 11) and LDL cholesterol (see Nonpatent Literature 12).

These biomarkers are revealed to vary so as to be correlated with metabolic syndrome, but are not evaluated for their ability to discriminate between non-metabolic syndrome and metabolic syndrome or do not have sufficient discrimination performance. For example, high-sensitive CRP shows statistically significant correlation with diagnostic items for metabolic syndrome, that is, abdominal circumference, HDL cholesterol, a logarithmic value of triglyceride, fasting blood glucose level, systolic blood pressure, and diastolic blood pressure (which are 0.22, - 0.15, 0.12, 0.03, 0.05 and 0.01, respectively, in terms of Spearman partial correlation coefficient) (see Nonpatent Literature 13), but does not have sufficient discrimination performance. Amino acids were known to vary in obesity and diabetes (see Nonpatent Literatures 14 and 15), but at that time there was no concept of metabolic syndrome, and discrimination of metabolic syndrome was not intended.

When metabolic syndrome proceeds and leads to diabetes and hypertension, its treatment is often prolonged, thus leading not only to a reduction in the quality of life of the patient but also to an increase in national cost of medical care as described above. Accordingly, there is a strong demand for establishment of a diagnostic method that can be carried out easily and accurately in a medical examination or the like and a prophylactic and therapeutic method including appropriate health guidance and health care.

It is considered that amino acid metabolism is influenced in peripheral tissues by insulin resistance attributable to accumulation of visceral fat and is related closely to glucose metabolism, lipid metabolism, inflammatory reaction and redox regulatory mechanism that are important for the development process of metabolic syndrome. Accordingly, if an amino acid varying specifically in peripheral blood in metabolic syndrome is found and an index using a concentration parameter of the varying amino acid can be created, the index can be widely applied for an easy and sensitive examination method reflecting an underlying metabolic change in metabolic syndrome. For methods of diagnosing a morbid state by using amino acids in blood, there are indices disclosed in Patent Literatures 1 and 2 (Patent Literature 2 is an undisclosed patent and is thus not a prior art), but the indices in Patent Literature 1 are those directed to discrimination of hepatitis C and non-hepatitis C as the clinically diagnosed subject, and the indices in Patent Literature 2 are those directed to discrimination between healthy subjects and patients with colitis or discrimination between healthy subjects and patients with Crohn's disease.

Nonpatent Literature 1: The Journal of Japanese Society of Internal Medicine, 94, 794, 2005, Metabolic Syndrome Diagnostic Criteria Examination Committee.
Nonpatent Literature 2: Diabetes care, Karn, R. Buse, J. Ferrannini, E. and Stern, M. 28, 2289(2005).
Nonpatent Literature 3: Report of a WHO Consultation. Geneva, World Health Org., 1999.
Nonpatent Literature 4: Executive summary of the Third Report of the National Cholesterol Education Program(NCEP) Expert Panel on Detection, Evaluation, and Treatment of High Blood Cholesterol in Adults (Adult Treatment Panel III), JAMS 285, 2486(2001).
Nonpatent Literature 5: Frohlich, M., Imhof, A., Berg, G. et al., Diabetea Care 23, 1835(2000).
Nonpatent Literature 6: Langenberg, C., Bergstrom, J., et al., Diabetes Care 29, 1363(2006).
Nonpatent Literature 7: MedStar Research Institute, US20060099608.
Nonpatent Literature 8: Mojiminiyi, OA., Abdella, NA., et al., Int. J. Obesity, Jun. 6(2006).
Nonpatent Literature 9: Kazumi, T., Kawaguchi, A., Horm Metab Res. 38, 119-24(2006).
Nonpatent Literature 10: Bonnet, F., Marre, M., et al., J Hypertens. 24,1157 (2006).
Nonpatent Literature 11: Kawamoto R, Tomita H, Oka Y, Ohtsuka N., Intern Med.,45,605(2006).
Nonpatent Literature 12: Gazi, I., Tsimihodimos, V., et al., Metabolism, 55, 885(2006).
Nonpatent Literature 13: Choi, EY., Park, EH., et al., Metabolism, 55, 415(2006).
Nonpatent Literature 14: Felig, P., Marliss, E., et al., New Engl. J. Med. 281,811(1969).
Nonpatent Literature 15: Felig, P., Marliss, E., et al., Diabetes, 19, 727(1979).
Patent Literature 1; WO2004/052191
Patent Literature 2: PCT/JP2006/304398

### DISCLOSURE OF INVENTION

### PROBLEM TO BE SOLVED BY THE INVENTION

However, there is no report on a metabolic pattern of amino acids in peripheral blood in a state of metabolic syndrome, and there is a problem of limit to amino acid change in obesity and diabetes. There is also a problem that there is no report on application to a diagnostic method for discrimination between 2 groups of non-metabolic syndrome and metabolic syndrome.

The present invention is made in view of the problems described above, and an object of the present invention is to provide a method of evaluating metabolic syndrome, a metabolic syndrome-evaluating apparatus, a metabolic syndrome-evaluating methods a metabolic syndrome-evaluating system, a metabolic syndrome-evaluating program, and a recording medium which are capable of evaluating the state of metabolic syndrome with high accuracy by utilizing the concentration of amino acids in blood.
Another object of the present invention is to provide a method of searching for prophylactic/ameliorating substance for metabolic syndrome which is capable of searching a substance for preventing or ameliorating metabolic syndrome efficiently by utilizing the metabolic syndrome-evaluating method described above.

### MEANS FOR SOLVING PROBLEMS

The present inventors have made extensive study for solving the problem described above, and as a result they have identified amino acid variables useful in discrimination between 2 groups of metabolic syndrome and non-metabolic syndrome by their amino acid concentration in blood (amino acid variables varying with a statistically significant difference between the 2 groups), and have found that a correlation equation (index) using the amino acid variables correlates significantly with the progress of a morbid state of metabolic syndrome, and the present invention was thereby completed. The present invention encompasses the following.

To solve the problem and achieve the object described above, a method of evaluating metabolic syndrome according to one aspect of the present invention includes a measuring step of measuring amino acid concentration data on the concentration value of amino acid in blood collected from a subject to be evaluated, and a concentration value criterion evaluating step of evaluating the state of metabolic syndrome in the subject, based on the amino acid concentration data of the subject measured at the measuring step.

Another aspect of the present invention is the method of evaluating metabolic syndrome, wherein the concentration value criterion evaluating step includes evaluating the state of metabolic syndrome in the subject, based on the concentration value of at least one of Val, Leu, Ile, Tyr, Trp, Glu, Ala, Asp, Gly, Ser and Thr contained in the amino acid concentration data of the subject measured at the measuring step.

Still another aspect of the present invention is the method of evaluating metabolic syndrome, wherein the concentration value criterion evaluating step further includes a concentration value criterion discriminating step of discriminating between metabolic syndrome and non-metabolic syndrome in the subject, based on the concentration value of at least one of Val, Leu, Ile, Tyr, Trp, Glu, Ala, Asp, Gly, Ser and Thr contained in the amino acid concentration data of the subject measured at the measuring step.'

Still another aspect of the present invention is the method of evaluating metabolic syndrome, wherein the concentration value criterion discriminating step includes discriminating between metabolic syndrome and non-metabolic syndrome in the subject, based on the concentration values of at least two of Val, Leu, Ile, Tyr, Trp, Glu, Ala, Asp, Gly, Ser and Thr contained in the amino acid concentration data of the subject measured at the measuring step.

Still another aspect of the present invention is the method of evaluating metabolic syndrome, wherein the concentration value criterion evaluating step further includes a discriminant value calculating step of calculating a discriminant value that is a value of multivariate discriminant, based on both the amino acid concentration data of the subject measured at the measuring step and a previously established multivariate discriminant with the concentration of the amino acid as a variable, and a discriminant value criterion evaluating step of evaluating the state of metabolic syndrome in the subject, based on the discriminant value calculated at the discriminant value calculating step.

Still another aspect of the present invention is the method of evaluating metabolic syndrome, wherein the multivariate discriminant contains at least one of Val, Leu, Ile, Tyr, Trp, Glu, Ala, Asp, Gly, Ser and Thr as the variable, and the discriminant value calculating step includes calculating the discriminant value, based on both the concentration value of at least one of Val, Leu, Ile, Tyr, Trp, Glu, Ala, Asp, Gly, Ser and Thr contained in the amino acid concentration data of the subject measured at the measuring step and the multivariate discriminant.

Still another aspect of the present invention is the method of evaluating metabolic syndrome, wherein the discriminant value criterion evaluating step further includes a discriminant value criterion discriminating step of discriminating between metabolic syndrome and non-metabolic syndrome in the subject, based on the discriminant value calculated at the discriminant value calculating step.

Still another aspect of the present invention is the method of evaluating metabolic syndrome, wherein the multivariate discriminant includes at least two of Val, Leu, Ile, Tyr, Trp, Glu, Ala, Asp, Gly, Ser and Thr as the variables, and the discriminant value calculating step includes calculating the discriminant- value, based on both the concentration values of at least two of Val, Leu, Ile, Tyr, Trp, Glu, Ala, Asp, Gly, Ser and Thr contained in the amino acid concentration data of the subject measured at the measuring step and the multivariate discriminant.

Still another aspect of the present invention is the method of evaluating metabolic syndrome, wherein the multivariate discriminant is expressed by one fractional expression or the sum of a plurality of the fractional expressions and contains either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant.

Still another aspect of the present invention is the method of evaluating metabolic syndrome, wherein the multivariate discriminant is formula 1;

Thr-Ser+(Glu+Ala)/Gly (formula 1)

Still another aspect of the present invention is the method of evaluating metabolic syndrome, wherein the multivariate discriminant is any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree.

Still another aspect of the present invention is the method of evaluating metabolic syndrome, wherein the multivariate discriminant contains Glu, Gly, Ala, Thr and Ser as the variables.

The present invention also relates to a metabolic syndrome-evaluating apparatus. One aspect of the present invention is the metabolic syndrome-evaluating apparatus including a control unit and a memory unit to evaluate the state of metabolic syndrome in a subject to be evaluated, wherein the control unit includes a discriminant value-calculating unit that calculates a discriminant value that is a value of multivariate discriminant, based on both previously obtained amino acid concentration data on the concentration value of amino acid in the subject and a multivariate discriminant with the concentration of the amino acid as variable stored in the memory unit, and a discriminant value criterion-evaluating unit that evaluates the state of metabolic syndrome in the subject, based on the discriminant value calculated by the discriminant value-calculating unit.

Another aspect of the present invention is the metabolic syndrome-evaluating apparatus, wherein the multivariate discriminant contains at least one of Val, Leu, Ile, Tyr, Trp, Glu, Ala, Asp, Gly, Ser and Thr as the variable, and the discriminant value-calculating unit calculates the discriminant value, based on both the concentration value of at least one of Val, Leu, Ile, Tyr, Trp, Glu, Ala, Asp, Gly, Ser and Thr contained in previously obtained amino acid concentration data of the subject and the multivariate discriminant.

Still another aspect of the present invention is the metabolic syndrome-evaluating apparatus, wherein the discriminant value criterion-evaluating unit further includes a discriminant value criterion-discriminating unit that discriminates between metabolic syndrome and non-metabolic syndrome in the subject, based on the discriminant value calculated by the discriminant value-calculating unit.

Still another aspect of the present invention is the metabolic syndrome-evaluating apparatus, wherein the multivariate discriminant includes at least two of Val, Leu, Ile, Tyr, Trp, Glu, Ala, Asp, Gly, Ser and Thr as the variables, and the discriminant value-calculating unit calculates the discriminant value, based on both the concentration values of at least two of Val, Leu, Ile, Tyr, Trp, Glu, Ala, Asp, Gly, Ser and Thr contained in the previously obtained amino acid concentration data of the subject and the multivariate discriminant.

Still another aspect of the present invention is the metabolic syndrome-evaluating apparatus, wherein the multivariate discriminant is expressed by one fractional expression or the sum of a plurality of the fractional expressions and contains either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant.

Still another aspect of the present invention is the metabolic syndrome-evaluating apparatus, wherein the multivariate discriminant is formula 1:

Thr/Ser+(Glu+Ala)/Gly (formula 1)

Still another aspect of the present invention is the metabolic syndrome-evaluating apparatus, wherein the multivariate discriminant is any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree.

Still another aspect of the present invention is the metabolic syndrome-evaluating apparatus, wherein the multivariate discriminant contains Glu, Gly, Ala, Thr and Ser as the variables.

Still another aspect of the present invention is the metabolic syndrome-evaluating apparatus, wherein the control unit further includes a multivariate discriminant-preparing unit that prepares the multivariate discriminant to be stored in the memory unit, based on metabolic syndrome state information containing the amino acid concentration data and metabolic syndrome state index data on an index for indicating the state of metabolic syndrome, stored in the memory unit, the multivariate discriminant-preparing unit further includes a candidate multivariate discriminant-preparing unit that prepares a candidate multivariate discriminant that is a candidate of the multivariate discriminant, based on a predetermined discriminant-preparing method from the metabolic syndrome state information, a candidate multivariate discriminant-verifying unit that verifies the candidate multivariate discriminant prepared by the candidate multivariate discriminant-preparing unit, based on a predetermined verifying method, and a variable-selecting unit that selects a variable of the candidate multivariate discriminant based on a predetermined variable-selecting method from the verification result obtained by the candidate multivariate discriminant-verifying unit, thereby selecting a combination of the amino acid concentration data contained in the metabolic syndrome state information used in preparing the candidate multivariate discriminant, and the multivariate discriminant-preparing unit prepares the multivariate discriminant by selecting the candidate multivariate discriminant used as the multivariate discriminant, from a plurality of the candidate multivariate discriminants, based on the verification results accumulated by repeatedly executing the candidate multivariate discriminant-preparing unit, the candidate multivariate discriminant-verifying unit and the variable-selecting unit.

The present invention also relates to a metabolic syndrome-evaluating method. One aspect of the present invention is the metabolic syndrome-evaluating method of evaluating the state of metabolic syndrome in a subject to be evaluated which is carried out with an information processing apparatus having a control unit and a memory unit, wherein the method includes a discriminant value calculating step of calculating a discriminant value that is a value of multivariate discriminant, based on both previously obtained amino acid concentration data on the concentration value of amino acid in the subject and a multivariate discriminant with the concentration of the amino acid as variable stored in the memory unit, and a discriminant value criterion evaluating step of evaluating the state of metabolic syndrome in the subject, based on the discriminant value calculated at the discriminant value calculating step, that are executed by the control unit.

Another aspect of the present invention is the metabolic syndrome-evaluating method, wherein the multivariate discriminant contains at least one of Val, Leu, Ile, Tyr, Trp, Glu, Ala, Asp, Gly, Ser and Thr as the variable, and the discriminant value calculating step includes calculating the discriminant value, based on both the concentration value of at least one of Val, Leu, Ile, Tyr, Trp, Glu, Ala, Asp, Gly, Ser and Thr contained in the previously obtained amino acid concentration data of the subject and the multivariate discriminant.

Still another aspect of the present invention is the metabolic syndrome-evaluating method, wherein the discriminant value criterion evaluating step further includes a discriminant value criterion discriminating step of discriminating between metabolic syndrome and non-metabolic syndrome in the subject, based on the discriminant value calculated at the discriminant value calculating step.

Still another aspect of the present invention is the metabolic syndrome-evaluating method, wherein the multivariate discriminant includes at least two of Val, Leu, Ile, Tyr, Trp, Glu, Ala, Asp, Gly, Ser and Thr as the variables, and the discriminant value calculating step includes calculating the discriminant value, based on both the concentration values of at least two of Val, Leu, Ile, Tyr, Trp, Glu, Ala, Asp, Gly, Ser and Thr contained in the previously obtained amino acid concentration data of the subject and the multivariate discriminant.

Still another aspect of the present invention is the metabolic syndrome-evaluating method, wherein the multivariate discriminant is expressed by one fractional expression or the sum of a plurality of the fractional expressions and contains either at least one of val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant.

Still another aspect of the present invention is the metabolic syndrome-evaluating method, wherein the multivariate discriminant is formula 1:

Thr/Ser+(Glu+Ala)/Gly (formula 1)

Still another aspect of the present invention is the metabolic syndrome-evaluating method, wherein the multivariate discriminant is any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree.

Still another aspect of the present invention is the metabolic syndrome-evaluating method, wherein the multivariate discriminant contains Glu, Gly, Ala, Thr and Ser as the variables.

Still another aspect of the present invention is the metabolic syndrome-evaluating method, wherein the method further includes a multivariate discriminant preparing step of preparing the multivariate discriminant to be stored in the memory unit, based on metabolic syndrome state information containing the amino acid concentration data and metabolic syndrome state index data on an index for indicating the state of metabolic syndrome, stored in the memory unit that is executed by the control unit, the multivariate discriminant preparing step further includes a candidate multivariate discriminant preparing step of preparing a candidate multivariate discriminant that is a candidate of the multivariate discriminant, based on a predetermined discriminant-preparing method from the metabolic syndrome state information, a candidate multivariate discriminant verifying step of verifying the candidate multivariate discriminant prepared at the candidate multivariate discriminant preparing step, based on a predetermined verifying method, and a variable selecting step of selecting variable of the candidate multivariate discriminant based on a predetermined variable-selecting method from the verification result obtained at the candidate multivariate discriminant verifying step, thereby selecting a combination of the amino acid concentration data contained in the metabolic syndrome state information used in preparing the candidate multivariate discriminant, and at the multivariate discriminant preparing step, the multivariate discriminant is prepared by selecting the candidate multivariate discriminant used as the multivariate discriminant, from a plurality of the candidate multivariate discriminants, based on the verification results accumulated by repeatedly executing the candidate multivariate discriminant preparing step, the candidate multivariate discriminant verifying step and the variable selecting step.

The present invention also relates to a metabolic syndrome-evaluating system. One aspect of the present invention is the metabolic syndrome-evaluating system including a metabolic syndrome-evaluating apparatus having a control unit and a memory unit to evaluate the state of metabolic syndrome in a subject to be evaluated and an information communication terminal apparatus that provides amino acid concentration data on the concentration value of amino acid in the subject that are connected to each other communicatively via a network, wherein the information communication terminal apparatus includes an amino acid concentration data-sending unit that transmits the amino acid concentration data of the subject to the metabolic syndrome-evaluating apparatus, and an evaluation result-receiving unit that receives the evaluation result of the state of metabolic syndrome of the subject transmitted from the metabolic syndrome-evaluating apparatus, and the control unit of the metabolic syndrome-evaluating apparatus includes an amino acid concentration data-receiving unit that receives the amino acid concentration data of the subject transmitted from the information communication terminal apparatus, a discriminant value-calculating unit that calculates a discriminant value that is a value of multivariate discriminant, based on both the amino acid concentration data of the subject received by the amino acid concentration data-receiving unit and a multivariate discriminant with the concentration of the amino acid as variable stored in the memory unit, a discriminant value criterion-evaluating unit that evaluates the state of metabolic syndrome in the subject, based on the discriminant value calculated by the discriminant value-calculating unit, and an evaluation result-sending unit that transmits the evaluation result of the subject obtained by the discriminant value criterion-evaluating unit to the information communication terminal apparatus.

Another aspect of the present invention is the metabolic syndrome-evaluating system, wherein the multivariate discriminant contains at least one of Val, Leu, Ile, Tyr, Trp, Glu, Ala, Asp, Gly, Ser and Thr as the variable, and the discriminant value-calculating unit calculates the discriminant value, based on both the concentration value of at least one of Val, Leu, Ile, Tyr, Trp, Glu, Ala, Asp, Gly, Ser and Thr contained in amino acid concentration data of the subject that is received by the amino acid concentration data-receiving unit and the multivariate discriminant.

Still another aspect of the present invention is the metabolic syndrome-evaluating system, wherein the discriminant value criterion-evaluating unit further includes a discriminant value criterion-discriminating unit that discriminates between metabolic syndrome and non-metabolic syndrome in the subject, based on the discriminant value calculated by the discriminant value-calculating unit.

Still another aspect of the present invention is the metabolic syndrome-evaluating system, wherein the multivariate discriminant includes at least two of Val, Leu, Ile, Tyr, Trp, Glu, Ala, Asp, Gly, Ser and Thr as the variables, and the discriminant value-calculating unit calculates the discriminant value, based on both the concentration values of at least two of Val, Leu, Ile, Tyr, Trp, Glu, Ala, Asp, Gly, Ser and Thr contained in the amino acid concentration data of the subject that is received by the amino acid concentration data-receiving unit and the multivariate discriminant.

Still another aspect of the present invention is the metabolic syndrome-evaluating system, wherein the multivariate discriminant is expressed by one fractional expression or the sum of a plurality of the fractional expressions and contains either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant.

Still another aspect of the present invention is the metabolic syndrome-evaluating system, wherein the multivariate discriminant is formula 1:

Thr/Ser+(Glu+Ala)/Gly (formula 1)

Still another aspect of the present invention is the metabolic syndrome-evaluating system, wherein the multivariate discriminant is any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree.

Still another aspect of the present invention is the metabolic syndrome-evaluating system, wherein the multivariate discriminant contains Glu, Gly, Ala, Thr and Ser as the variables.

Still another aspect of the present invention is the metabolic syndrome-evaluating system, wherein the control unit of the metabolic syndrome-evaluating apparatus further includes a multivariate discriminant-preparing unit that prepares the multivariate discriminant to be stored in the memory unit, based on metabolic syndrome state information containing the amino acid concentration data and metabolic syndrome state index data on an index for indicating the state of metabolic syndrome, stored in the memory unit, the multivariate discriminant-preparing unit further includes a candidate multivariate discriminant-preparing unit that prepares a candidate multivariate discriminant that is a candidate of the multivariate discriminant, based on a predetermined discriminant-preparing method from the metabolic syndrome state information, a candidate multivariate discriminant-verifying unit that verifies the candidate multivariate discriminant prepared by the candidate multivariate discriminant-preparing unit, based on a predetermined verifying method, and a variable-selecting unit that selects a variable of the candidate multivariate discriminant based on a predetermined variable-selecting method from the verification result obtained by the candidate multivariate discriminant-verifying unit, thereby selecting a combination of the amino acid concentration data contained in the metabolic syndrome state information used in preparing the candidate multivariate discriminant, and the multivariate discriminant-preparing unit prepares the multivariate discriminant by selecting the candidate multivariate discriminant used as the multivariate discriminant, from a plurality of the candidate multivariate discriminants, based on the verification results accumulated by repeatedly executing the candidate multivariate discriminant-preparing unit, the candidate multivariate discriminant-verifying unit and the variable-selecting unit.

The present invention also relates to a metabolic syndrome-evaluating program. One aspect of the present invention is the metabolic syndrome-evaluating program for evaluating the state of metabolic syndrome in a subject to be evaluated, that makes an information processing apparatus including a control unit and a memory unit execute a discriminant value calculating step of calculating a discriminant value that is a value of multivariate discriminant, based on both previously obtained amino acid concentration data on the concentration value of amino acid in the subject and a multivariate discriminant with the concentration of the amino acid as variable stored in the memory unit, and a discriminant value criterion evaluating step of evaluating the state of metabolic syndrome in the subject, based on the discriminant value calculated at the discriminant value calculating step, that are executed by the control unit.

Another aspect of the present invention is the metabolic syndrome-evaluating program, wherein the multivariate discriminant contains at least one of Val, Leu, Ile, Tyr, Trp, Glu, Ala, Asp, Gly, Ser and Thr as the variable, and the discriminant value calculating step includes calculating the discriminant value, based on both the concentration value of at least one of Val, Leu, Ile, Tyr, Trp, Glu, Ala, Asp, Gly, Ser and Thr contained in the previously obtained amino acid concentration data of the subject and the multivariate discriminant.

Still another aspect of the present invention is the metabolic syndrome-evaluating program, wherein the discriminant value criterion evaluating step further includes a discriminant value criterion discriminating step of discriminating between metabolic syndrome and non-metabolic syndrome in the subject, based on the discriminant value calculated at the discriminant value calculating step.

Still another aspect of the present invention is the metabolic syndrome-evaluating program, wherein the multivariate discriminant includes at least two of Val, Leu, Ile, Tyr, Trp, Glu, Ala, Asp, Gly, Ser and Thr as the variables, and the discriminant value calculating step includes calculating the discriminant value, based on both the concentration values of at least two of Val, Leu, Ile, Tyr, Trp, Glu, Ala, Asp, Gly, Ser and Thr contained in the previously obtained amino acid concentration data of the subject and the multivariate discriminant.

Still another aspect of the present invention is the metabolic syndrome-evaluating program, wherein the multivariate discriminant is expressed by one fractional expression or the sum of a plurality of the fractional expressions and contains either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant.

Still another aspect of the present invention is the metabolic syndrome-evaluating program, wherein the multivariate discriminant is formula 1:

Thr/Ser+(Glu+Ala)/Gly (formula 1)

Still another aspect of the present invention is the metabolic syndrome-evaluating program, wherein the multivariate discriminant is any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree.

Still another aspect of the present invention is the metabolic syndrome-evaluating program, wherein the multivariate discriminant contains Glu, Gly, Ala, Thr and Ser as the variables.

Still another aspect of the present invention is the metabolic syndrome-evaluating program, wherein the control unit further executes a Multivariate discriminant preparing step of preparing the multivariate discriminant to be stored in the memory unit, based on metabolic syndrome state information containing the amino acid concentration data and metabolic syndrome state index data on an index for indicating the state of metabolic syndrome, stored in the memory unit, the Multivariate discriminant preparing step further includes a candidate multivariate discriminant preparing step of preparing a candidate multivariate discriminant that is a candidate of the multivariate discriminant, based on a predetermined discriminant-preparing method from the metabolic syndrome state information, a candidate multivariate discriminant verifying step of verifying the candidate multivariate discriminant prepared at the candidate multivariate discriminant preparing step, based on a predetermined verifying method, and a variable selecting step of selecting variable of the candidate multivariate discriminant based on a predetermined variable-selecting method from the verification result obtained at the candidate Multivariate discriminant verifying step, hereby selecting a combination of the amino acid concentration data contained in the metabolic syndrome state information used in preparing the candidate multivariate discriminant, and at the multivariate discriminant preparing step, the multivariate discriminant is prepared by selecting the candidate multivariate discriminant used as the multivariate discriminant, from a plurality of the candidate multivariate discriminants, based on the verification results accumulated by repeatedly executing the candidate multivariate discriminant preparing step, the candidate multivariate discriminant verifying step and the variable selecting step.

The present invention also relates to a recording medium. One aspect of the present invention is the recording medium that includes the above-described metabolic syndrome-evaluating program recorded thereon.

The present invention also relates to a method of searching for prophylactic/ameliorating substance for metabolic syndrome. One aspect of the present invention is the method of searching for prophylactic/ameliorating substance for metabolic syndrome, wherein the method includes a measuring step of measuring amino acid concentration data on the concentration value of amino acid in blood collected from a subject to be evaluated to which a desired substance group consisting of one or more substances has been administered, a concentration value criterion evaluating step of evaluating the state of metabolic syndrome in the subject, based on the amino acid concentration data measured at the measuring step, and a judging step of judging whether the desired substance group prevents or ameliorates metabolic syndrome, based on the evaluation result at the concentration value criterion evaluating step.

Another aspect of the present invention is the method of searching for prophylactic/ameliorating substance for metabolic syndrome, wherein the concentration value criterion evaluating step includes evaluating the state of metabolic syndrome in the subject, based on the concentration value of at least one of Val, Leu, Ile, Tyr, Trp, Glu, Ala, Asp, Gly, Ser and Thr contained in the amino acid concentration data of the subject measured at the measuring step.

Still another aspect of the present invention is the method of searching for prophylactic/ameliorating substance for metabolic syndrome, wherein the concentration value criterion evaluating step further includes a concentration value criterion discriminating step of discriminating between metabolic syndrome and non-metabolic syndrome in the subject, based on the concentration value of at least one of Val, Leu, Ile, Tyr, Trp, Glu, Ala, Asp, Gly, Ser and Thr contained in the amino acid concentration data of the subject measured at the measuring step.

Still another aspect of the present invention is the method of searching for prophylactic/ameliorating substance for metabolic syndrome, wherein the concentration value criterion discriminating step includes discriminating between metabolic syndrome and non-metabolic syndrome in the subject, based on the concentration values of at least two of Val, Leu, Ile, Tyr, Trp, Glu, Ala, Asp, Gly, Ser and Thr contained in the amino acid concentration data of the subject measured at the measuring step.

Still another aspect of the present invention is the method of searching for prophylactic/ameliorating substance for metabolic syndrome, wherein the concentration value criterion evaluating step further includes a discriminant value calculating step of calculating a discriminant value that is a value of multivariate discriminant, based on both the amino acid concentration data of the subject measured at the measuring step and a previously established multivariate discriminant with the concentration of the amino acid as a variable, and a discriminant value criterion evaluating step of evaluating the state of metabolic syndrome in the subject, based on the discriminant value calculated at the discriminant value calculating step.

Still another aspect of the present invention is the method of searching for prophylactic/ameliorating substance for metabolic syndrome, wherein the multivariate discriminant contains at least one of Val, Leu, Ile, Tyr, Trp, Glu, Ala, Asp, Gly, Ser and Thr as the variable, and the discriminant value calculating step includes calculating the discriminant value, based on both the concentration value of at least one of Val, Leu, Ile, Tyr, Trp, Glu, Ala, Asp, Gly, Ser and Thr contained in the amino acid concentration data of the subject measured at the measuring step and the multivariate discriminant.

Still another aspect of the present invention is the method of searching for prophylactic/ameliorating substance for metabolic syndrome, wherein the discriminant value criterion evaluating step further includes a discriminant value criterion discriminating step of discriminating between metabolic syndrome and non-metabolic syndrome in the subject, based on the discriminant value calculated at the discriminant value calculating step.

Still another aspect of the present invention is the method of searching for prophylactic/ameliorating substance for metabolic syndrome, wherein the multivariate discriminant includes at least two of Val, Leu, Ile, Tyr, Trp, Glu, Ala, Asp, Gly, Ser and Thr as the variables, and the discriminant value calculating step includes calculating the discriminant value, based on both the concentration values of at least two of Val, Leu, Ile, Tyr, Trp, Glu, Ala, Asp, Gly, Ser and Thr contained in the amino acid concentration data of the subject measured at the measuring step and the multivariate discriminant.

Still another aspect of the present invention is the method of searching for prophylactic/ameliorating substance for metabolic syndrome, wherein the multivariate discriminant is expressed by one fractional expression or the sum of a plurality of the fractional expressions and contains either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant.

Still another aspect of the present invention is the method of searching for prophylactic/ameliorating substance for metabolic syndrome, wherein the multivariate discriminant is formula 1:

Thr/Ser+(Glu+Ala)/Gly (formula 1)

Still another aspect of the present invention is the method of searching for prophylactic/ameliorating substance for metabolic syndrome, wherein the multivariate discriminant is any one of a logistic regression equation, a linear discriminant, a multiple regression equation, a formula prepared by a support vector machine, a formula prepared by a Mahalanobis' generalized distance methods a formula prepared by canonical discriminant analysis, and a formula prepared by a decision tree.

Still another aspect of the present invention is the method of searching for prophylactic/ameliorating substance for metabolic syndrome, wherein the multivariate discriminant contains Glu, Gly, Ala, Thr and Ser as the variables.

### EFFECTS OF THE INVENTION

According to the method of evaluating metabolic syndrome of the present invention, amino acid concentration data on the concentration value of amino acid in blood collected from a subject to be evaluated is measured, and the state of metabolic syndrome in the subject is evaluated based on the amino acid concentration data of the subject. Thus, the concentrations of the amino acids in blood can be utilized to bring about an effect of enabling accurate evaluation of the state of metabolic syndrome.

According to the method of evaluating metabolic syndrome of the present invention, the state of metabolic syndrome in the subject is evaluated based on the concentration value of at least one of Val, Leu, Ile, Tyr, Trp, Glu, Ala, Asp, Gly Ser and Thr contained in the amino acid concentration data of the subject. Thus, the concentrations of the amino acids which among amino acids in blood, are related to the state of metabolic syndrome can be utilized to bring about an effect of enabling accurate evaluation of the state of metabolic syndrome.

According to the method of evaluating metabolic syndrome of the present invention, the subject is discriminated between metabolic syndrome and non-metabolic syndrome based on the concentration value of at least one of Val, Leu, Ile, Tyr, Trp, Glu, Ala, Asp, Gly, Ser and Thr contained in the amino acid concentration data of the subject. Thus, the concentrations of the amino acids which among amino acids in blood, are useful for discriminating between the 2 groups of metabolic syndrome and non-metabolic syndrome can be utilized to bring about an effect of enabling accurate discrimination between the 2 groups of metabolic syndrome and non-metabolic syndrome.

According to the method of evaluating metabolic syndrome of the present invention, the subject is discriminated between metabolic syndrome and non-metabolic syndrome based on the concentration values of at least two of Val, Leu, Ile, Tyr, Trp, Glu, Ala, Asp, Gly, Ser and Thr contained in the amino acid concentration data of the subject. Thus, the concentrations of the amino acids which among amino acids in blood, are useful for discriminating between the 2 groups of metabolic syndrome and non-metabolic syndrome can be utilized to bring about an effect of enabling accurate discrimination between the 2 groups of metabolic syndrome and non-metabolic syndrome.

According to the method of evaluating metabolic syndrome of the present invention, a discriminant value that is a value of multivariate discriminant is calculated based on both the amino acid concentration data of the subject measured and a previously established multivariate discriminant with the concentration of the amino acid as a variable, and the state of metabolic syndrome in the subject is evaluated based on the discriminant value calculated. Thus, a discriminant value obtained in a multivariate discriminant wherein the concentrations of amino acids are variables can be utilized to bring about an effect of enabling accurate evaluation of the state of metabolic syndrome.

According to the method of evaluating metabolic syndrome of the present invention, the discriminant value is calculated based on both the concentration value of at least one of Val, Leu, Ile, Tyr, Trp, Glu, Ala, Asp, Gly, Ser and Thr contained in the amino acid concentration data of the subject measured and the multivariate discriminant containing at least one of Val, Leu, Ile, Tyr, Trp, Glu, Ala, Asp, Gly, Ser and Thr as the variable. Thus, a discriminant value obtained in a Multivariate discriminant correlated significantly with the state of metabolic syndrome can be utilized to bring about an effect of enabling accurate evaluation of the state of metabolic syndrome.

According to the method of evaluating metabolic syndrome of the present invention, the subject is discriminated between metabolic syndrome and noun-metabolic syndrome based on the discriminant value calculated. Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

According to the method of evaluating metabolic syndrome of the present invention, the discriminant value is calculated based on both the concentration values of at least two of Val, Leu, Ile, Tyr, Trp, Glu, Ala, Asp, Gly, Ser and Thr contained in the amino acid concentration data of the subject measured and the multivariate discriminant containing at least two of Val, Leu, Ile, Tyr, Trp, Glu, Ala, Asp, Gly, Ser and Thr as the variables. Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

According to the method of evaluating metabolic syndrome of the present invention, the multivariate discriminant is expressed by one fractional expression or the sum of a plurality of the fractional expressions and contains either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

According to the method of evaluating metabolic syndrome of the present invention, the multivariate discriminant is formula 1. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

Thr/Ser+(Glu+Ala)/Gly (formula 1)

According to the method of evaluating metabolic syndrome of the present invention, the multivariate discriminant is any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

According to the method of evaluating metabolic syndrome of the present invention, the multivariate discriminant contains Glu, Gly, Ala, Thr and Ser as the variables. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

According to the metabolic syndrome-evaluating apparatus, the metabolic syndrome-evaluating method, and the metabolic syndrome-evaluating program, a discriminant value that is a value of multivariate discriminant is calculated based on both previously obtained amino acid concentration data on the concentration value of amino acid in the subject and a multivariate discriminant with the concentration of the amino acid as variable stored in the memory unit, and the state of metabolic syndrome is evaluated in the subject based on the discriminant value calculated. Thus, a discriminant value obtained in a multivariate discriminant wherein the concentrations of amino acids are variables can be utilized to bring about an effect of enabling accurate evaluation of the state of metabolic syndrome.

According to the metabolic syndrome-evaluating apparatus, the metabolic syndrome-evaluating method, and the metabolic syndrome-evaluating program, the discriminant value is calculated based on both the concentration value of at least one of Val, Leu, Ile, Tyr, Trp, Glu, Ala, Asp, Gly, Ser and Thr contained in previously obtained amino acid concentration data of the subject and the multivariate discriminant containing at least one of Val, Leu, Ile, Tyr, Trp, Glu, Ala, Asp, Gly, Ser and Thr as the variable. Thus, a discriminant value obtained in a multivariate discriminant correlated significantly with the state of metabolic syndrome can be utilized to bring about an effect of enabling accurate evaluation of the state of metabolic syndrome.

According to the metabolic syndrome-evaluating apparatus, the metabolic syndrome-evaluating method, and the metabolic syndrome-evaluating program, the subject is discriminated between metabolic syndrome and non-metabolic syndrome based on the discriminant value calculated. Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

According to the metabolic syndrome-evaluating apparatus, the metabolic syndrome-evaluating method, and the metabolic syndrome-evaluating program, the discriminant value is calculated based on both the concentration values of at least two of Val, Leu, Ile, Tyr, Trp, Glu, Ala, Asp, Gly, Ser and Thr contained in the previously obtained amino acid concentration data of the subject and the multivariate discriminant containing at least two of Val, Leu, Ile, Tyr, Trp, Glu, Ala, Asp, Gly, Ser and Thr as the variables. Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

According to the metabolic syndrome-evaluating apparatus, the metabolic syndrome-evaluating method, and the metabolic syndrome-evaluating program, the multivariate discriminant is expressed by one fractional expression or the sum of a plurality of the fractional expressions and contains either at least one of Val, Leu, Ile, Tyt, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

According to the metabolic ayndrome-evaluating apparatus, the metabolic syndrome-evaluating method, and the metabolic syndrome-evaluating program, the multivariate discriminant is formula 1. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

Thr/Ser+(Glu+Ala)/Gly (formula 1)

According to the metabolic syndrome-evaluating apparatus, the metabolic syndrome-evaluating method, and the metabolic syndrome-evaluating program, the multivariate discriminant is any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

According to the metabolic syndrome-evaluating apparatus, the metabolic syndrome-evaluating method, and the metabolic syndrome-evaluating program, the multivariate discriminant contains Glu, Gly, Ala, Thr and Ser as the variables. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

According to the metabolic syndrome-evaluating apparatus, the metabolic syndrome-evaluating method, and the metabolic syndrome-evaluating program, the multivariate discriminant to be stored in the memory unit is prepared based on metabolic syndrome state information containing the amino acid concentration data and metabolic syndrome state index data on an index for indicating the state of metabolic syndrome, stored in the memory unit. Specially, (1) a candidate multivariate discriminant that is a candidate of the multivariate discriminant is prepared based on a predetermined discriminant-preparing method from the metabolic syndrome state information, (2) the candidate multivariate discriminant prepared is verified based on a predetermined verifying method, (3) a variable of the candidate multivariate discriminant is selected based on a predetermined variable-selecting method from the verification result in (2), thereby selecting a combination of the amino acid concentration data contained in the metabolic syndrome state information used in preparing the candidate multivariate discriminant, and (4) a candidate multivariate discriminant used as the multivariate discriminant is selected from a plurality of the candidate multivariate discriminants based on the verification results accumulated by repeatedly executing (1), (2) and (3) to prepare the multivariate discriminant. There can thereby be brought about an effect of enabling preparation of the multivariate discriminant most appropriate for evaluation of the state of metabolic syndrome (specifically a multivariate discriminant correlating significantly with the state of metabolic syndrome (more specifically, a multivariate discriminant useful for discrimination of the 2 groups of metabolic syndrome and non-metabolic syndrome).

According to the metabolic syndrome-evaluating system of the present invention, the information communication terminal apparatus first transmits the amino acid concentration data of the subject to the metabolic syndrome-evaluating apparatus. The metabolic syndrome-evaluating apparatus receives the amino acid concentration data of the subject transmitted from the information communication terminal apparatus, calculates a discriminant value that is a value of multivariate discriminant, based on both the amino acid concentration data of the subject received and a multivariate discriminant with the concentration of the amino acid as variable stored in the memory unit, evaluates the state of metabolic syndrome in the subject, based on the discriminant value calculated, and transmits the evaluation result of the subject to the information communication terminal apparatus. The information communication terminal apparatus receives the evaluation result of the state of metabolic syndrome of the subject transmitted from the metabolic syndrome-evaluating apparatus. Thus, a discriminant value obtained in a multivariate discriminant wherein the concentrations of amino acids are variables can be utilized to bring about an effect of enabling accurate evaluation of the state of metabolic syndrome.

According to the metabolic syndrome-evaluating system of the present invention, the discriminant value is calculated based on both the concentration value of at least one of Val, Leu, Ile, Tyr, Trp, Glu, Ala, Asp, Gly, Ser and Thr contained in amino acid concentration data of the subject received and the multivariate discriminant containing at least one of Val, Leu, Ile, Tyr, Trp, Glu, Ala, Asp, Gly, Ser and Thr as the variable. Thus, a discriminant value obtained in a multivariate discriminant correlated significantly with the state of metabolic syndrome can be utilized to bring about an effect of enabling accurate evaluation of the state of metabolic syndrome.

According to the metabolic syndrome-evaluating system of the present invention, the subject is discriminated between metabolic syndrome and non-metabolic syndrome based on the discriminant value calculated. Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

According to the metabolic syndrome-evaluating system of the present invention, the discriminant value is calculated based on both the concentration values of at least two of Val, Leu, Ile, Tyr, Trp, Glu, Ala, Asp, Gly, Ser and Thr contained in the previously obtained amino acid concentration data of the subject and the multivariate discriminant containing at least two of Val, Leu, Ile, Tyr, Trp, Glu, Ala, Asp, Gly, Ser and Thr as the variables. Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

According to the metabolic syndrome-evaluating system of the present invention, the multivariate discriminant is expressed by one fractional expression or the sum of a plurality of the fractional expressions and contains either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyre Trp, Glu, Ala, Asp and Thr as the variable in the denominator, in the fractional expression constituting the multivariate discriminant. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

According to the metabolic syndrome-evaluating system of the present invention, the multivariate discriminant is formula 1. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

Thr/Ser+(Glu+Ala)/Gly (formula 1)

According to the metabolic syndrome-evaluating system of the present invention, the multivariate discriminant is any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

According to the metabolic syndrome-evaluating system of the present invention, the multivariate discriminant contains Glu, Gly, Ala, Thr'and Ser as the variables. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

According to the metabolic syndrome-evaluating system of the present invention, the multivariate discriminant to be stored in the memory unit is prepared based on metabolic syndrome state information containing the amino acid concentration data and metabolic syndrome state index data on an index for indicating the state of metabolic syndrome, stored in the memory unit. Specially, (1) a candidate multivariate discriminant that is a candidate of the multivariate discriminant is prepared based on a predetermined discriminant-preparing method from the metabolic syndrome state information, (2) the candidate multivariate discriminant prepared is verified based on a predetermined verifying method, (3) a variable of the candidate multivariate discriminant is selected based on a predetermined variable-selecting method from the verification result in (2), thereby selecting a combination of the amino acid concentration data contained in the metabolic syndrome state information used in preparing the candidate multivariate discriminant, and (4) a candidate multivariate discriminant used as the multivariate discriminant is selected from a plurality of the candidate multivariate discriminants based on the verification results accumulated by repeatedly executing (1), (2) and (3) to prepare the multivariate discriminant. There can thereby be brought about an effect of enabling preparation of the multivariate discriminant most appropriate for evaluation of the state of metabolic syndrome (specifically a multivariate discriminant correlating significantly with the state of metabolic syndrome (more specifically, a multivariate discriminant useful for discrimination of the 2 groups of metabolic syndrome and non-metabolic syndrome).

According to the recording medium of the present invention, the metabolic syndrome-evaluating program recorded on the recording medium is read and executed by the computer, thereby allowing the computer to execute the metabolic syndrome-evaluating program, thus bringing about an effect of obtaining the same effect as in the metabolic syndrome-evaluating program.

According to the method of searching for prophylactic/ameliorating substance for metabolic syndrome of the present invention, blood collected from a subject to which a desired substance group has been administered is used to measure amino acid concentration data on the concentration values of amino acids, and the state of metabolic syndrome in the subject is evaluated based on the measured amino acid concentration data, and it is judged whether the desired substance group prevents or ameliorates metabolic syndrome, based on the evaluation results. Accordingly, the metabolic syndrome evaluation method capable of accurately evaluating a state of metabolic syndrome by utilizing the concentrations of amino acids in blood can be used to bring about an effect of enabling accurate search for a substance for preventing or ameliorating metabolic syndrome. By the method of searching for prophylactic/ameliorating substance for metabolic syndrome according to the present invention, information on amino acid concentration variation pattern typical of metabolic syndrome or a multivariate discriminant corresponding to metabolic syndrome can be used for selecting a clinically effective chemical at an early stage or in an existing animal model partially reflecting the state of metabolic syndrome.

According to the method of searching for prophylactic/ameliorating substance for metabolic syndrome of the present invention, the state of metabolic syndrome in the subject is evaluated based on the concentration value of at least one of Val, Leu, Ile, Tyr, Trp, Glu, Ala, Asp, Gly, Ser and Thr contained in the amino acid concentration data of the subject. Thus, the concentrations of the amino acids which among amino acids in blood, are related to the state of metabolic syndrome can be utilized to bring about an effect of enabling accurate evaluation of the state of metabolic syndrome.

According to the method of searching for prophylactic/ameliorating substance for metabolic syndrome of the present invention, the subject is discriminated between metabolic syndrome and non-metabolic syndrome based on the concentration value of at least one of Val, Leu, Ile, Tyr, Trp, Glu, Ala, Asp, Gly, Ser and Thr contained in the amino acid concentration data of the subject. Thus, the concentrations of the amino acids which among amino acids in blood, are useful for discriminating between the 2 groups of metabolic syndrome and non-metabolic syndrome can be utilized to bring about an effect of enabling accurate discrimination between the 2 groups of metabolic syndrome and non-metabolic syndrome.

According to the method of searching for prophylactic/ameliorating substance for metabolic syndrome of the present invention, the subject is discriminated between metabolic syndrome and non-metabolic syndrome based on the concentration values of at least two of Val, Leu, Ile, Tyr, Trp, Glu, Ala, Asp, Gly, Ser and Thr contained in the'amino acid concentration data of the subject. Thus, the concentrations of the amino acids which among amino acids in blood, are useful for discriminating between the 2 groups of metabolic syndrome and non-metabolic syndrome can be utilized to bring about an effect of enabling accurate discrimination between the 2 groups of metabolic syndrome and non-metabolic syndrome.

According to the method of searching for prophylactic/ameliorating substance for metabolic syndrome of the present invention, a discriminant value that is a value of multivariate discriminant is calculated based on both the amino acid concentration data of the subject measured and a previously established multivariate discriminant with the concentration of the amino acid as a variable, and the state of metabolic syndrome in the subject is evaluated based on the discriminant value calculated. Thus, a discriminant value obtained in a multivariate discriminant wherein the concentrations of amino acids are variables can be utilized to bring about an effect of enabling accurate evaluation of the state of metabolic syndrome.

According to the method of searching for prophylactic/ameliorating substance for metabolic syndrome of the present invention, the discriminant value is calculated based on both the concentration value of at least one of Val, Leu, Ile, Tyr, Trp, Glu, Ala, Asp, Gly, Ser and Thr contained in the amino acid concentration data of the subject measured and the multivariate discriminant containing at least one of Val, Leu, Ile, Tyr, Trp, Glu, Ala, Asp, Gly, Ser and Thr as the variable. Thus, a discriminant value obtained in a multivariate discriminant correlated significantly with the state of metabolic syndrome can be utilized to bring about an effect of enabling accurate evaluation of the state of metabolic syndrome.

According to the method of searching for prophylactic/ameliorating substance for metabolic syndrome of the present invention, the subject is discriminated between metabolic syndrome and non-metabolic syndrome based on the discriminant value calculated. Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

According to the method of searching for prophylactic/ameliorating substance for metabolic syndrome of the present invention, the discriminant value is calculated based on both the concentration values of at least two of Val, Leu, Ile, Tyr, Trp, Glu, Ala, Asp, Gly, Ser and Thr contained in the amino acid concentration data of the subject measured and the multivariate discriminant containing at least two of Val, Leu, Ile, Tyr, Trp, Glu, Ala, Asp, Gly, Ser and Thr as the variables. Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

According to the method of searching for prophylactic/ameliorating substance for metabolic syndrome of the present invention, the multivariate discriminant is expressed by one fractional expression or the sum of a plurality of the fractional expressions and contains either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

According to the method of searching for prophylactic/ameliorating substance for metabolic syndrome of the present invention, the multivariate discriminant is formula 1. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

Thr/Ser+(Glu+Ala)/Gly (formula 1)

According to the method of searching for prophylactic/ameliorating substance for metabolic syndrome of the present invention, the multivariate discriminant is any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

According to the method of searching for prophylactic/ameliorating substance for metabolic syndrome of the present invention, the multivariate discriminant contains Glu, Gly, Ala, Thr and Ser as the variables. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

When the state of metabolic syndrome is evaluated (specifically discrimination between metabolic syndrome and non-metabolic syndrome is conducted) in the present invention, the concentrations of other metabolites (biological metabolites), the protein expression level, the age and sex of the subject, biological indices or the like may be used in addition to the amino acid concentration. When the state of metabolic syndrome is evaluated (specifically discrimination between metabolic syndrome and non-metabolic syndrome is conducted) in the present invention, the concentrations of other metabolites (biological metabolites), the protein expression level, the age and sex of the subject,biological indices or the like may be used as variables in the multivariate discriminant in addition to the amino acid concentration. As described above, metabolic syndrome includes symptoms of hyperglycemia, hypertension and hyperlipidemia based on insulin resistance, and thus the present invention is also effective in evaluation or discrimination thereof.

### BRIEF DESCRIPTION OF THE DRAWING

FIG. 1 is a principle configurational diagram showing the basic principle of the present invention;
FIG. 2 is a flowchart showing one example of the method of evaluating metabolic syndrome according to the first embodiment;
FIG. 3 is a principle configurational diagram showing the basic principle of the present invention;
FIG. 4 is a diagram showing an example of the entire configuration of the present system;
FIG. 5 is a diagram showing another example of the entire configuration of the present system;
FIG. 6 is a block diagram showing an example of the configuration of the metabolic syndrome-evaluating apparatus 100 in the present system;
FIG. 7 is'a chart showing an example of the information stored in the user information file 106a;
FIG. 8 is a chart showing an example of the information stored in the amino acid concentration data file 106b;
FIG. 9 is a chart showing an example of the information stored in the metabolic syndrome state information file 106c;
FIG. 10 is a chart showing an example of the information stored in the designated metabolic syndrome state information file 106d;
FIG. 11 is a chart showing an example of the information stored in the candidate multivariable discriminant file 106e1;
FIG. 12 is a chart showing an example of the information stored in the verification result file 106e2;
FIG. 13 is a chart showing an example of the information stored in the selected metabolic syndrome state information file 106e3;
FIG. 14 is a chart showing an example of the information stored in the multivariable discriminant file 106e4;
FIG. 15 is a chart showing an example of the information stored in the discriminant value file 106f;
FIG. 16 is a chart showing an example of the information stored in the evaluation result file 106g;
FIG. 17 is a block diagram showing the configuration of the multivariable discriminant-preparing part 102h;
FIG. 18 is a block diagram showing the configuration of the discriminant criterion-evaluating part 102j;
FIG. 19 is a block diagram showing an example of the configuration of the client apparatus 200 in the present system;
FIG. 20 is a block diagram showing an example of the configuration of the database apparatus 400 in the present system;
FIG. 21 is a flowchart showing an example of the metabolic syndrome evaluation service processing performed in the present system;
FIG. 22 is a flowchart showing an example of the multivariate discriminant-preparing processing performed in the metabolic syndrome-evaluating apparatus 100 in the present system;
FIG. 23 is a principle configurational diagram showing the basic principle of the present invention;
FIG. 24 is a flowchart showing one example of the method of searching for prophylactic/ameliorating substance for metabolic syndrome according to the third embodiment;
FIG. 25 is a boxplot showing the distribution of amino acid variables between 2 groups of non-metabolic syndrome and metabolic syndrome;
FIG. 26 is a graph showing an ROC curve for evaluation of diagnostic performance between 2 groups;
FIG. 27 is a graph showing an ROC curve for evaluation of diagnostic performance between 2 groups;
FIG. 28 is a chart showing the cutoff value, sensitivity, specificity, positive predictive value, negative predictive value, and efficiency in discrimination of 2 groups;
FIG. 29 is a chart showing a list of AUCs of ROC curves for evaluation of diagnostic performance between 2 groups;
FIG. 30 is a chart showing a list of AUCs of ROC curves for evaluation of diagnostic performance between 2 groups;
FIG. 31 is a chart showing a list of AUCs of ROC curves for evaluation of diagnostic performance between 2 groups;
FIG. 32 is a chart showing a list of AUCs of ROC curves for evaluation of diagnostic performance between 2 groups;
FIG.33 is a chart showing a concrete example of indices according to each rank in FIG. 31;
FIG.34 is a chart showing a concrete example of indices according to each rank in FIG. 32;
FIG. 35 is a chart showing a list of error rates for evaluation of diagnostic performance between 2 groups;
FIG. 36 is a chart showing a list of error rates for evaluation of diagnostic performance between 2 groups;
FIG.37 is a chart showing a concrete example of indices according to each rank in FIG. 35; and
FIG.38 is a chart showing a concrete example of indices according to each rank in FIG. 36.

### EXPLANATION OF LETTERS OR NUMERALS

- 100: Metabolic syndtome-evaluating apparatus
- 102: Control device
102a Request-interpreting part
102b Browsing processing part
102c Authentication-processing part
102d Electronic mail-generating part
102e Web page-generating part
102f Receiving part
102g Metabolic syndrome state information-designating part
102h Multivariate discriminant-preparing part
102h1 Candidate multivariate discriminant-preparing part
102h2 Candidate multivariate discriminant-verifying part
102h3 Variable-selecting part
102i Discriminant value-calculating part
102j Discriminant value criterion-evaluating part
102j1 Discriminant value criterion- discriminating part
102k Result outputting part
102m Sending part
- 104: Communication interface
- 106: Memory device 106a User information file 106b Amino acid concentration data file 106c Metabolic syndrome state information file 106d Designated metabolic syndrome state information file
106e Multivariate discriminant-related information database
106e1 Candidate multivariate discriminant file
106e2 Verification result file
106e3 Selected metabolic syndrome state information file
106e4 Multivariate discriminant file
106f Discriminant value file
106g Evaluation result file
- 108: Input/output interface
- 112: Input device
- 114: Output device
- 200: Client apparatus (information communication terminal apparatus)
- 300: Network
- 400: Database apparatus

### BEST MODE(S) FOR CARRYING OUT THE INVENTION

Hereinafter, an embodiment (first embodiment) of the method of evaluating metabolic syndrome of the present invention, an embodiment (second embodiment) of the metabolic syndrome-evaluating apparatus, the metabolic syndrome-evaluating method, the metabolic syndrome-evaluating system, the metabolic syndrome-evaluating program and the recording medium of the present invention, and an embodiment (third embodiment) of the method of searching for prophylactic/ameliorating substance for metabolic syndrome of the present invention are described in detail with reference to the drawings. The present invention is not limited to these embodiments.

### First Embodiment

### 1-1. Outline of the Invention

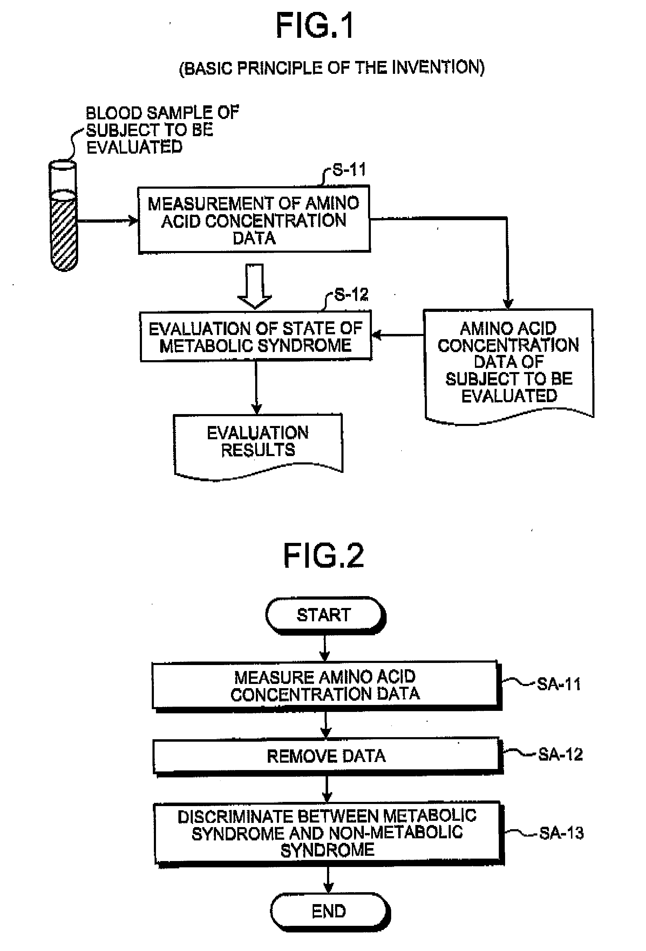

Here, an outline of the method of evaluating metabolic syndrome of the present invention will be described with reference to FIG. 1. FIG. 1 is a principle configurational diagram showing the basic principle of the present invention.

In the present invention, the amino acid concentration data on concentration values of amino acids in blood collected from a subject (for example, an individual such as animal or human) to be evaluated are first measured (step S-11). The concentrations of amino acids in blood were analyzed in the following manner. A blood sample is collected in a heparin-treated tube, and then the blood plasma is separated by centrifugation of the collected blood sample. All blood plasma samples separated were frozen and stored at -70°C before measurement of amino acid concentration. Before measurement of amino acid concentration, the blood plasma sample was deproteinized by adding sulfosalicylic acid to a concentration of 3%. An amino acid analyzer by high-performance liquid chromatography (HPLC) by using ninhydrin reaction in the post column was used for measurement of amino acid concentration. The unit of amino acid concentration is for example molar concentration or weight concentration, which may be subjected to addition, subtraction, multiplication and division by an arbitrary constant.

In the present invention, the state of metabolic syndrome in a subject to be evaluated is evaluated based on the amino acid concentration data of the subject to be evaluated measured in the step S-11 (step S-12).

According to the present invention described above, amino acid concentration data on the concentration value of amino acid in blood collected from a subject to be evaluated is measured, and the state of metabolic syndrome in the subject is evaluated based on the measured amino acid concentration data of the subject. Thus, the concentrations of the amino acids in blood can be utilized to bring about an effect of enabling accurate evaluation of the state of metabolic syndrome.

Before step S-12 is executed, data containing defective and outliers may be removed from the amino acid concentration data of the subject to be evaluated measured in step S-11. Thereby, the state of metabolic syndrome can be more accurately evaluated.

In step S-12, the state of metabolic syndrome in the subject may be evaluated based on the concentration value of at least one of Val, Leu, Ile, Tyr, Trp, Glu, Ala, Asp, Gly, Ser and Thr contained in the amino acid concentration data of the subject measured in step S-11. Thus, the concentrations of the amino acids which among amino acids in blood, are related to the state of metabolic syndrome can be utilized to bring about an effect of enabling accurate evaluation of the state of metabolic syndrome.

In step S-12, the subject may be discriminated between metabolic syndrome and non-metabolic syndrome based on the concentration value of at least one of Val, Leu, Ile, Tyr, Trp, Glu, Ala, Asp, Gly, Ser and Thr contained in the amino acid concentration data of the subject measured in step S-11. Specifically, at least one concentration value of Val, Leu, Ile, Tyr, Trp, Glu, Ala, Asp, Gly, Ser and Thr may be compared with a previously established threshold (cutoff value), thereby discriminating between metabolic syndrome and non-metabolic syndrome in the subject. Thus, the concentrations of the amino acids which among amino acids in blood, are useful for discriminating between the 2 groups of metabolic syndrome and non-metabolic syndrome can be utilized to bring about an effect of enabling accurate discrimination between the 2 groups of metabolic syndrome and non-metabolic syndrome.

In step S-12, the subject may be discriminated between metabolic syndrome and non-metabolic syndrome based on the concentration values of at least two of Val, Leu, Ile, Tyr, Trp, Glu, Ala, Asp, Gly, Ser and Thr contained in the amino acid concentration data of the subject measured in step S-11. Specifically, at least two of Val, Leu, Ile, Tyr, Trp, Glu, Ala, Asp, Gly, Ser and Thr may be compared with a previously established threshold (cutoff value), thereby discriminating between metabolic syndrome and non-metabolic syndrome in the subject. Thus, the concentrations of the amino acids which among amino acids in blood, are useful for discriminating between the 2 groups of metabolic syndrome and non-metabolic syndrome can be utilized to bring about an effect of enabling accurate discrimination between the 2 groups of metabolic syndrome and non-metabolic syndrome.

In step S-12, a discriminant value that is a value of multivariate discriminant may be calculated based on both the amino acid concentration data of the subject measured in step S-11 and a previously established multivariate discriminant with the concentration of the amino acid as a variable, and the state of metabolic syndrome in the subject may be evaluated based on the discriminant value calculated. Thus, a discriminant value obtained in a multivariate discriminant wherein the concentrations of amino acids are variables can be utilized to bring about an effect of enabling accurate evaluation of the state of metabolic syndrome.

In step S-12, the discriminant value may be calculated based on both the concentration value of at least one of Val, Leu, Ile, Tyr, Trp, Glu, Ala, Asp, Gly, Ser and Thr contained in the amino acid concentration data of the subject measured in step S-11 and the multivariate discriminant containing at least one of Val, Leu, Ile, Tyr, Trp, Glu, Ala, Asp, Gly, Ser and Thr as the variable. Thus, a discriminant value obtained in a multivariate discriminant correlated significantly with the state of metabolic syndrome can be utilized to bring about an effect of enabling accurate evaluation of the state of metabolic syndrome.

In step S-12, the subject may be discriminated between metabolic syndrome and non-metabolic syndrome based on the discriminant value calculated. Specifically, the discriminant value may be compared with a previously established threshold (cutoff value), thereby discriminating between metabolic syndrome and non-metabolic syndrome in the subject. Thus, a discriminant value obtained in a Multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

In step S-12, the discriminant value may be calculated based on both the concentration values of at least two of Val, Leu, Ile, Tyr, Trp, Glu, Ala, Asp, Gly, Ser and Thr contained in the amino acid concentration data of the subject measured in step S-11 and the multivariate discriminant containing at least two of Val, Leu, Ile, Tyr, Trp, Glu, Ala, Asp, Gly, Ser and Thr as the variables. Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

The multivariate discriminant may be expressed by one fractional expression or the sum of a plurality of the fractional expressions and may contain either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant. Specially, the multivariate discriminant may be formula 1. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

Thr/Ser+(Glu+Ala)/Gly (formula 1)

The multivariate discriminant may be any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree. Specially, the multivariate discriminant may contain Glu, Gly, Ala, Thr and Ser as the variables. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

The multivariate discriminants described above can be prepared by a method described in International Publication WO 2004/052191 that is an international application filed by the present applicant or by a method (multivariate discriminant-preparing processing described in the second embodiment described later) described in International Publication PCT/JP2006/304398 that is an international application filed by the present applicant. Any multivariate discriminants obtained by these methods can be preferably used in evaluation of the state of metabolic syndrome, regardless of the unit of amino acid concentration in the amino acid concentration data as input data.

In a fractional expression, the numerator of the fractional expression is expressed by the sum of amino acids A, B, C etc. and/or the denominator of the fractional expression is expressed by the sum of amino acids a, b, c etc.. The fractional expression also includes the sum of fractional expressions α, β, γ etc. (for example, α+β) having such constitution. The fractional expression also includes divided fractional expressions, Amino acids used in the numerator or denominator may have suitable coefficients respectively. The amino acids used in the numerator or denominator may appear repeatedly. Each fractional expression may have a suitable coefficient. The value of a coefficient for each variable and the value for a constant term may be any real numbers.

Specifically, the fractional expression has a combination of amino acid concentrations in Groups A to D below, and is preferably "represented by one or more fractional expressions containing 2 to 8 amino acid concentration variables, wherein the numerator has (1 or more variables from Groups A and B + 0 to 2 variables from Group D) and the denominator has (1 or more variables from Group C + 0 to 2 variables from Group D), more preferably "represented by 2 or more fractional expressions containing 4 to 8 amino acid concentration variables, wherein the numerator has (1 or more variables from Group B + 0 to 1 variable from Group A + 0 to 2 variables from Group D) and the denominator has (1 or more variables from Group C + 0 to 2 variables from Group D). In a combination where variables in the numerator and variables in the denominator in the fractional expression are switched with each other, the positive (or negative) sign is generally reversed in correlation with objective variables, but because their correlation is maintained, such combination can be assumed to be equivalent to the original, and thus the fractional expression may include such combination.
Group A: Leu, Ile, Val
Group B: Ala, Glu, Asp, Tyr, Trp, Thr
Group C: Gly, Ser
Group D: amino acids (or amino acid metabolites thereof) not contained in Groups A, B and C

The multivariate discriminant refers to a form of equation used generally in multivariate analysis and includes, for example, multiple regression equation, multiple logistic regression equation, linear discriminant function, Mahalanobis' generalized distance, canonical discriminant function, support vector machine, and decision tree. The multivariate-discriminant also includes an equation shown by the sum of different forms of multivariate discriminants. In the multiple regression equation, multiple logistic regression equation and canonical discriminant function, a coefficient and constant term are added to each variable, and the coefficient and constant term in this case are preferably real numbers, more preferably values in the range of 99% confidence interval for the coefficient and constant term obtained from data for discrimination, more preferably in the range of 95% confidence interval for the coefficient and constant term obtained from data for discrimination. The value of each coefficient and the confidence interval thereof may be those multiplied by a real number, and the value of each constant term and the confidence interval thereof may be those having an arbitrary actual constant added or subtracted or those multiplied or divided by an arbitrary actual constant.

When the state of metabolic syndrome is evaluated (specifically discrimination between metabolic syndrome and non-metabolic syndrome) in the present invention, the concentrations of other metabolites (biological metabolites), the protein expression level, the age and sex of the subject, biological indices or the like may be used in addition to the amino acid concentration data. When the state of metabolic syndrome is evaluated (specifically discrimination between metabolic syndrome and non-metabolic syndrome) in the present invention, the concentrations of other metabolites (biological metabolites), the protein expression level, the age and sex of the subject, biological indices or the like may be used as variables in the multivariate discriminant in addition to the amino acid concentration.

### 1-2. Method of evaluating metabolic syndrome in accordance with the first embodiment

Herein, the method of evaluating metabolic syndrome according to the first embodiment is described with reference to FIG. 2. FIG. 2 is a flowchart showing one example of the method of evaluating metabolic syndrome according to the first embodiment.

From blood collected from an individuals such as animal or human, amino acid concentration data on the concentration values of amino acids are measured (step SA-11). Measurement of the concentration values of amino acids is conducted by the method described above.

From the amino acid concentration data measured in step SA-11, data containing defective and outliers are then removed (step SA-12).

Then, at least one concentration value of Val, Leu, Ile, Tyr, Trp, Glu, Ala, Asp, Gly, Ser and Thr contained in the amino acid concentration data of the individual from which defective and outliers have been removed in step SA-12 is compared with a previously established threshold (cutoff value), thereby discriminating between metabolic syndrome and non-metabolic syndrome in the individual (step SA-13).

### 1-3. Summary of the first embodiment and other embodiments

In the method of evaluating metabolic syndrome as described above in detail, (1) amino acid concentration data are measured from blood collected from an individual, (2) data containing defective and outliers are removed from the measured amino acid concentration data of the individual, and (3) at least one concentration value of Val, Leu, Ile, Tyr, Trp, Glu, Ala, Asp, Gly, Ser and Thr contained in the amino acid concentration data of the individual from which defective and outliers have been removed is compared with a previously established threshold (cutoff value), thereby discriminating between metabolic syndrome and non-metabolic syndrome in the individual. Thus, the concentrations of the amino acids which among amino acids in blood, are useful for discriminating between the 2 groups of metabolic syndrome and non-metabolic syndrome can be utilized to bring about an effect of enabling accurate discrimination between the 2 groups of metabolic syndrome and non-metabolic syndrome.

In step SA-13, discrimination between metabolic syndrome and non-metabolic syndrome in the individual may be conducted based on at least two concentration values of Val, Leu, Ile, Tyr, Trp, Glu, Ala, Asp, Gly, Ser and Thr contained in the amino acid concentration data of the individual from which the data containing defective and outliers was removed in step SA-12. Thus, the concentrations of the amino acids which among amino acids in blood, are useful for discriminating between the 2 groups of metabolic syndrome and non-metabolic syndrome can be utilized to bring about an effect of enabling accurate discrimination between the 2 groups of metabolic syndrome and non-metabolic syndrome.

In step SA-13, the discriminant value may be calculated based on both at least one concentration value of Val, Leu, Ile, Tyr, Trp, Glu, Ala, Asp, Gly, Ser and Thr contained in the amino acid concentration data of the individual from which the data containing defective and outliers was removed in step SA-12 and the multivariate discriminant containing at least one of Val, Leu, Ile, Tyr, Trp, Glu, Ala, Asp, Gly, Ser and Thr as the variable, and the discriminant value calculated may be compared with a previously established threshold (cutoff value), hereby discriminating between metabolic syndrome and non-metabolic syndrome in the individual. Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

In step SA-13, the discriminant value may be calculated based on both at least two concentration values of Val, Leu, Ile, Tyr, Trp, Glu, Ala, Asp, Gly, Ser and Thr contained in the amino acid concentration data of the individual from which the data containing defective and outliers was removed in step SA-12 and the multivariate discriminant containing at least two of Val, Leu, Ile, Tyr, Trp, Glu, Ala, Asp, Gly, Ser and Thr as the variables, and the discriminant value calculated may be compared with a previously established threshold (cutoff value), thereby discriminating between metabolic syndrome and non-metabolic syndrome in the individual. Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

In step SA-13, the multivariate discriminant may be expressed by one fractional expression or the sum of a plurality of the fractional expressions and may contain either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant. Specially, the multivariate discriminant may be formula 1. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

Thr/Ser+(Glu+Ala)/Gly (formula 1)

In step SA-13, the multivariate discriminant may be any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree. Specially, the multivariate discriminant contains Glu, Gly, Ala, Thr and Ser as the variables. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

The multivariate discriminants described above can be prepared by a method described in International Publication WO 2004/052191 that is an international application filed by the present applicant or by a method (multivariate discriminant-preparing processing described in the second embodiment described later) described in International Publication PCT/JP2006/304398 that is an international application filed by the present applicant. Any multivariate discriminants obtained by these methods can be preferably used in evaluation of the state of metabolic syndrome, regardless of the unit of amino acid concentration in the amino acid concentration data as input data.

### Second Embodiment

### 2-1. Outline of the Invention

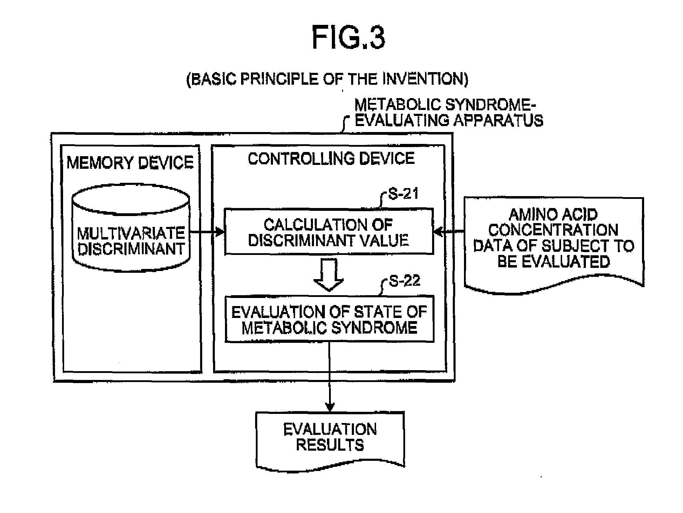

Herein, an outline of the metabolic syndrome-evaluating apparatus, the metabolic syndrome-evaluating method, the metabolic syndrome-evaluating system, the metabolic syndrome-evaluating program and the recording medium of the present invention are described in detail with reference to FIG. 3. FIG. 3 is a principle configurational diagram showing the basic principle of the present invention.

In the present invention, a discriminant value that is the value of multivalent discriminant is calculated in a control device based on both the previously obtained amino acid concentration data of an subject to be evaluated (for example, an individual such as animal or human) and the previously established multivariate discriminant with the concentration of amino acid as variable, stored in the memory device (step S-21).

In the present invention, the state of metabolic syndrome in the subject to be evaluated is evaluated in the control device based on the discriminant value calculated in step S-21 (step S-22).

According to the present invention, a discriminant value that is a value of multivariate discriminant is calculated based on both previously obtained amino acid concentration data on the concentration value of amino acid in the subject and a multivariate discriminant with the concentration of the amino acid as variable stored in the memory unit, and the state of metabolic syndrome is evaluated in the subject based on the discriminant value calculated. Thus, a discriminant value obtained in a multivariate discriminant wherein the concentrations of amino acids are variables can be utilized to bring about an effect of enabling accurate evaluation of the state of metabolic syndrome.

In step S-21, the discriminant value may be calculated based on both the concentration value of at least one of Val, Leu, Ile, Tyr, Trp, Glu, Ala, Asp, Gly, Ser and Thr contained in previously obtained amino acid concentration data of the subject and the multivariate discriminant containing at least one of Val, Leu, Ile, Tyr, Trp, Glu, Ala, Asp, Gly, Ser and Thr as the variable. Thus, a discriminant value obtained in a multivariate discriminant correlated significantly with the state of metabolic syndrome can be utilized to bring about an effect of enabling accurate evaluation of the state of metabolic syndrome.

In step S-22, the subject may be discriminated between metabolic syndrome and non-metabolic syndrome based on the discriminant value calculated in step S-21. Specially, the discriminant value may be compared with a previously established threshold (cutoff value), thereby discriminating between metabolic syndrome and non-metabolic syndrome in the subject. Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome,

In step S-21, the discriminant value may be calculated based on both the concentration values of at least two of Val, Leu, Ile, Tyr, Trp, Glu, Ala, Asp, Gly, Ser and Thr contained in the previously obtained amino acid concentration data of the subject and the multivariate discriminant containing at least two of Val, Leu, Ile, Tyr, Trp, Glu, Ala, Asp, Gly, Ser and Thr as the variables.
Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

In step S-21, the multivariate discriminant may be expressed by one fractional expression or the sum of a plurality of the fractional expressions and may contain either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant. Specially, the multivariate discriminant may be formula 1. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

Thr/Ser+(Glu+Ala)/Gly (formula 1)

In step S-21, the multivariate discriminant may be any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree. Specially, the multivariate discriminant may contain Glu, Gly, Ala, Thr and Ser as the variables. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

The multivariate discriminants described above can be prepared by a method described in International Publication WO 2004/052191 that is an international application filed by the present applicant or by a method (multivariate discriminant-preparing processing described later) described in International Publication PCT/JP2006/304398 that is an international application filed by the present applicant. Any multivariate discriminants obtained by these methods can be preferably used in evaluation of the state of metabolic syndrome, regardless of the unit of amino acid concentration in the amino acid concentration data as input data.

In a fractional expression, the numerator of the fractional expression is expressed by the sum of amino acids A, B, C etc. and/or the denominator of the fractional expression is expressed by the sum of amino acids a, b, c etc. The fractional expression also includes the sum of fractional expressions α, β, γ etc. (for example, α+β) having such constitution. The fractional expression also includes divided fractional expressions. Amino acids used in the numerator or denominator may have suitable coefficients respectively. The amino acids used in the numerator or denominator may appear repeatedly. Each fractional expression may have a suitable coefficient. The value of a coefficient for each variable and the value for a constant term may be any real numbers.

Specifically, the fractional expression has a combination of amino acid concentrations in Groups A to D below, and is preferably "represented by one or more fractional expressions containing 2 to 8 amino acid concentration variables, wherein the numerator has (1 or more variables from Groups A and B + 0 to 2 variables from Group D) and the denominator has (1 or more variables from Group C + 0 to 2 variables from Group D), more preferably "represented by 2 or more fractional expressions containing 4 to 8 amino acid concentration variables, wherein the numerator has (1 or more variables from Group B + 0 to 1 variable from Group A + 0 to 2 variables from Group D) and the denominator has (1 or more variables from Group C + 0 to 2 variables from Group D). In a combination where variables in the numerator and variables in the denominator in the fractional expression are switched with each other, the positive (or negative) sign is generally reversed in correlation with objective variables, but because their correlation is maintained, such combination can be assumed to be equivalent to the original, and thus the fractional expression may include such combination.
Group A: Leu, Ile, Val
Group B: Ala, Glu, Asp, Tyr, Trp, Thr
Group C: Gly, Ser
Group D: amino acids (or amino acid metabolites thereof) not contained in Groups A, B and C

The multivariate discriminant refers to a form of equation used generally in multivariate analysis and includes, for example, multiple regression equation, multiple logistic regression equation, linear discriminant function, Mahalanobis' generalized distance, canonical discriminant function, support vector machine, and decision tree. The multivariate discriminant also includes an equation shown by the sum of different forms of multivariate discriminants. In the multiple regression equation, multiple logistic regression equation and canonical discriminant function, a coefficient and constant term are added to each variable, and the coefficient and constant term in this case are preferably real numbers, more preferably values in the range of 99% confidence interval for the coefficient and constant term obtained from data for discrimination, more preferably in the range of 95% confidence interval for the coefficient and constant term obtained from data for discrimination. The value of each coefficient and the confidence interval thereof may be those multiplied by a real number, and the value of each constant term and the confidence interval thereof may be those having an arbitrary actual constant added or subtracted or those multiplied or divided by an arbitrary actual constant.

When the state of metabolic syndrome is evaluated (specifically discrimination between metabolic syndrome and non-metabolic syndrome) in the present invention, the concentrations of other metabolites (biological metabolites), the protein expression level, the age and sex of the subject, biological indices or the like may be used in addition to the amino acid concentration data. When the state of metabolic syndrome is evaluated (specifically discrimination between metabolic syndrome and non-metabolic syndrome) in the present invention, the concentrations of other metabolites (biological metabolites), the protein expression level, the age and sex of the subject, biological indices or the like may be used as variables in the multivariate discriminant in addition to the amino acid concentration.

Here, the summary of the multivariate discriminant-preparing processing (steps 1 to 4) is described in detail.

First, from metabolic syndrome state information including amino acid concentration data and metabolic syndrome state index data concerning an index showing the state of metabolic syndrome stored in a memory device, a candidate multivariate discriminant that is a candidate for a multivariate discriminant (e.g., y=a₁x₁+a₂x₂+...+aₙxₙ, y: metabolic syndrome state index data, xᵢ: amino acid concentration data, aᵢ: constant, i = 1,2, ... , n) is prepared by a predetermined discriminant-preparing method at the control device (step 1). Data containing defective and outliers may be removed in advance from the metabolic syndrome state information.

In step 1, a plurality of candidate multivariate discriminants may be prepared from the metabolic syndrome state information by using a plurality of different discriminant-preparing methods (including those for multivariate analysis such as principal component analysis, discriminant analysis, support vector machine, multiple regression analysis, logistic regression analysis, k-means method, cluster analysis, and decision tree). Specifically, a plurality of candidate multivariate discriminant groups may be prepared simultaneously and concurrently by using a plurality of different algorithms with metabolic syndrome state information which is multivariate data composed of amino acid concentration data and metabolic syndrome state index data obtained by analyzing blood samples from a large number of healthy groups and metabolic syndrome patient groups. For example, two different candidate multivariate discriminants may be formed by performing discriminant analysis and logistic regression analysis simultaneously with different algorithms. Alternatively, a candidate multivariate discriminant may be formed by converting metabolic syndrome state information with the candidate multivariate discriminant prepared by performing principal component analysis and then performing discriminant analysis of the converted metabolic syndrome state information. In this way, it is possible to finally prepare a candidate multivariate discriminant suitable for diagnostic condition.

The candidate multivariate discriminant prepared by principal component analysis is a linear expression consisting of amino acid variables maximizing the variance of all amino acid concentration data. The candidate multivariate discriminant prepared by discriminant analysis is a high-powered expression (including exponential and logarithmic expressions) consisting of amino acid variables minimizing the ratio of the sum of the variances in respective groups to the variance of all amino acid concentration data. The candidate multivariate discriminant prepared by using support vector machine is a high-powered expression (including kernel function) consisting of amino acid variables maximizing the boundary between groups. The candidate multivariate discriminant prepared by multiple regression analysis is a high-powered expression consisting of amino acid variables minimizing the sum of the distances from all amino acid concentration data. The candidate multivariate discriminant prepared by logistic regression analysis is a fraction expression having, as a component, the natural logarithm having a linear expression consisting of amino acid variables maximizing the likelihood as the exponent. The k-means method is a method of searching k pieces of neighboring amino acid concentration data in various groups designating the group containing the greatest number of the neighboring points as its data-belonging group, and selecting an amino acid variable that makes the group to which input amino acid concentration data belong agree well with the designated group. The cluster analysis is a method of clustering the points closest in entire amino acid concentration data. The decision tree is a method of ordering amino acid variables and predicting the group of amino acid concentration data from the pattern possibly held by the higher-ordered amino acid variable.

Returning to the description of the multivariate discriminant-preparing processing, the candidate multivariate discriminant prepared in step 1 is verified (mutually verified) in the control device by a particular verification method (step 2). Verification of the candidate multivariate discriminant is performed on each other to each candidate multivariate discriminant prepared in step 1.

In step 2, at least one of the discrimination rate, sensitivity, specificity, information criterion, and the like of the candidate multivariate discriminant may be verified by at least one of the bootstrap method, holdout method, leave-one-out method, and the like. In this way, it is possible to prepare a candidate multivariate discriminant higher in predictability or reliability, by taking the metabolic syndrome state information and the diagnostic condition into consideration.

The discrimination rate is the rate of the data wherein the state of metabolic syndrome evaluated according to the present invention is correct, in all input data. The sensitivity is the rate of the states of metabolic syndrome judged correct according to the present invention in the states of metabolic syndrome declared in the input data. The specificity is the rate of the states of metabolic syndrome judged correct according to the present invention in the states of metabolic syndrome described healthy in the input data. The information criterion is the sum of the number of the amino acid variables in the candidate multivariate discriminant prepared in step 1 and the difference in number between the states of metabolic syndrome evaluated according to the present invention and those described in input data. The predictability is the average of the discrimination rate, the sensitivity, or the specificity obtained by repeating verification of the candidate multivariate discriminant. The reliability is the variance of the discrimination rate, the sensitivity, or the specificity obtained by repeating verification of the candidate multivariate discriminant.

Returning to the description of the multivariate discriminant-preparing processing, a combination of amino acid concentration data contained in the metabolic syndrome state information used in preparing the candidate multivariate discriminant is selected by selecting a variable of the candidate multivariate discriminant from the verification result in step 2 according to a predetermined variable selection method in the control device (step 3). The selection of amino acid variable is performed on each candidate multivariate discriminant prepared in step 1. In this way, it is possible to select the amino acid variable of the candidate multivariate discriminant properly. The step 1 is executed once again by using the metabolic syndrome state information including the amino acid concentration data selected in step 3.

From the verification result in step 2, an amino acid variable of the candidate multivariate discriminant may be selected in step 3, based on at least one of stepwise method, best path method, local search method, and genetic algorithm.

The best path method is a method of selecting an amino acid variable by optimizing the evaluation index of the candidate multivariate discriminant while eliminating the variables contained in the candidate multivariate discriminant one by one.

Returning to the description of the multivariate discriminant-preparing processing, the steps 1, 2 and 3 are repeatedly performed in the control device, and based on verification results thus accumulated, a candidate multivariate discriminant used as multivariate discriminant is selected from a plurality of candidate multivariate discriminants, thereby preparing the multivariate discriminant (step 4). In selection of the candidate multivariate discriminants, there are cases where the optimum multivariate discriminant is selected from candidate multivariate discriminants prepared in the same method or the optimum multivariate discriminant is selected from all candidate multivariate discriminants.

As described above, processing for preparation of candidate multivariate discriminants based on metabolic syndrome state information, verification of the candidate multivariate discriminants, and selection of variables in the candidate multivariate discriminants are performed in a series of operations in a systematized manner in the multivariate discriminant-preparing processing, whereby the optimum multivariate discriminant for evaluation of the state of metabolic syndrome can be prepared. In other words, in the multivariate discriminant-preparing processing, amino acid concentration is used in multivariate statistical analysis, and for selecting the optimum and robust combination of variables, the variable selection method is combined with cross-validation to extract a multivariate discriminant having high diagnosis performance. Logistic regression equation, linear discriminant function, support vector machine, Mahalanobis' generalized distance, multiple regression analysis, cluster analysis and the like can be used in the multivariate discriminant.

### 2-2. System configuration

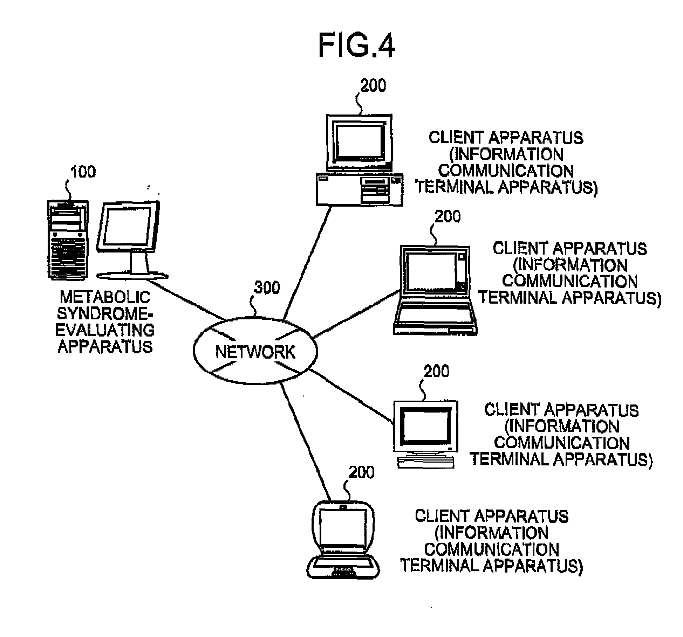

Hereinafter, the configuration of the metabolic syndrome-evaluating system according to the second embodiment (hereinafter referred to sometimes as the present system) will be described with reference to FIGS. 4 to 20. This system is merely one example, and the present invention is not limited thereto.

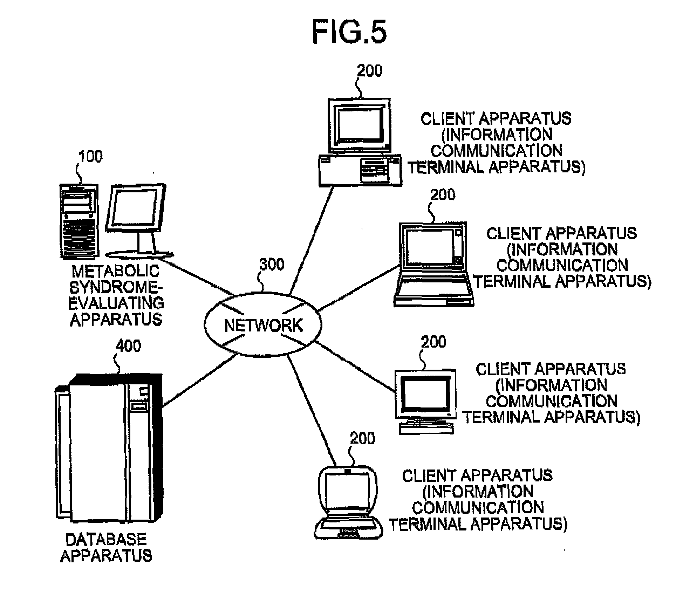

First, the entire configuration of the present system will be described with reference to FIGS. 4 and 5. FIG. 4 is a diagram showing an example of the entire configuration of the present system. FIG. 5 is a diagram showing another example of the entire configuration of the present system. As shown in FIG. 4, the present system is constituted in which a metabolic syndrome-evaluating apparatus 100 that evaluates the state of metabolic syndrome in a subject to be evaluated, and a client apparatus 200 (corresponding to the information communication terminal apparatus of the present invention) which provides the amino acid concentration data on the concentration values of amino acids in the subject, are communicatively connected to each other via a network 300.

In the present system as shown in FIG. 5, in addition to the metabolic syndrome-evaluating apparatus 100 and the client apparatus 200, a database apparatus 400 storing, for example, the metabolic syndrome state information used in preparing a multivariate discriminant and the multivariate discriminant used in evaluating the state of metabolic syndrome in the metabolic syndrome-evaluating apparatus 100, may be communicatively connected via the network 300. In this configuration, the information on the state of metabolic syndrome etc. is provided via the network 300 from the metabolic syndrome-evaluating apparatus 100 to the client apparatuses 200 and the database apparatus 400, or from the client apparatuses 200 and the database apparatus 400 to the metabolic syndrome-evaluating apparatus 100. The "information on the state of metabolic syndrome" is information on the measured values of particular items of the state of metabolic syndrome of organisms including human. The information on the state of metabolic syndrome is generated in the metabolic syndrome-evaluating apparatus 100, client apparatus 200, and other apparatuses (e.g., various measuring apparatuses) and stored mainly in the database apparatus 400.

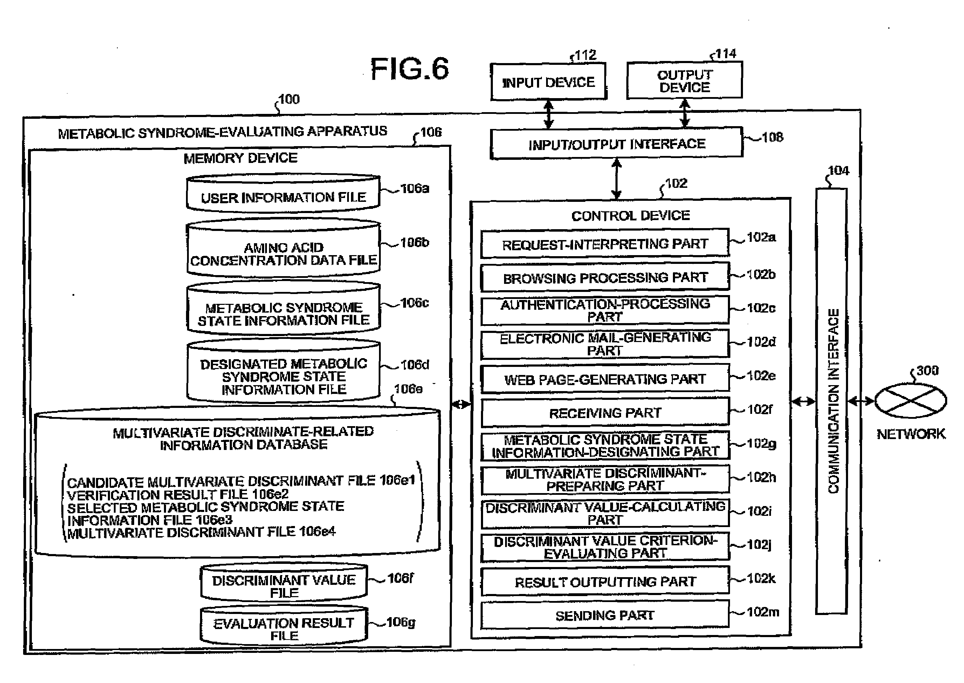

Now, the configuration of the metabolic syndrome-evaluating apparatus 100 in the present system will be described with reference to FIGS. 6 to 18. FIG. 6 is a block diagram showing an example of the configuration of the metabolic syndrome-evaluating apparatus 100 in the present system, showing conceptually only the region relevant to the present invention.

The metabolic syndrome-evaluating apparatus 100 includes a control device 102, such as CPU (Central Processing Unit), that integrally controls the metabolic syndrome-evaluating apparatus 100, a communication interface 104 that connects the Metabolic syndrome-evaluating apparatus 100 to the network 300 communicatively via communication apparatuses such as router and a wired or wireless communication line such as private line, a memory device 106 that stores various databases, tables, files and others, and an input/output interface 108 connected to an input device 112 and an output device 114, that are connected to each other communicatively via any communication channel. The metabolic syndrome-evaluating apparatus 100 may be present together with various analyzers (e.g., amino acid analyzer) in a same housing. Typical configuration of disintegration/integration of the metabolic syndrome-evaluating apparatus 100 is not limited to that shown in the figure, and all or a part of it may be disintegrated or integrated functionally or physically in any unit, for example, according to various loads applied. For example, a part of the processing may be performed via a CGI (Common Gateway Interface).

The memory device 106 is a storage means, and examples thereof include memory apparatuses such as RAM (Random Access Memory) and ROM (Read Only Memory), fixed disk drives such as hard disk, flexible disk, optical disk, and the like. The memory device 106 stores computer programs giving instructions to CPU for various processing, together with OS (Operating System). As shown in the figure, the memory device 106 stores a user information file 106a, an amino acid concentration data file 106b, a metabolic syndrome state information file 106c, a designated metabolic syndrome state information file 106d, a multivariate discriminant-related information database 106e, a discriminant value file 106f and an evaluation result file 106g.

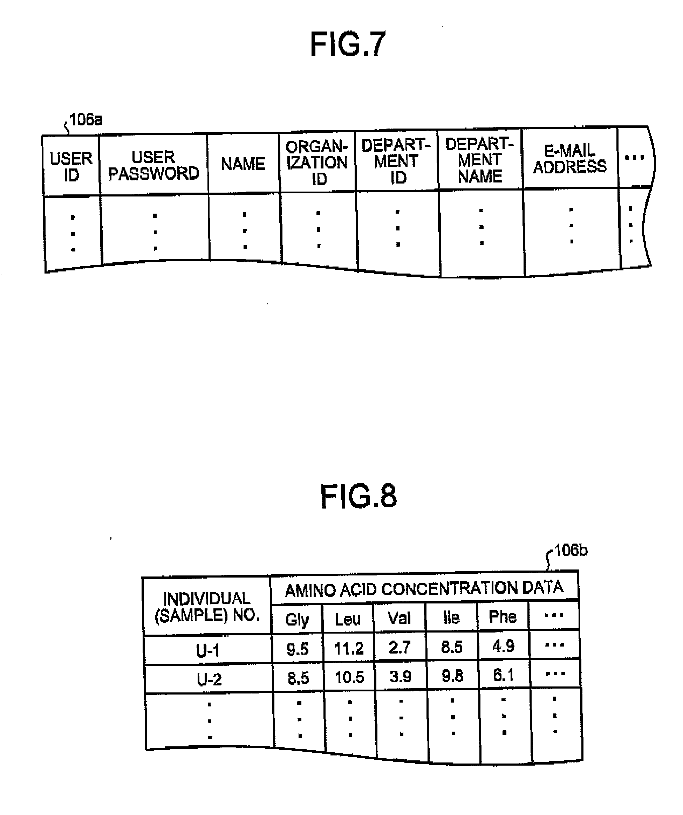

The user information file 106a stores user information on users. FIG. 7 is a chart showing an example of the information stored in the user information file 106a. As shown in FIG. 7, the information stored in the user information file 106a includes user ID (identification) for identifying the user uniquely, user password for authentication of the user, user name, organization ID uniquely identifying the organization of the user, department ID for uniquely identifying the department of the user organization, department name, and electronic mail address of the user that are correlated to one another.

Returning to FIG. 6, the amino acid concentration data file 106b stores amino acid concentration data on amino acid concentration values. FIG. 8 is a chart showing an example of the information stored in the amino acid concentration data file 106b. As shown in FIG. 8, the information stored in the amino acid concentration data file 106b includes individual number for uniquely identifying an individual (sample) as an subject to be evaluated and amino acid concentration data that are correlated to one another. In FIG. 8, the amino acid concentration data are assumed to be numerical values, i.e., on continuous scale, but the amino acid concentration data may be expressed on nominal scale or ordinal scale. In the case of nominal or ordinal scale, any number may be allocated to each state for analysis. The amino acid concentration data may be combined with other biological information (e.g., sex difference, age, height, weight, BMI index, abdominal circumference, insulin resistance index, uric acid level, blood glucose level, triglyceride, body fat percentage, total cholesterol, HDL cholesterol, LDL cholesterol, systolic pressure, diastolic pressure, hemoglobin Alc, arteriosclerosis index, smoking or not, digitalized electrocardiogram waveform, enzyme concentration, gene expression level, and the concentrations of metabolites other than amino acids).

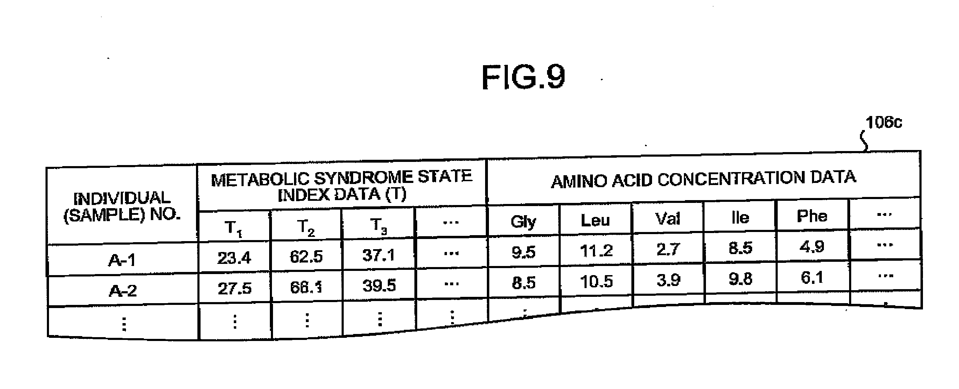

Returning to FIG. 6, the metabolic syndrome state information file 106c stores the metabolic syndrome state information used in preparing a multivariate discriminant. FIG. 9 is a chart showing an example of the information stored in the metabolic syndrome state information file 106c. As shown in FIG. 9, the information stored in the metabolic syndrome state information file 106c includes individual (sample) number, metabolic syndrome state index data (T) corresponding to the metabolic syndrome state index (index T₁, index T₂, index T₃ ...), and amino acid concentration data that are correlated to one another. In FIG. 9, the metabolic syndrome state index data and the amino acid concentration data are assumed to be numerical values, i.e., on continuous scale, but the metabolic syndrome state index data and the amino acid concentration data may be expressed on nominal scale or ordinal scale. In the case of nominal or ordinal scale, any number may be allocated to each state for analysis. The metabolic syndrome state index data is a single known state index serving as a marker of the state of metabolic syndrome, and numerical data may be used.

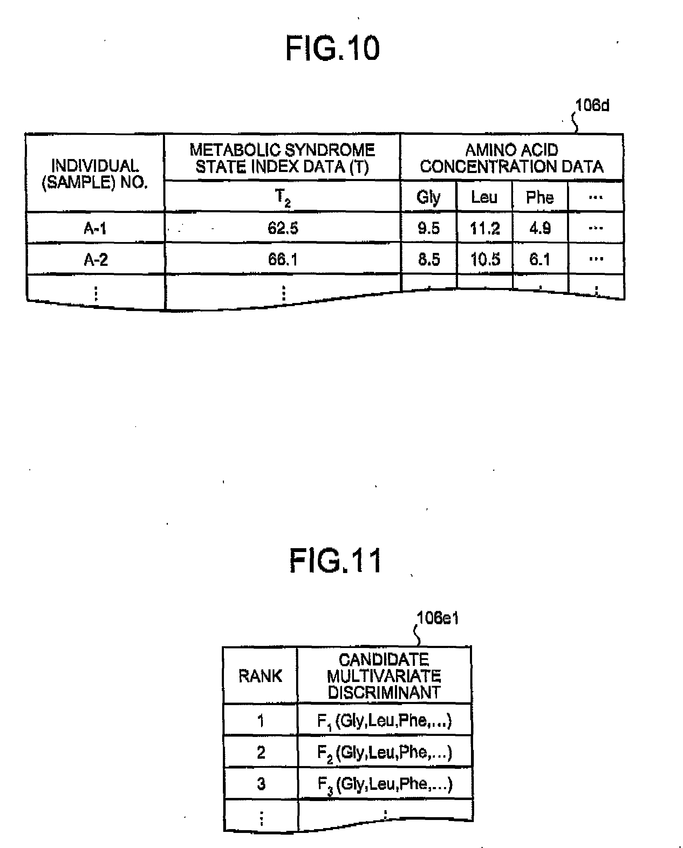

Returning to FIG. 6, the designated metabolic syndrome state information file 106d stores the metabolic syndrome state information designated in the metabolic syndrome state information-designating part 102g described below. FIG. 10 is a chart showing an example of the information stored in the designated metabolic syndrome state information file 106d. As shown in FIG. 10, the information stored in the designated metabolic syndrome state information file 106d includes individual number, designated metabolic syndrome state index data, and designated amino acid concentration data that are correlated to one another.

Returning to FIG. 6, the multivariate discriminant-related information database 106e is composed of a candidate multivariate discriminant file 106e1 storing the candidate multivariate discriminant prepared in the candidate multivariate discriminant-preparing part 102h1 described below; a verification result file 106e2 storing the verification results in the candidate multivariate discriminant-verifying part 102h2 described below; a selected metabolic syndrome state information file 106e3 storing the metabolic syndrome state information containing the combination of amino acid concentration data selected in the variable-selecting part 102h3 described below; and a multivariate discriminant file 106e4 storing the multivariate discriminant prepared in the multivariate discriminant-preparing part 102h described below.

The candidate multivariate discriminant file 106e1 stores the candidate multivariate discriminant prepared in the candidate multivariate discriminant-preparing part 102h1 described below. FIG. 11 is a chart showing an examples of the information stored in the candidate multivariate discriminant file 106e1. As shown in FIG. 11, the information stored in the candidate multivariate discriminant file 106e1 includes rank, and candidate multivariate discriminant (e.g., F₁ (Gly, Leu, Phe, ...), F₂ (Gly, Leu, Phe, ...), or F₃ (Gly, Leu, Phe, ...) in FIG. 11) that are correlated to each other.

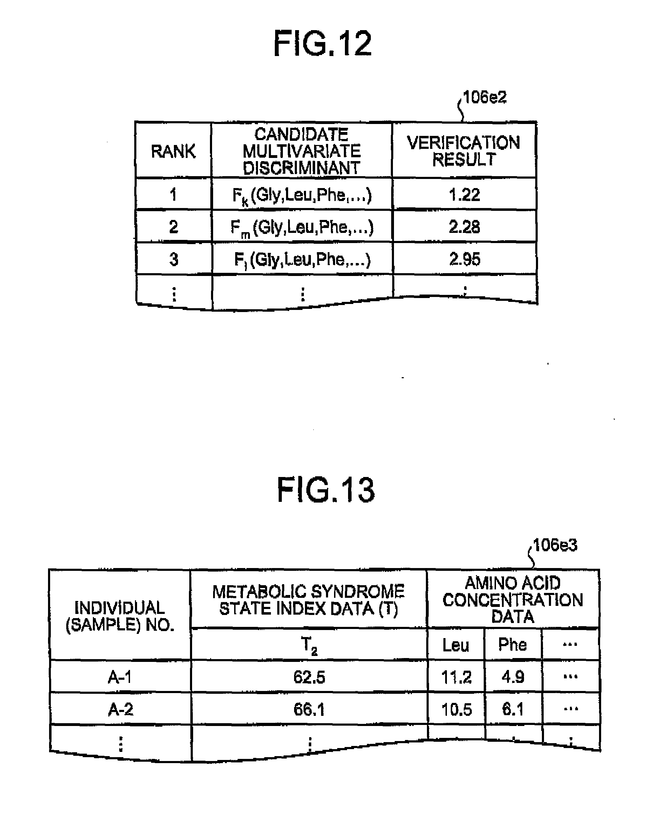

Returning to FIG. 6, the verification result file 106e2 stores the verification results verified in the candidate multivariate discriminant-verifying part 102h2 described below. FIG. 12 is a chart showing an example of the information stored in the verification result file 106e2. As shown in FIG. 12, the information stored in the verification result file 106e2 includes rank, candidate multivariate discriminant (e.g., Fₖ (Gly, Leu, Phe, ...), Fₘ (Gly, Leu, Phe, ...), Fl (Gly, Leu, Phe, ...) in FIG. 12), and the verification results of each candidate multivariate discriminant (e.g., evaluation value of each candidate multivariate discriminant) that are correlated to one another.

Returning to FIG. 6, the selected metabolic syndrome state information file 106e3 stores the metabolic syndrome state information including the combination of amino acid concentration data corresponding to the variable selected in the variable-selecting part 102h3 described below. FIG. 13 is a chart showing an example of the information stored in the selected metabolic syndrome state information file 106e3. As shown in FIG. 13, the information stored in the selected metabolic syndrome state information file 106e3 includes individual number, the metabolic syndrome state index data designated in the metabolic syndrome state information-designating part 102g described below, and the amino acid concentration data selected in the variable-selecting part 102h3 described below that are correlated to one another.

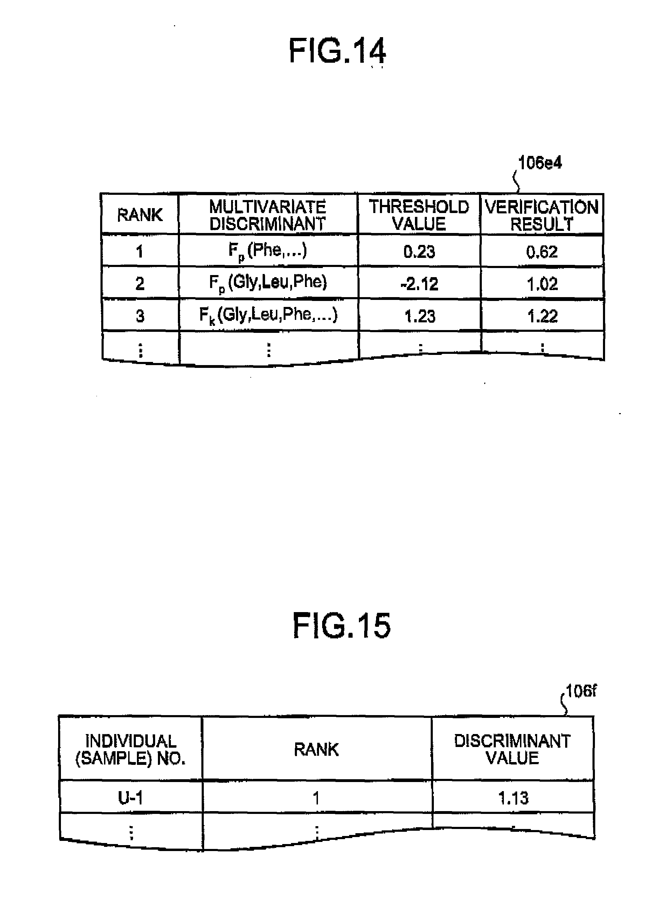

Returning to FIG. 6, the multivariate discriminant file 106e4 stores the multivariate discriminant prepared in the multivariate discriminant-preparing part 102h described below. FIG. 14 is a chart showing an example of the information stored in the multivariate discriminant file 106e4. As shown in FIG. 14, the information stored in the multivariate discriminant file 106e4 includes rank, multivariate discriminant (e.g., Fₚ (Phe, ...), Fₚ (Gly, Leu, Phe), Fₖ (Gly, Leu, Phe, in FIG. 14), a threshold corresponding to each discriminant-preparing method, and verification results of each multivariate discriminant (e.g., evaluation value of each (multivariate discriminant) that are correlated to one another.

Returning to FIG. 6, the discriminant value file 106f stores the discriminant value calculated in the discriminant value-calculating part 102i described below. FIG. 15 is a chart showing an example of the information stored in the discriminant value file 106f. As shown in FIG. 15, the information stored in the discriminant value file 106f includes individual number for uniquely identifying an individual (sample) as a subject to be evaluated, rank (number for uniquely identifying the multivariate discriminant), and discriminant value that are correlated to one another.

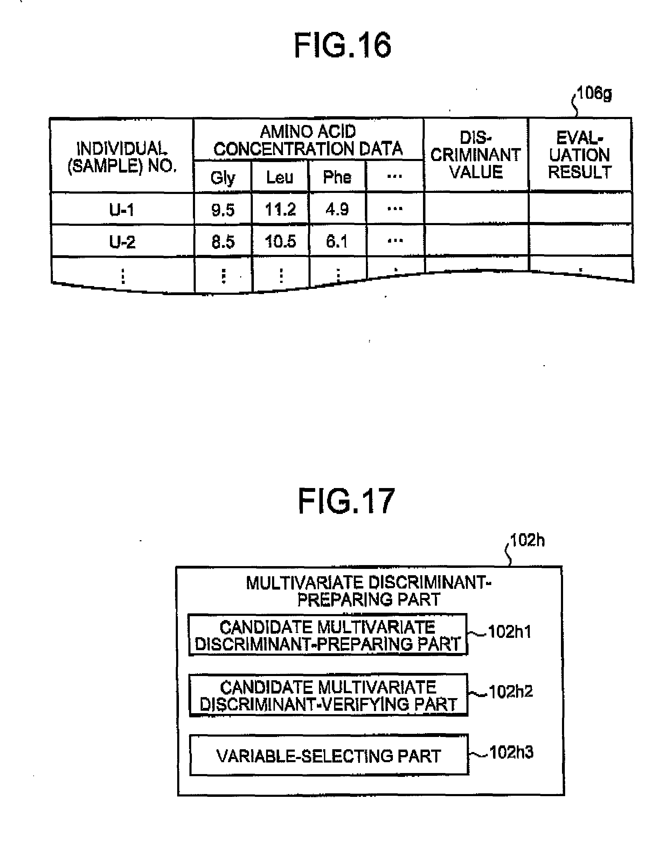

Returning to FIG. 6, the evaluation result file 106g stores the evaluation results obtained in the discriminant value criterion-evaluating part 102j described below (specifically the discrimination results obtained in the discriminant value criterion-discriminating part 102j1). FIG. 16 is a chart showing an example of the information stored in the evaluation result file 106g. The information stored in the evaluation result file 106g includes individual number for uniquely identifying an individual (sample) as a subject to be evaluated, the previously obtained amino acid concentration data on a subject to be evaluated, the discriminant value calculated in the multivariate discriminant, and the evaluation results on the metabolic syndrome state (specifically, discrimination results as to discrimination between metabolic syndrome and non-metabolic syndrome) that are correlated to one another.

Returning to FIG. 6, the memory device 106 stores various Web data, CGI programs, and others for providing the client apparatuses 200 with web site information as information other than the information described above. The Web data include various data for displaying the Web page described below and others, and the data are generated as, for example, a HTML (HyperText Markup Language) or XML (Extensible Markup Language) text file. Other temporary files such as files for the components for generation of Web data and for operation, and others are also stored in the memory device 106. In addition, it may store as needed sound files in the WAVE or AIFF (Audio Interchange File Format) format for transmission to the client apparatuses 200 and image files of still image or motion picture in the JPEG (Joint Photographic Experts Group) or MPEG2 (Moving Picture Experts Group phase 2) format.

The communication interface 104 allows communication between the metabolic syndrome-evaluating apparatus 100 and the network 300 (or communication apparatus such as router). Thus, the communication interface 104 has a function to communicate data via a communication line with other terminals.

The input/output interface 108 is connected to the input device 112 and the output device 114. A monitor (including home television), a speaker, or a printer may be used as the output device 114 (hereinafter, the output device 114 may be described as monitor 114). A keyboard, a mouse, a microphone, or a monitor functioning as a pointing device together with a mouse may be used as the input device 112.

The control device 102 has an internal memory storing control programs such as OS (Operating System), programs for various processing procedures, and other needed data, and performs information processing according to these programs. As shown in the figure, the control device 102 includes mainly the request-interpreting part 102a, a browsing processing part 102b, an authentication-processing part 102c, an electronic mail-generating part 102d, a Web page-generating part 102e, a receiving part 102f, a metabolic syndrome state information-designating part 102g, a multivariate discriminant-preparing part 102h, a discriminant value-calculating part 102i, a discriminant value criterion-evaluating part 102j, a result outputting part 102k and a sending part 102m. The control device 102 performs data processing such as removal of data including defective or many outliers and of variables for the defective value-including data in the metabolic syndrome state information transmitted from the database apparatus 400 and in the amino acid concentration data transmitted from the client apparatus 200.

The request-interpreting part 102a interprets the request from the client apparatus 200 or the database apparatus 400 and sends the request to other parts in the control device 102 according to the analytical result. Upon receiving browsing request for various screens from the client apparatus 200, the browsing processing part 102b generates and transmits the web data for these screens. Upon receiving authentication request from the client apparatus 200 or the database apparatus 400, the authentication-processing part 102c performs authentication. The electronic mail-generating part 102d generates an electronic mail including various kinds of information. The Web page-generating part 102e generates a Web page for a user to browse with the client apparatus 200.

The receiving part 102f receives, via the network 300, the information (specifically, the amino acid concentration data, metabolic syndrome state information, multivariate discriminant etc.) transmitted from the client apparatus 200 and the database apparatus 400. The metabolic syndrome state information-designating part 102g designates the objective metabolic syndrome state index data and amino acid concentration data in preparing the multivariate discriminant.

The multivariate discriminant-preparing part 102h generates a multivariate discriminant based on the metabolic syndrome state information received in the receiving part 102f and the metabolic syndrome state information designated in the metabolic syndrome state information-designating part 102g. Specifically, the multivariate discriminant-preparing part 102h generates a multivariate discriminant by selecting a candidate multivariate discriminant to be used as the multivariate discriminant from a plurality of candidate multivariate discriminants, according to the verification results accumulated by repeating the processings in the candidate multivariate discriminant-preparing part 102h1, the candidate multivariate discriminant-verifying part 102h2 and the variable-selecting part 102h3 from the metabolic syndrome state information.

If a previously generated multivariate discriminant is stored in a predetermined region of the memory device 106, the multivariate discriminant-preparing part 102h may generate a multivariate discriminant by selecting a desired multivariate discriminant out of the memory device 106. Alternatively, the multivariate discriminant-preparing part 102h may generate the multivariate discriminant by selecting and downloading a desired multivariate discriminant from the multivariate discriminants previously stored in another computer apparatus (e.g., database apparatus 400).

Hereinafter, the configuration of the multivariate discriminant-preparing part 102h will be described with reference to FIG. 17. FIG. 17 is a block diagram showing the configuration of the multivariate discriminant-preparing part 102h, and only a part in the configuration related to the present invention is shown conceptually. The multivariate discriminant-preparing part 102h has a candidate multivariate discriminant-preparing part 102h1, a candidate multivariate discriminant-verifying part 102h2, and a variable-selecting part 102h3, additionally. The candidate multivariate discriminant-preparing part 102h1 generates a candidate multivariate discriminant that is a candidate of the multivariate discriminant from the metabolic syndrome state information according to a predetermined discriminant-preparing method. Specifically, the candidate Multivariate discriminant-preparing part 102h1 may generate a plurality of candidate multivariate discriminants from the metabolic syndrome state information, by using a plurality of different discriminant-preparing methods. The candidate multivariate discriminant-verifying part 102h2 verifies the candidate multivariate discriminants prepared in the candidate multivariate discriminant-preparing part 102h1 according to a particular verification method. Specifically, the candidate multivariate discriminant-verifying part 102h2 may verify at least one of the discrimination rate, sensitivity, specificity, and information criterion of the candidate multivariate discriminants according to at least one of bootstrap method, holdout method, and leave-one-out method. The variable-selecting part 102h3 selects the combination of the amino acid concentration data contained in the metabolic syndrome state information to be used in preparing the candidate multivariate discriminant, by selecting a variable of the candidate multivariate discriminant from the verification results in the candidate multivariate discriminant-verifying part 102h2 according to a particular variable selection method. The variable-selecting part 102h3 may select the variable of the candidate multivariate discriminant from the verification results according to at least one of stepwise method, best path method, local search method, and genetic algorithm.

Returning to FIG. 6, the discriminant value-calculating part 102i calculates a discriminant value that is the value of the multivariate discriminant, based on both the multivariate discriminant (for example, containing at least one or two of Val, Leu, Ile, Tyr, Trp, Glu, Ala, Asp, Gly, Ser and Thr as variable(s)) prepared in the multivariate discriminant-preparing part 102h and the amino acid concentration data (for example, containing at least one or two concentration values of Val, Leu, Ile, Tyr, Trp, Glu, Ala, Asp, Gly, Ser and Thr) of an subject to be evaluated received in the receiving part 102f.

The multivariate discriminant may be expressed by one fractional expression or the sum of a plurality of the fractional expressions and may contain either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant. Specially, the multivariate discriminant may be formula 1.

Thr/Ser+(Glu+Ala)/Gly (formula 1)

The multivariate discriminant may be any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree. Specially, the multivariate discriminant may contain Glu, Gly, Ala, Thr and Ser as the variables.

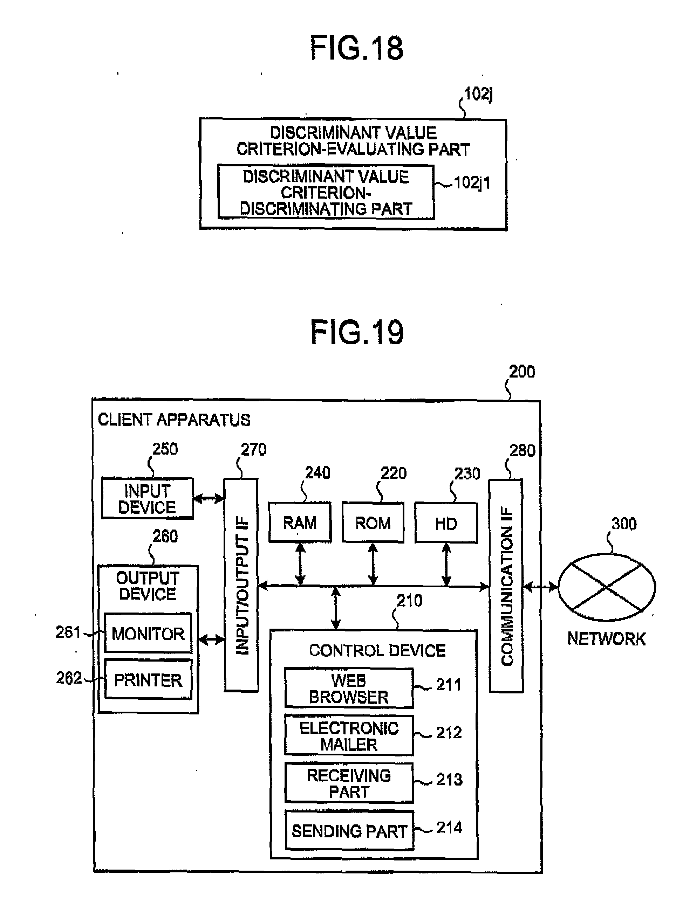

The discriminant value criterion-evaluating part 102j evaluates the state of metabolic syndrome in the subject to be evaluated, based on the discriminant value calculated by the discriminant value-calculating part 102i. The discriminant value criterion-evaluating part 102j further includes a discriminant value criterion-discriminating part 102j1. Now, the configuration of the discriminant value criterion-evaluating part 102j will be described with reference to FIG. 18. FIG. 18 is a block diagram showing the configuration of the discriminant value criterion-evaluating part 102j, and only a part in the configuration related to the present invention is shown conceptually. Based on the discriminant value, the discriminant value criterion-discriminating part 102j1 discriminates between metabolic syndrome and non-metabolic syndrome in the subject to be evaluated. Specifically, the discriminant value criterion-discriminating part 102j1 compares the discriminant value with a predetermined threshold value (cutoff value), thereby discriminating between metabolic syndrome and non-metabolic syndrome in the subject to be evaluated.

Returning to FIG. 6, the result outputting part 102k outputs, into the output device 114, the processing results in each processing part in the control device 102 (the evaluation results in the discriminant value criterion-evaluating part 102j (specifically the discrimination results in the discriminant value criterion-discriminating part 102j1)) etc.

The sending part 102m sends the evaluation results to the client apparatus 200 that is the sender of the amino acid concentration data of the subject to be evaluated or sends the multivariate discriminant prepared in the metabolic syndrome-evaluating apparatus 100, and the evaluation results, to the database apparatus 400.

Hereinafter, the configuration of the client apparatus 200 in the present system will be described with reference to FIG. 19. FIG. 19 is a block diagram showing an example of the configuration of the client apparatus 200 in the present system, and only the part in the configuration relevant to the present invention is shown conceptually.

The client apparatus 200 includes a control device 210, ROM 220, HD (Hard Disk) 230, RAM 240, an input device 250, an output device 260, input/output IF 270, and communication IF 280 that are connected communicatively to one another through a communication channel.

The control device 210 has a Web browser 211, an electronic mailer 212, a receiving part 213, and a sending part 214. The Web browser 211 performs browsing processing of interpreting Web data and displaying the interpreted Web data on a monitor 261 described below. The Web browser 211 may have various plug-in software, such as stream player, having functions to receive, display and feedback streaming screen image. The electronic mailer 212 sends and receives electronic mails using a particular protocol (e.g., SMTP (Simple Mail Transfer Protocol) or POP3 (Post Office Protocol version 3)). The receiving part 213 receives various information, such as the evaluation results transmitted from the metabolic syndrome-evaluating apparatus 100, via the communication IF 280. The sending part 214 sends various information such as the amino acid concentration data on the subject to be evaluated, via communication IF 280, to the metabolic syndrome-evaluating apparatus 100.

The input device 250 is for example a keyboard, a mouse or a microphone. The monitor 261 described below also functions as a pointing device together with a mouse. The output device 260 is an output means for outputting the information received via the communication IF 280, and includes the monitor (including home television) 261 and a printer 262. In addition, the output device 260 may have a speaker or the like additionally. The input/output IF 270 is connected to the input device 250 and the output device 260.

The communication IF 280 connects the client apparatus 200 to the network 300 (or communication apparatus such as router) communicatively. In other words, the client apparatuses 200 are connected to the network 300 via a communication apparatus such as modem, TA (Terminal Adapter) or router, and a telephone line, or a private line. In this way, the client apparatuses 200 can access to the metabolic syndrome-evaluating apparatus 100 by using a particular protocol.

The client apparatus 200 may be realized by installing software (including programs, data and others) for Web data-browsing function and electronic mail-processing function to information processing apparatus (for example, information processing terminal such as known personal computer, workstation, family computer, Internet TV (Television), PHS (Personal Handyphone System) terminal, mobile phone terminal, mobile unit communication terminal or PDA (Personal Digital Assistants)) connected as needed with peripheral devices such as printer, monitor, and image scanner.

All or a part of processings of the control device 210 in the client apparatus 200 may be performed by a CPU and programs read and executed by the CPU. Thus, computer programs for giving instructions to the CPU and executing various processings together with the OS (Operating System) are recorded in the ROM 220 or HD 230. The computer programs, which are executed as they are loaded in the RAM 240, constitute the control device 210 with the CPU. The computer programs may be stored in an application program server connected via any network to the client apparatus 200, and the client apparatus 200 may download all or a part of them as needed. All or any part of processings of the control device 210 may be realized by hardware such as wired-logic.

Hereinafter, the network 300 in the present system will be described with reference to FIGS. 4 and 5. The network 300 has a function to connect the metabolic syndrome-evaluating apparatus 100, the client apparatuses 200, and the database apparatus 400 mutually, communicatively to one another, and is for example the Internet, intranet, or LAN (Local Area Network (both wired/wireless)). The network 300 may be VAN (Value Added Network), personal computer communication network, public telephone network (including both analog and digital), leased line network (including both analog and digital), CATV (Community Antenna Television) network, portable switched network or portable packet-switched network (including IMT2000 (International Mobile Telecommunication 2000) system, GSM (Global System for Mobile Communications) system, or PDC (Personal Digital Cellular)/PDC-P system), wireless calling network, local wireless network such as Bluetooth (registered trademark), PHS network, satellite communication network (including CS (Communication Satellite), BS (Broadcasting Satellite), and ISDB (Integrated Services Digital Broadcasting)), or the like

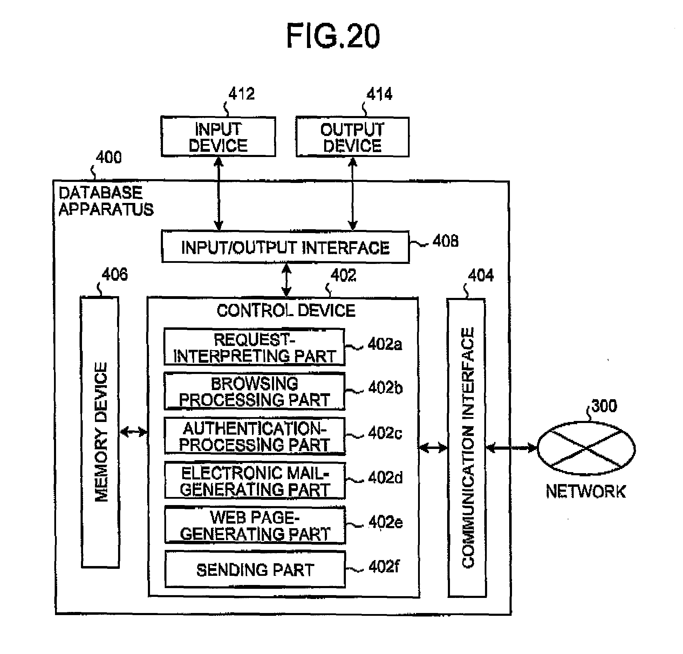

Hereinafter, the configuration of the database apparatus 400 in the present system will be described with reference to FIG. 20. FIG. 20 is a block diagram showing an example of the configuration of the database apparatus 400 in the present system, showing conceptually only the region relevant to the present invention.

The database apparatus 400 has functions to store, for example, the metabolic syndrome state information used in preparing a multivariate discriminant in the metabolic syndrome-evaluating apparatus 100 or in the database apparatus, the multivariate discriminant prepared in the metabolic syndrome-evaluating apparatus 100, and the evaluation results in the metabolic syndrome-evaluating apparatus 100. As shown in FIG. 20, the database apparatus 400 includes a control device 402, such as CPU, which controls the entire database apparatus 400 integrally, a communication interface 404 connecting the database apparatus to the network 300 communicatively via a communication apparatus such as router and via a wired or wireless communication circuit such as private line, a memory device 406 storing various data, tables and files (for example, file for Web page), and an input/output interface 408 connected to an input device 412 and an output device 414, and these parts are connected communicatively to each other via any communication channel.

The memory device 406 is a storage means, and may be, for example, memory apparatus such as RAM or ROM, fixed disk drive such as hard disk, flexible disk, optical disk, or the like. Various programs used in various processings are stored in the memory device 406. The communication interface 404 allows communication between the database apparatus 400 and the network 300 (or communication apparatus such as router). Thus, the communication interface 404 has a function to communicate data with other terminal via a communication line. The input/output interface 408 is connected to the input device 412 and the output device 414. A monitor (including home television), a speaker, or a printer may be used as the output device 414 (hereinafter, the output device 414 may be described as monitor 414). A keyboard, a mouse, a microphone, or a monitor functioning as a pointing device together with a mouse may be used as the input device 412.

The control device 402 has an internal memory storing control programs such as OS (Operating System), programs for various processing procedures, and other needed data, and performs various information processing according to these programs. As shown in the figure, the control device 402 includes mainly the request-interpreting part 402a, a browsing processing part 402b, an authentication-processing part 402c, an electronic mail-generating part 402d, a Web page-generating part 402e, and a sending part 402f.

The request-interpreting part 402a interprets the request from the metabolic syndrome-evaluating apparatus 100 and sends the request to other parts in the control device 402 according to the analytical result. Upon receiving various screen-browsing request from the metabolic syndrome-evaluating apparatus 100, the browsing processing part 402b generates and transmits web data for these screens. Upon receipt of authentication request from the metabolic syndrome-evaluating apparatus 100, the authentication-processing part 402c performs authentication. The electronic mail-generating part 402d generates an electronic mail including various information. The Web page-generating part 402e generates a Web page for a user to browse with the client apparatus 200. The sending part 402f sends the information such as the metabolic syndrome state information and the multivariate discriminant to the metabolic syndrome-evaluating apparatus 100.

### 2-3. Processing in the present system

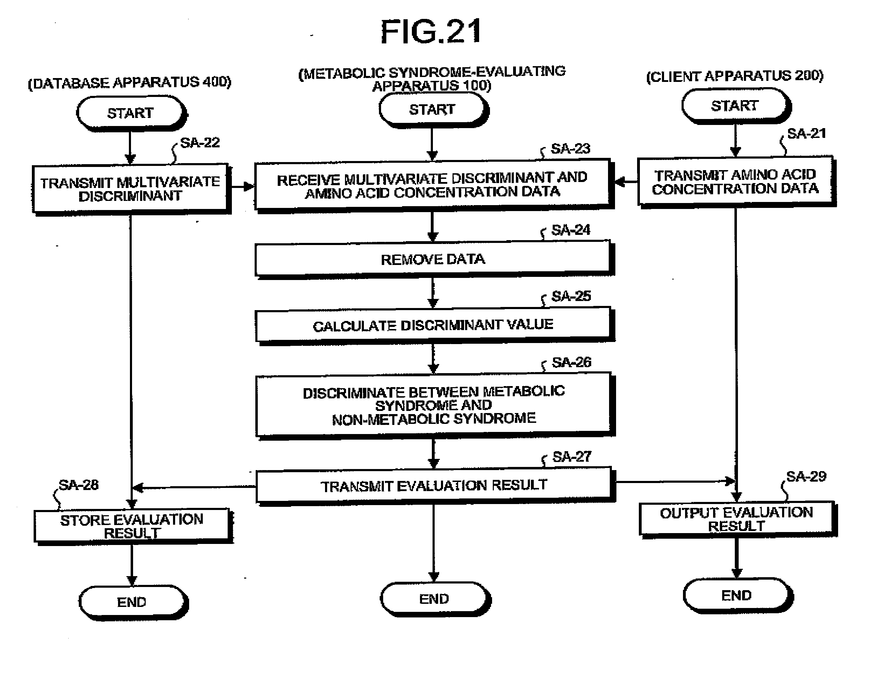

Here, an example of the metabolic syndrome evaluation service processing performed in the present system constituted as described above will be described with reference to FIG. 21. FIG. 21 is a flowchart showing an example of the metabolic syndrome evaluation service processing.

The amino acid concentration data used in the present processing concerns amino acid concentration value obtained by analyzing blood previously collected from an individual. Hereinafter, the method of analyzing blood amino acid will be described briefly. First, a blood sample is collected in a heparin-treated tube, and then the blood plasma is separated by centrifugation of the tube. All blood plasma samples separated are frozen and stored at -70°C before measurement of amino acid concentration. Before measurement of amino acid concentration, the blood plasma sample is deproteinized by adding sulfosalicylic acid to a concentration of 3%. An amino acid analyzer by high-performance liquid chromatography (HPLC) by using ninhydrin reaction in the post column was used for measurement of amino acid concentration.

First, the client apparatus 200 accesses the metabolic syndrome-evaluating apparatus 100 when the user specifies the Web site address (such as URL) provided from the metabolic syndrome-evaluating apparatus 100, via the input device 250 on the screen displaying Web browser 211. Specifically, when the user instructs update of the Web browser 211 screen on the client apparatus 200, the Web browser 211 sends the Web site's address provided from the metabolic syndrome-evaluating apparatus 100 by a particular protocol, thereby transmitting a request demanding transmission of the Web page corresponding to the amino acid concentration data transmission screen to the metabolic syndrome-evaluating apparatus 100 based on the routing of the address.

Then, upon receipt of the request from the client apparatus 200, the request-interpreting part 102a in the metabolic syndrome-evaluating apparatus 100 analyzes the transmitted request and sends the request to other parts in the control device 102 according to the analytical result. Specifically, when the transmitted request is a request to send the Web page corresponding to the amino acid concentration data transmission screen, mainly the browsing processing part 102b in the metabolic syndrome-evaluating apparatus 100 obtains the Web data for display of the Web page stored in a predetermined region of the memory device 106 and sends the obtained Web data to the client apparatus 200. More specifically, upon receiving the Web page transmission request corresponding to the amino acid concentration data transmission screen by the user, the control device 102 in the metabolic syndrome-evaluating apparatus 100 demands input of user ID and user password from the user. If the user ID and password are input, the authentication-processing part 102c in the metabolic syndrome-evaluating apparatus 100 examines the input user ID and password by comparing them with the user ID and user password stored in the user information file 106a for authentication. Only when the user is authenticated, the browsing processing part 102b in the metabolic syndrome-evaluating apparatus 100 sends, to the client apparatus 200, the Web data for displaying the Web page corresponding to the amino acid concentration data transmission screen. The client apparatus 200 is identified with the IP (Internet Protocol) address transmitted from the client apparatus 200 together with the transmission request.

Then, the client apparatus 200 receives, in the receiving part 213, the Web data (for displaying the Web page corresponding to the amino acid concentration data transmission screen) transmitted from the metabolic syndrome-evaluating apparatus 100, interprets the received Web data with the Web browser 211, and displays the amino acid concentration data transmission screen on the monitor 261.

When the user inputs and selects, via the input device 250, for example the amino acid concentration data of the individual on the amino acid concentration data transmission screen displayed on the monitor 261, the sending part 214 of the client apparatus 200 sends an identifier for identifying input information and selected items to the metabolic syndrome-evaluating apparatus 100, thereby transmitting the amino acid concentration data of the individual as the subject to be evaluated to the metabolic syndrome-evaluating apparatus 100 (step SA-21). In step SA-21, transmission of the amino acid concentration data may be realized for example by using an existing file transfer technology such as FTP (File Transfer Protocol).

Then, the request-interpreting part 102a of the metabolic syndrome-evaluating apparatus 100 interprets the identifier transmitted from the client apparatus 200 thereby analyzing the request from the client apparatus 200, and requests the database apparatus 400 to send the multivariate discriminant for metabolic syndrome evaluation (specifically for discrimination of the 2 groups of metabolic syndrome and non-metabolic syndrome) containing at least one or two of Val, Leu, Ile, Tyr, Trp, Glu, Ala, Asp, Gly, Ser and Thr as variable(s).

Then, the request-interpreting part 402a of the database apparatus 400 interprets the transmission request from the metabolic syndrome-evaluating apparatus 100 and transmits, to the metabolic syndrome-evaluating apparatus 100, the multivariate discriminant (for example, the updated newest multivariate discriminant) containing at least one or two of Val, Leu, Ile, Tyr, Trp, Glu, Ala, Asp, Gly, Ser and Thr as variable(s), stored in a predetermined region of the memory device 406 (step SA-22).

In step SA-22, the multivariate discriminant to be transmitted to the metabolic syndrome-evaluating apparatus 100 may be expressed by one fractional expression or the sum of a plurality of the fractional expressions and may contain either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant. Specially, the multivariate discriminant may be formula 1:

Thr/Ser+(Glu+Ala)/Gly (formula 1)

In step SA-22, the multivariate discriminant to be transmitted to the metabolic syndrome-evaluating apparatus 100 may be any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree. Specially, the multivariate discriminant may contain Glu, Gly, Ala, Thr and Ser as the variables.

Then, the metabolic syndrome-evaluating apparatus 100 receives, in the receiving part 102f, the amino acid concentration data of the individual transmitted from the client apparatuses 200, receives the multivariate discriminant transmitted from the database apparatus 400, stores the received amino acid concentration data in a predetermined memory region of the amino acid concentration data file 106b, and stores the received multivariate discriminant in a predetermined memory region of a multivariate discriminant file 106e4 (step SA-23).

In the control device 102 of the metabolic syndrome-evaluating apparatus 100, data containing defective and outliers is then removed from the amino acid concentration data of the individual received in step SA-23 (step SA-24).

Then, the metabolic syndrome-evaluating apparatus 100 calculates a discriminant value in the discriminant value-calculating part 102i, based on the multivariate discriminant received in step SA-23 and at least one or two concentrations of Val, Leu, Ile, Tyr, Trp, Glu, Ala, Asp, Gly, Ser and Thr contained in the amino acid concentration data of the individual from which defective and outliers have been removed in step SA-24 (step SA-25).

Then, the discriminant value criterion-discriminating part 102j1 of the metabolic syndrome-evaluating apparatus 100 compares the discriminant value calculated in step SA-25 with a previously established threshold (cutoff value), thereby discriminating between metabolic syndrome and non-metabolic syndrome in the subject to be evaluated, and stores the discrimination results in a predetermined memory region of the evaluation result file 106g (step SA-26).

The sending part 102m of the metabolic syndrome-evaluating apparatus 100 then sends the discrimination results (discrimination results as to discrimination between metabolic syndrome and non-metabolic syndrome) obtained in step SA-26 to the client apparatus 200 that has sent the amino acid concentration data and to the database apparatus 400 (step SA-27). Specifically, the metabolic syndrome-evaluating apparatus 100 first generates a Web page for display of discrimination results in the Web page-generating part 102e and stores the Web data corresponding to the generated Web page, in a predetermined memory region of the memory device 106. Then, the user is authenticated as described above by inputting a predetermined URL (Uniform Resource Locator) into the Web browser 211 of the client apparatus 200 via the input device 250, and the client apparatus 200 sends a Web page browsing request to the metabolic syndrome-evaluating apparatus 100. The metabolic syndrome-evaluating apparatus 100 then examines the browsing request transmitted from the client apparatus 200 in the browsing processing part 102b and reads the Web data corresponding to the Web page for displaying the discrimination results, out of the predetermined memory region of the memory device 106. The sending part 102m of the metabolic syndrome-evaluating apparatus 100 then sends the read-out Web data to the client apparatus 200 and simultaneously sends the Web data or the discrimination results to the database apparatus 400.

In step SA-27, the control device 102 of the metabolic syndrome-evaluating apparatus 100 may notify the discrimination results to the user client apparatus 200 by electronic mail. Specifically, the metabolic syndrome-evaluating apparatus 100 first acquires the user electronic mail address in the electronic mail-generating part 102d at the transmission timing for example based on the user ID, with reference to the user information stored in the user information file 106a. The metabolic syndrome-evaluating apparatus 100 then generates electronic mail data including user name and discrimination result, with the electronic mail address obtained as its mail address in the electronic mail-generating part 102d. The sending part 102m of the metabolic syndrome-evaluating apparatus 100 then sends the generated data to the user client apparatus 200.

Also in step SA-27, the metabolic syndrome-evaluating apparatus 100 may send the discrimination results to the user client apparatus 200 by using an existing file transfer technology such as FTP.

Returning to FIG. 21, the control device 402 in the database apparatus 400 receives the discrimination results or the Web data transmitted from the metabolic syndrome-evaluating apparatus 100 and stores (accumulates) the received discrimination results or Web data in a predetermined memory region of the memory device 406 (step SA-28).

The receiving part 213 of the client apparatus 200 receives the Web data transmitted from the metabolic syndrome-evaluating apparatus 100, and the received Web data are interpreted with the Web browser 211, to display on the monitor 261 the Web page screen displaying the discrimination result of the individual (step SA-29). When the discrimination results are sent from the metabolic syndrome-evaluating apparatus 100 by electronic mail, the electronic mail transmitted from the metabolic syndrome-evaluating apparatus 100 is received at any timing, and the received electronic mail is displayed on the monitor 261 with the known function of the electronic mailer 212 of the client apparatus 200.

In this way, the user knows discrimination results as to discrimination of 2 groups of metabolic syndrome and non-metabolic syndrome in the individual, by browsing the Web page displayed on the monitor 261. The user can print out the content of the Web page displayed on the monitor 261 by a printer 262.

When the discrimination results are transmitted by electronic mail from the metabolic syndrome-evaluating apparatus 100, the user reads the electronic mail displayed on the monitor 261, whereby the user can confirm discrimination results as to discrimination of 2 groups of metabolic syndrome and non-metabolic syndrome in the individual. The user may print out the content of the electronic mail displayed on the monitor 261 by the printer 262.

Given the foregoing description, the explanation of the metabolic syndrome evaluation service processing is finished.

### 2-4. Summary of the second embodiment and other embodiments

According to the metabolic syndrome-evaluating system described above in detail, the client apparatus 200 sends the amino acid concentration data of the individual to the metabolic syndrome-evaluating apparatus 100, and upon receiving a request from the metabolic syndrome-evaluating apparatus 100, the database apparatus 400 transmits the multivariate discriminant for discrimination between the 2 groups to the metabolic syndrome-evaluating apparatus 100. By the metabolic syndrome-evaluating apparatus 100, (1) amino acid concentration data are received from the client apparatus 200, and simultaneously the multivariate discriminant is received from the database apparatus 400, (2) data containing defective and outliers is removed from the received amino acid concentration data of the individual, (3) a discriminant value is calculated based on the amino acid concentration data of the individual from which defective and outliers have been removed and the received multivariate discriminant, (4) the calculated discriminant value is compared with a previously established threshold, thereby discriminating between metabolic syndrome and non-metabolic syndrome in the individual, and (5) this discrimination result is transmitted to the client apparatus 200 and database apparatus 400. Then, the client apparatus 200 receives and displays the discrimination result transmitted from the metabolic syndrome-evaluating apparatus 100, and the database apparatus 400 receives and stores the discrimination result transmitted from the metabolic syndrome-evaluating apparatus 100. Thus, a discriminant value obtained in a multivariate discriminant using amino acid variables useful for discriminating between the 2 groups of metabolic syndrome and non-metabolic syndrome can be utilized to bring about an effect of enabling accurate discrimination between the 2 groups of metabolic syndrome and non-metabolic syndrome.

According to the metabolic syndrome-evaluating system, the multivariate discriminant may be expressed by one frictional expression or the sum of a plurality of the fractional expressions and may contain either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant. Specially, the multivariate discriminant may be formula 1. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

Thr/ser+(Glu+Ala)/Gly (formula 1)

According to the metabolic syndrome-evaluating system, the multivariate discriminant may be any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree. Specially, the multivariate discriminant may contain Glu, Gly, Ala, Thr and Ser as the variables. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and noun-metabolic syndrome.

The multivariate discriminants described above can be prepared by a method described in International Publication WO 2004/052191 that is an international application filed by the present applicant or by a method (multivariate discriminant-preparing processing described below) described in International Publication PCT/JP2006/304398 that is an international application filed by the present applicant. Any multivariate discriminants obtained by these methods can be preferably used in evaluation of the state of metabolic syndrome, regardless of the unit of amino acid concentration in the amino acid concentration data as input data.

In addition to the second embodiment described above, the metabolic syndrome-evaluating apparatus, the metabolic syndrome-evaluating method, the metabolic syndrome-evaluating system, the metabolic syndrome-evaluating program and the recording medium according to the present invention can be practiced in various different embodiments within the technological scope of the claims. For example, among the processings described in the second embodiment above, all or a part of the processings described above as performed automatically may be performed manually, and all or a part of the manually conducted processings may be performed automatically by known methods. In addition, the processing procedure, control procedure, specific name, various registered data, information including parameters such as retrieval condition, screen, and database configuration shown in the description above or drawings may be modified arbitrarily, unless specified otherwise. For example, the components of the metabolic syndrome-evaluating apparatus 100 shown in the figures are conceptual and functional and may not be the same physically as those shown in the figure. In addition, all or a part of the operational function of each component and each device in the metabolic syndrome-evaluating apparatus 100 (in particular, processings in control device 102) may be executed by the CPU (Central Processing Unit) or the programs executed by the CPU, and may be realized as wired-logic hardware.

The "program" is a data processing method written in any language or by any description method and may be of any format such as source code or binary code. The "program" may not be configured singly, and may be operated together with plurality of modules and libraries or with a different program such as OS (Operating System) to achieve the function. The program is stored on a recording medium and read mechanically as needed by the metabolic syndrome-evaluating apparatus 100. Any well-known configuration or procedure may be used for reading the programs recorded on the recording medium in each apparatus and for reading procedure and installation of the procedure after reading.

The "recording media" includes any "portable physical media", "fixed physical media", and "communication media". Examples of the "portable physical media" include flexible disk, magnetic optical disk, ROM, EPROM (Erasable Programmable Read Only Memory), EEPROM (Electronically Erasable and Programmable Read Only Memory), CD-ROM (Compact Disk Read Only Memory), MO (Magneto-Optical disk), DVD (Digital Versatile Disk), and the like. Examples of the "fixed physical media" include various media installed in a computer system such as ROM, RAM, and HD. The "communication media" for example stores the program for a short period of time such as communication line and carrier wave when the program is transmitted via a network such as LAN (Local Area Network), WAN (Wide Area Network), or the Internet.

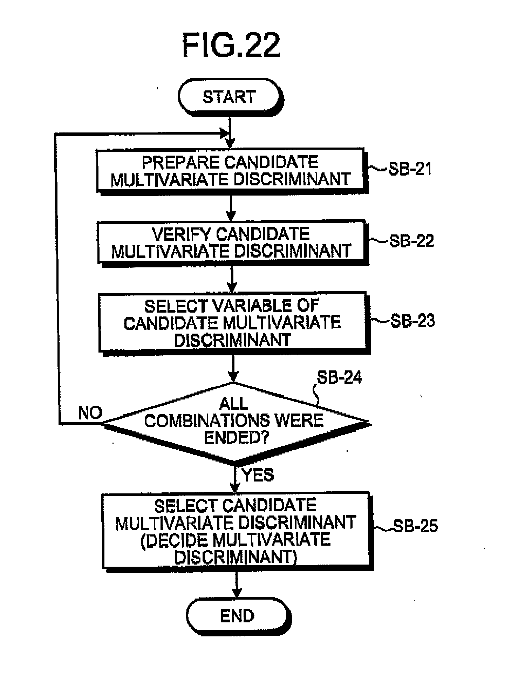

Finally, an example of the multivariate discriminant-preparing processing performed in the metabolic syndrome-evaluating apparatus 100 is described in detail with reference to FIG. 22. FIG. 22 is a flowchart showing an example of the multivariate discriminant-preparing processing. The multivariate discriminant-preparing processing may be performed in the database apparatus 400 handling the metabolic syndrome state information.

In the present description, the metabolic syndrome-evaluating apparatus 100 stores the metabolic syndrome state information previously obtained from the database apparatus 400 in a predetermined memory region of the metabolic syndrome state information file 106c. The metabolic syndrome-evaluating apparatus 100 shall store, in a predetermined memory region of the designated metabolic syndrome state information file 106d, the metabolic syndrome state information including the metabolic syndrome state index data and amino acid concentration data designated previously in the metabolic syndrome state information-designating part 102g.

According to a predetermined discriminant-preparing method, the candidate multivariate discriminant-preparing part 102h1 in the multivariate discriminant-preparing part 102h first prepares a candidate multivariate discriminant from the metabolic syndrome state information stored in a predetermine memory region of the designated metabolic syndrome state information file 106d, and the prepare candidate multivariate discriminate is stored in a predetermined memory region of the candidate multivariate discriminant file 106el (step SB-21). Specifically, the candidate multivariate discriminant-preparing part 102h1 in the multivariate discriminant-preparing part 102h first selects a desired method out of a plurality of different discriminant-preparing methods (including multivariate analysis methods such as principal component analysis, discriminant analysis, support vector machine, multiple regression analysis, logistic regression analysis, k-means method, cluster analysis, and decision tree and the like) and determines the form of the candidate multivariate discriminant to be prepared based on the selected discriminant-preparing method. The candidate multivariate discriminant-preparing part 102h1 in the multivariate discriminant-preparing part 102h then performs various calculation corresponding to the selected function-selecting method (e.g., average or variance), based on the metabolic syndrome state information. The candidate multivariate discriminant-preparing part 102h1 in the multivariate discriminant-preparing part 102h then determines the parameters for the calculation result and the determined candidate multivariate discriminant. In this way, a candidate multivariate discriminant is generated based on the selected discriminant-preparing method. When candidate multivariate discriminants are generated simultaneously and concurrently (in parallel) by using a plurality of different discriminant-preparing methods in combination, the processings described above may be executed concurrently for each selected discriminant-preparing method. Alternatively when candidate multivariate discriminants are to be generated in series by using a plurality of different discriminant-preparing methods in combination, for example, candidate multivariate discriminants may be generated by converting metabolic syndrome state information with a candidate multivariate discriminant prepared by performing principal component analysis and performing discriminant analysis of the converted metabolic syndrome state information.

The candidate multivariate discriminant-verifying part 102h2 in the multivariate discriminant-preparing part 102h verifies (mutually verifies) the candidate multivariate discriminant prepared in step SB-21 according to a particular verification method and stores the verification result in a predetermined memory region of verification result file 106e2 (step SB-22). Specifically, the candidate multivariate discriminant-verifying part 102h2 in the multivariate discriminant-preparing part 102h first generates the verification data to be used in verification of the candidate multivariate discriminant, based on the metabolic syndrome state information stored in a predetermined memory region of the designated metabolic syndrome state information file 106d, and verifies the candidate multivariate discriminant according to the generated verification data. If a plurality of candidate multivariate discriminants are generated by using a plurality of different discriminant-preparing methods in step SB-21, the candidate multivariate discriminant-verifying part 102he in the multivariate discriminant-preparing part 102h verifies each candidate multivariate discriminant corresponding to each discriminant-preparing method according to a particular verification method. Here in step SB-22, at least one of the discrimination rate, sensitivity, specificity, information criterion, and the like of the candidate multivariate discriminant may be verified based on at least one method of the bootstrap, holdout, leave-one-out, and other methods. Thus, it is possible to select a candidate multivariate discriminant higher in predictability or reliability, based on the metabolic syndrome state information and diagnostic condition.

Then, the variable-selecting part 102h3 in the multivariate discriminant-preparing part 102h selects the combination of amino acid concentration data contained in the metabolic syndrome state information to be used in preparing the candidate multivariate discriminant by selecting a variable of the candidate multivariate discriminant from the verification results in step SB-22 according to a particular variable selection method, and stores the metabolic syndrome state information including the selected combination of amino acid concentration data in a predetermined memory region of the selected metabolic syndrome state information file 106e3 (step SB-23). When a plurality of candidate multivariate discriminants are generated by using a plurality of different discriminant-preparing methods in step SB-21 and each candidate multivariate discriminant corresponding to each discriminant-preparing method is verified according to a particular verification method in step SB-22, the variable-selecting part 102h3 in the multivariate discriminant-preparing part 102h selects the variable of the candidate multivariate discriminant for each candidate multivariate -discriminant corresponding to the verification result obtained in step SB-22, according to a particular variable selection method in step SB-23. Here in step SB-23, the variable of the candidate multivariate discriminant may be selected from the verification results according to at least one of stepwise method, best path method, local search method, and genetic algorithm. The best path method is a method of selecting a variable by optimizing the evaluation index of the candidate multivariate discriminant while eliminating the variables contained in the candidate multivariate discriminant one by one. In step SB-23, the variable-selecting part 102h3 in the multivariate discriminant-preparing part 102h may select the combination of amino acid concentration data based on the metabolic syndrome state information stored in a predetermined memory region of the designated metabolic syndrome state information file 106d.

The multivariate discriminant-preparing part 102h then judges whether all combinations of the amino acid concentration data contained in the metabolic syndrome state information stored in a predetermined memory region of the designated metabolic syndrome state information file 106d are processed, and if the judgment result is "End" (Yes in step SB-24), the processing advances to the next step (step SB-25), and if the judgment result is not "End" (No in step SB-24), it returns to step SB-21. The multivariate discriminant-preparing part 102h judges whether the processing is performed a predetermined number of times, and if the judgment result is "End" (Yes in step SB-24), the processing may advance to the next step (step SB-25), and if the judgment result is not "End" (No in step SB-24), it returns to step SB-21. The multivariate discriminant-preparing part 102h may judge whether the combination of the amino acid concentration data selected in step SB-23 is the same as the combination of the amino acid concentration data contained in the metabolic syndrome state information stored in a predetermined memory region of the designated metabolic syndrome state information file 106d or the combination of the amino acid concentration data selected in the previous step SB-23, and if the judgment result is "the same" (Yes in step SB-24), the processing may advance to the next step (step SB-25) and if the judgment result is not "the same" (No in step SB-24), it may return to step SB-21. If the verification result is specifically the evaluation value for each multivariate discriminant, the multivariate discriminant-preparing part 102h may advance to step SB-25 or return to step Sub-21, based on the comparison of the evaluation value with a particular threshold corresponding to each discriminant-preparing method.

Then, the multivariate discriminant-preparing part 102h determines the multivariate discriminant based on the verification results by selecting a candidate multivariate discriminant to be used as the multivariate discriminant among the candidate multivariate discriminants, and stores the determined multivariate discriminant (selected candidate multivariate discriminant) in particular memory region of the multivariate discriminant file 106e4 (step SB-25). Here, in step SB-25, for example, the optimal multivariate discriminant may be selected from the candidate multivariate discriminants prepared by the same discriminant-preparing method or from all candidate multivariate discriminants.

These are description of the multivariate discriminant-preparing processing.

### Third Embodiment

### 3-1. outline of the Invention

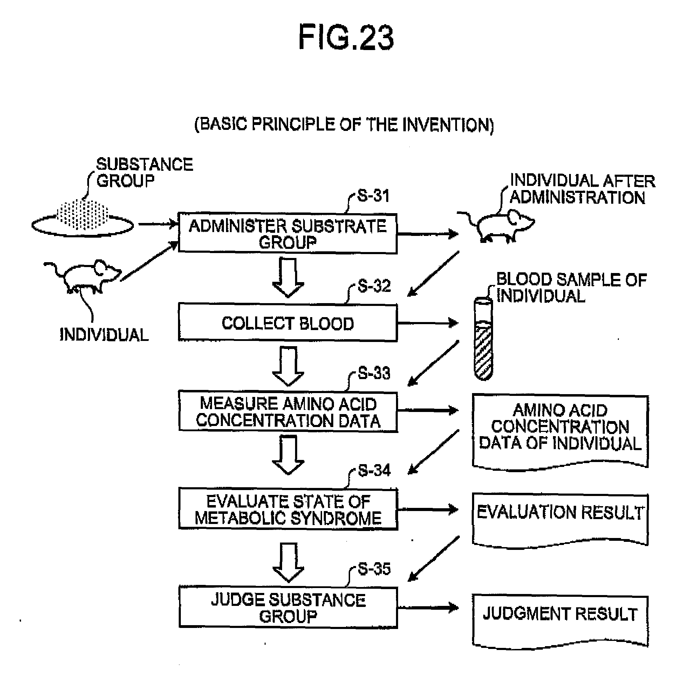

Herein, the method of searching for prophylactic/ameliorating substance for metabolic syndrome according to the present invention is described in detail with reference to FIG. 23. FIG. 23 its a principle configurational diagram showing the basic principle of the present invention.

First, a desired substance group consisting of one or more substances is administered to a subject to be evaluated (for example, an individual such as animal or human) (step S-31). For example, a suitable combination of an existing drug, amino acid, food and supplement capable of administration to humans (for example, a suitable combination of a drug, supplement and anti-obesity drug that are known to be effective in amelioration of various symptoms of metabolic syndrome) may be administered over a predetermined period (for example in the range of 1 day to 12 months) in a predetermined amount at predetermined frequency and timing (for example 3 times per day, after food) by a predetermined administration method (for example, oral administration). The administration method, dose, and dosage form may be suitably combined depending on the condition of a patient. The dosage form may be determined based on techniques known in the art. The dose is not particularly limited, and for example, a drug containing 1 µg to 100 g active ingredient may be given.

From the subject administered with the substance group in step S-31, blood is then collected (step S-32).

From the blood collected in step S-32, amino acid concentration data on the concentration values of amino acids are measured (step S-33). The concentrations of amino acids in blood may be analyzed in the following manner. A blood sample is collected in a heparin-treated tube, and then the blood plasma is separated by centrifugation of the collected blood sample. All blood plasma samples separated were frozen and stored at -70°C before measurement of amino acid concentration. Before measurement of amino acid concentration, the blood samples is defrost, and the blood plasma sample is deproteinized by adding sulfosalicylic acid to a concentration of 3%. An amino acid analyzer by high-performance liquid chromatography (HPLC) by using ninhydrin reaction in the post column is used for measurement of amino acid concentration.

Then, the state of metabolic syndrome in a subject to be evaluated is evaluated based on the amino acid concentration data of the subject to be evaluated measured in the step S-33 (step S-34).

Then, whether the desired substance group administered in step S-31 prevents or ameliorates metabolic syndrome is judged based on the evaluation result in the step S-34 (step S-35).

When the judgment result in step S-35 is "preventive or ameliorative", the substance group administered in step S-31 is searched as one preventing or ameliorating metabolic syndrome.

According to the present invention, a desired substance group is administered to a subject to be evaluated, blood is collected from the subject, amino acid concentration data on the concentration values of amino acids is measured, and the state of metabolic syndrome in the subject is evaluated based on the measured amino acid concentration data, and it is judged whether the desired substance group prevents or ameliorates metabolic syndrome based on the evaluation results. Thus, the metabolic syndrome evaluation method capable of accurately evaluating the state of metabolic syndrome by utilizing the concentrations of amino acids in blood can be used to bring about an effect of enabling accurate search for a substance for preventing or ameliorating metabolic syndrome.

Before step S-34 is executed, data containing defective and outliers may be removed from the amino acid concentration data. Thereby, the state of metabolic syndrome can be more accurately evaluated.

In step S-34, the state of metabolic syndrome in the subject may be evaluated based on the concentration value of at least one of Val, Leu, Ile, Tyr, Trp, Glu, Ala, Asp, Gly, Ser and Thr contained in the amino acid concentration data of the subject measured in step S-33. Thus, the concentrations of the amino acids which among amino acids in blood, are related to the state of metabolic syndrome can be utilized to bring about an effect of enabling accurate evaluation of the state of metabolic syndrome.

In step S-34, the subject may be discriminated between metabolic syndrome and non-metabolic syndrome based on the concentration value of at least one of Val, Leu, Ile, Tyr, Trp, Glu, Ala, Asp, Gly, Ser and Thr contained in the amino acid concentration data of the subject measured in step S-33, Specifically, at least one concentration value of Val, Leu, Ile, Tyr, Trp, Glu, Ala, Asp, Gly, Ser and Thr may be compared with a previously established threshold (cutoff value), thereby discriminating between metabolic syndrome and non-metabolic syndrome in the subject. Thus, the concentrations of the amino acids which among amino acids in blood, are useful for discriminating between the 2 groups of metabolic syndrome and non-metabolic syndrome can be utilized to bring about an effect of enabling accurate discrimination between the 2 groups of metabolic syndrome and non-metabolic syndrome.

In step S-34, the subject may be discriminated between metabolic syndrome and non-metabolic syndrome based on the concentration values of at least two of Val, Leu, Ile, Tyr, Trp, Glu, Ala, Asp, Gly, Ser and Thr contained in the amino acid concentration data of the subject measured in step S-33. Specifically, at least two of Val, Leu, Ile, Tyr, Trp, Glu, Ala, Asp, Gly, Ser and Thr may be compared with a previously established threshold (cutoff value), thereby discriminating between metabolic syndrome and non-metabolic syndrome in the subject. Thus, the concentrations of the amino acids which among amino acids in blood, are useful for discriminating between the 2 groups of metabolic syndrome and non-metabolic syndrome can be utilized to bring about an effect of enabling accurate discrimination between the 2 groups of metabolic syndrome and non-metabolic syndrome.

In step S-34, a discriminant value that is a value of multivariate discriminant may be calculated based on both the amino acid concentration data of the subject measured in step S-33 and a previously established multivariate discriminant with the concentration of the amino acid as a variable, and the state of metabolic syndrome in the subject may be evaluated based on the discriminant value calculated. Thus, a discriminant value obtained in a multivariate discriminant wherein the concentrations of amino acids are variables can be utilized to bring about an effect of enabling accurate evaluation of the state of metabolic syndrome.

In step S-34, the discriminant value may be calculated based on both the concentration value of at least one of Val, Leu, Ile, Tyr, Trp, Glu, Ala, Asp, Gly, Ser and Thr contained in the amino acid concentration data of the subject measured in step S-33 and the multivariate discriminant containing at least one of Val, Leu, Ile, Tyr, Trp, Glu, Ala, Asp, Gly, Ser and Thr as the variable. Thus, a discriminant value obtained in a multivariate discriminant correlated significantly with the state of metabolic syndrome can be utilized to bring about an effect of enabling accurate evaluation of the state of metabolic syndrome.

In step S-34, the subject may be discriminated between metabolic syndrome and non-metabolic syndrome based on the discriminant value calculated. Specifically, the discriminant value may be compared with a previously established threshold (cutoff value), thereby discriminating between metabolic syndrome and non-metabolic syndrome in the subject. Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

In step S-34, the discriminant value may be calculated based on both the concentration values of at least two of Val, Leu, Ile, Tyr, Trp, Glu, Ala, Asp, Gly, Ser and Thr contained in the amino acid concentration data of the subject measured in step S-33 and the multivariate discriminant containing at least two of Val, Leu, Ile, Tyr, Trp, Glu, Ala, Asp, Gly, Ser and Thr as the variables. Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

The multivariate discriminant may be expressed by one fractional expression or the sum of a plurality of the fractional expressions and may contain either at least one of Val, Leu, Ile, Tyr, Trp, Glu, Alma, Asp and Thr as the variable in the numerator and at least one of Gly and Ser as the variable in the denominator or at least one of Gly and Ser as the variable in the numerator and at least one of Val, Leu, Ile, Thr, Trp, Glu, Ala, Asp and Thr as the variable in the denominator, in the fractional expression constituting the multivariate discriminant. Specially, the multivariate discriminant may be formula 1. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

Thr/Ser+(Glu+Ala)/Gly (formula 1)

The multivariate discriminant may be any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree. Specially, the multivariate discriminant may contain Glu, Gly, Ala, Thr and Ser as the variables. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

The multivariate discriminants described above can be prepared by a method described in International Publication WO 2004/052191 that is an international application filed by the present applicant or by a method (multivariate discriminant-preparing processing described in the second embodiment described above) described in International Publication PCT/JP2006/304398 that is an international application filed by the present applicant. Any multivariate discriminants obtained by these methods can be preferably used in evaluation of the state of metabolic syndrome, regardless of the unit of amino acid concentration in the amino acid concentration data as input data.

In a fractional expression, the numerator of the fractional expression is expressed by the sum of amino acids A, B, C etc. and/or the denominator of the fractional expression is expressed by the sum of amino acids a, b, c etc. The fractional expression also includes the sum of fractional expressions α, β, γ etc. (for example, α+β) having such constitution. The fractional expression also includes divided fractional expressions. Amino acids used in the numerator or denominator may have suitable coefficients respectively. The amino acids used in the numerator or denominator may appear repeatedly. Each fractional expression may have a suitable coefficient. The value of a coefficient for each variable and the value for a constant term may be any real numbers.

Specifically, the fractional expression has a combination of amino acid concentrations in Groups A to D below, and is preferably "represented by one or more fractional expressions containing 2 to 8 amino acid concentration variables, wherein the numerator has (1 or more variables from Groups A and B + 0 to 2 variables from Group D) and the denominator has (1 or more variables from Group C + 0 to 2 variables from Group D), more preferably "represented by 2 or more fractional expressions containing 4 to 8 amino acid concentration variables, wherein the numerator has (1 or more variables from Group B + 0 to 1 variable from Group A + 0 to 2 variables from Group D) and the denominator has (1 or more variables from Group C + 0 to 2 variables from Group D). In a combination where variables in the numerator and variables in the denominator in the fractional expression are switched with each other, the positive (or negative) sign is generally reversed in correlation with objective variables, but because their correlation is maintained, such combination can be assumed to be equivalent to the original, and thus the fractional expression may include such combination.
Group A: Leu, Ile, Val
Group B: Ala, Glu, Asp, Tyr, Trp, Thr
Group C: Gly, Ser
Group D: amino acids (or amino acid metabolites thereof) not contained in Groups A, B and

The multivariate discriminant refers to a form of equation used generally in multivariate analysis and includes, for example, multiple regression equation, multiple logistic regression equation, linear discriminant function, Mahalanobis' generalized distance, canonical discriminant function, support vector machine, and decision tree. The multivariate discriminant also includes an equation shown by the sum of different forms of multivariate discriminants. In the multiple regression equation, multiple logistic regression equation and canonical discriminant function, a coefficient and constant term are added to each variable, and the coefficient and constant term in this case are preferably real numbers, more preferably values in the range of 99% confidence interval for the coefficient and constant term obtained from data for discrimination, more preferably in the range of 95% confidence interval for the coefficient and constant term obtained from data for discrimination. The value of each coefficient and the confidence interval thereof may be those multiplied by a real number, and the value of each constant term and the confidence interval thereof may be those having an arbitrary actual constant added or subtracted or those multiplied or divided by an arbitrary actual constant.

When the state of metabolic syndrome is evaluated (specifically discrimination between metabolic syndrome and non-metabolic syndrome) in the present invention, the concentrations of other metabolites (biological metabolites), the protein expression level, the age and sex of the subject, biological indices or the like may be used in addition to the amino acid concentration data. When the state of metabolic syndrome is evaluated (specifically discrimination between metabolic syndrome and non-metabolic syndrome) in the present invention, the concentrations of other metabolites (biological metabolites), the protein expression level, the age and sex of the subject, biological indices or the like may be used as variables in the multivariate discriminant in addition to the amino acid concentration.

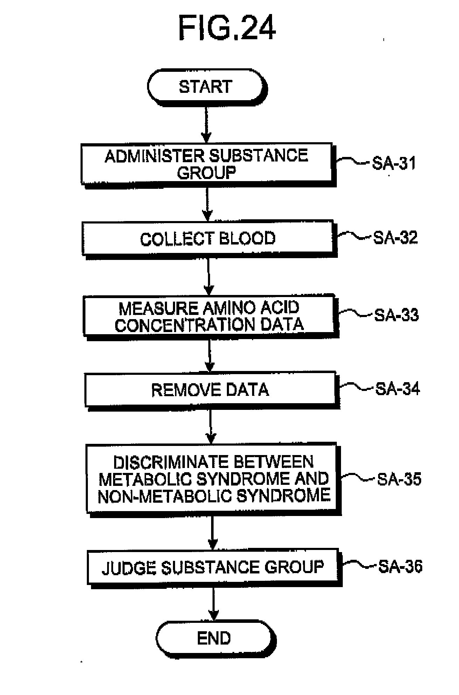

3-2. An example of the method of searching for prophylactic/ameliorating substance for metabolic syndrome according to the third embodiment
Here, an example of the method of searching for prophylactic/ameliorating substance for metabolic syndrome according to the third embodiment will be described with reference to FIG. 24. FIG. 24 is a flowchart showing an example of the method of searching for prophylactic/ameliorating substance for metabolic syndrome according to the third embodiment.

First, a desired substance group consisting of one or more substances is administered to an individual with metabolic syndrome such as animal or human (step SA-31).

From the individual administered with the substance group in step S-31, blood is then collected (step SA-32).

From the blood collected in step S-32, amino acid concentration data on the concentration values of amino acids are measured (step SA-33). Measurement of the amino acid concentration values is conducted by the method described above.

From the amino acid concentration data of the individual measured in step S-33, data such as defective and outliers is then removed (step SA-34).

Then, the concentration value of at least one of Val, Leu, Ile, Tyr, Trp, Glu, Ala, Asp, Gly, Ser and Thr contained in the amino acid concentration data of the individual from which the data such as defective and outliers was removed in step SA-34 is compared with a previously established threshold (cutoff value), thereby discriminating between metabolic syndrome and non-metabolic syndrome in the individual (step SA-35).

Based on the discrimination results in step SA-35, it is then judged whether the substance group administered in step SA-31 prevents metabolic syndrome or ameliorates the state of metabolic syndrome (step SA-36).

When the judgment result in step SA-36 is "preventive or ameliorative", the substance group administered in step SA-31 is searched as one preventing or ameliorating metabolic syndrome. The substances searched by the searching method of the present invention include, for example, an amino acid group of Val, Leu, Ile, Tyre Trp, Glu, Ala, Asp, Gly, Ser and Thr.

### 3-3. Summary of the third embodiment and other embodiments

According to the method of searching for prophylactic/ameliorating substance for metabolic syndrome according to the third embodiment described in detail above, (1) a desired substance group is administered to an individual, (2) blood is collected from the individual administered with the substance group in (1), (3) amino acid concentration data are measured from the blood collected in (2), (4) data such as defective and outliers is removed from the amino acid concentration data of the individual measured, (5) the concentration value of at least one of Val, Leu, Ile, Tyr, Trp, Glu, Ala, Asp, Gly, Ser and Thr contained in the amino acid concentration data of the individual from which the data such as defective and outliers was removed is compared with a previously established threshold (cutoff value), thereby discriminating between metabolic syndrome and non-metabolic syndrome in the individual, and (6) based on the discrimination result in (5), it is judged whether the substance group administered in (1) prevents or ameliorates metabolic syndrome. Thus, the metabolic syndrome evaluation method capable of accurately evaluating a state of metabolic syndrome by utilizing the concentrations of amino acids useful for discriminating between the 2 groups of metabolic syndrome and non-metabolic syndrome can be used to bring about an effect of enabling accurate search for a substance for preventing or ameliorating metabolic syndrome.

In step SA-35, the subject may be discriminated between metabolic syndrome and non-metabolic syndrome based on the concentration values of at least two of Val, Leu, Ile, Tyr, Trp, Glu, Ala, Asp, Gly, Ser and Thr contained in the amino acid concentration data of the individual measured in step A-34. Thus, the concentrations of the amino acids which among amino acids in blood are useful for discriminating between the 2 groups of metabolic syndrome and non-metabolic syndrome can be utilized to bring about an effect of enabling accurate discrimination between the 2 groups of metabolic syndrome and non-metabolic syndrome.

In step SA-35, the discriminant value may be calculated based on both at least one concentration value of Val, Leu, Ile, Tyr, Trip, Glu, Ala, Asp, Gly Ser and Thr contained in the amino acid concentration data of the individual from which the data containing defective and outliers was removed in step SA-34 and the multivariate discriminant containing at least one of Val, Leu, Ile, Tyr, Trp, Glu, Ala, Asp, Gly, Ser and Thr as the variable, and the discriminant value calculated may be compared with a previously established threshold (cutoff value), thereby discriminating between metabolic syndrome and non-metabolic syndrome in the individual. Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

In step SA-35, the discriminant value may be calculated based on both at least two concentration values of Val, Leu, Ile, Tyr, Trp, Glu, Ala, Asp, Gly, Ser and Thr contained in the amino acid concentration data of the individual from which the data containing defective and outliers was removed in step SA-34 and the multivariate discriminant containing at least two of Val, Leu, Ile, Tyr, Trp, Glu, Ala, Asp, Gly, Ser and Thr as the variables, and the discriminant value calculated may be compared with a previously established threshold (cutoff value), thereby discriminating between metabolic syndrome and non-metabolic syndrome in the individual. Thus, a discriminant value obtained in a multivariate discriminant useful for discriminating between the 2 groups of metabolic syndrome and non-metabolic syndrome can be utilized to bring about an effect of enabling accurate discrimination between the 2 groups of metabolic syndrome and non-metabolic syndrome.

In step SA-35, the multivariate discriminant may be expressed by one fractional expression or the sum of a plurality of the fractional expressions and may contain either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant. Specially, the multivariate discriminant may be formula 1. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

Thr/Ser+(Glu+Ala)/Gly (formula 1)

In step SA-35, the multivariate discriminant may be any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree. Specially, the multivariate discriminant may contain Glu, Gly, Ala, Thr and Ser as the variables. Thus, a discriminant value obtained in a multivariate discriminant useful particularly for discriminating between the 2 groups of metabolic syndrome and non-metabolic syndrome can be utilized to bring about an effect of enabling more accurate discrimination between the 2 groups of metabolic syndrome and non-metabolic syndrome.

The multivariate discriminants described above can be prepared by a method described in International Publication WO 2004/052191 that is an international application filed by the present applicant or by a method (multivariate discriminant-preparing processing described in the second embodiment described later) described in International Publication PCT/JP2006/304398 that is an international application filed by the present applicant. Any multivariate discriminants obtained by these methods can be preferably used in evaluation of the state of metabolic syndrome, regardless of the unit of amino acid concentration in the amino acid concentration data as input data.

In the method of searching for prophylactic/ameliorating substance for metabolic syndrome according to the third embodiment, the concentration value of the amino acid group containing at least one of Val, Leu, Ile, Tyr, Trp, Glu, Alas Asp, Gly, Ser and Thr, or a substance that restores normal value to a discriminant value of each of the multivariate discriminants, can be selected by the metabolic syndrome evaluation method in the first embodiment or by the metabolic syndrome evaluating apparatus in the second embodiment.

In the method of searching for prophylactic/ameliorating substance for metabolic syndrome in the third embodiment, "searching for prophylactic/ameliorating substance" includes not only discovery of a novel substance effective in preventing and ameliorating metabolic syndrome, but also new discovery of use of a known substance in preventing and ameliorating metabolic syndrome, discovery of a novel composition consisting of a combination of existing drugs and supplements having efficacy expectable for prevention and amelioration of metabolic syndrome, discovery of the suitable usage, dose and combination described above to form them into a kit, presentation of a prophylactic and therapeutic menu including a diet, exercise etc., and presentation of a necessary change in menu for each individual by monitoring the effect of the prophylactic and therapeutic menu.

### Example 1

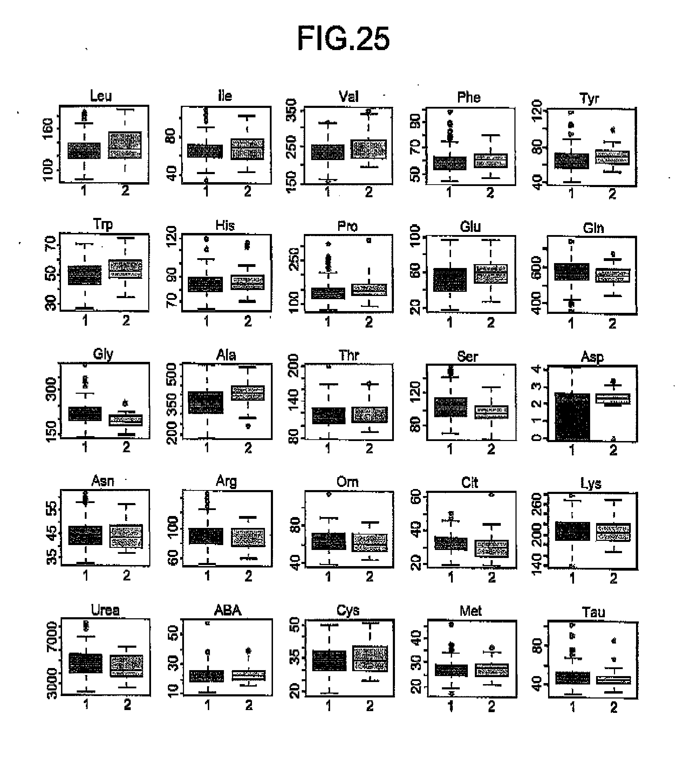

Based on the above-mentioned diagnostic criteria for Japanese metabolic syndrome established by the 8 related societies including Japanese Society of Internal Medicine, 205 subjects who had have a complete medical checkup were divided into a non-metabolic syndrome group (173 subjects) and a metabolic syndrome group (32 subjects). From their blood samples, the concentrations of amino acids in blood were measured by the amino acid analysis method described above. The 205 subjects did not include those under treatment of diseases such as hypertension and diabetes. FIG. 25 is boxplots showing the distribution of amino acid variables in the 2 groups of non-metabolic syndrome and metabolic syndrome (on the abscissa, non-metabolic syndrome group: 1, metabolic syndrome group: 2, and "ABA" in the graph is α-ABA (aminobutyric acid), and "Cys" is Cystine). For the purpose of discrimination between the 2 groups, t-test of the 2 groups was performed.

In the metabolic syndrome group as compared with the non-metabolic syndrome group, Val, Tyr, Trp, Glu, Ala and Asp were significantly increased (significant difference probability P<0.05), and Gly and Ser were significantly reduced. It was revealed that the amino acid variables Val, Tyr, Trp, Glu, Ala, Asp, Gly and Ser have an ability to discriminate between the 2 groups. The Pearson correlation coefficients (r) of branched amino acid Val had 0.867 and 0.797 to Leu and Ile respectively, and the Pearson correlation coefficient (r) between Leu and Ile was 0.869. It was revealed that the 3 variables Val, Leu and Ile have a similar ability to discriminate the 2 groups. As shown below in Examples 4 and 5, the variable Thr appears frequently (with a frequency of 100/100 in Example 4 and 95/100 in Example 5) in identified Multivariate discriminants superior in discrimination performance, and was revealed to be a variable having a high degree of contribution in the multivariate discriminants.

### Example 2

The sample data used in Example 1 were used in Example 2. Using a method described in International Publication WO 2004/052191 that is an international application filed by the present applicant, a multivariate discriminant for maximizing the performance of discriminating the 2 groups of non-metabolic syndrome and metabolic syndrome was extensively searched to give a plurality of multivariate discriminants having similar performance represented by index 1 (specifically, the index 1 is a multivariate discriminant consisting one expression or the sum of the fractional expressions in which the numerator of the fractional expression includes at least one amino acid variable from Val, Leu, Ile, Tyr, Trp, Glu, Ala, Asp and Thr, and the denominator of the fractional expression includes at least one amino acid variable from Gly and Ser). As an example of the multivariate discriminant, index 2 (Thr)/(Ser)+(Glu+Ala)/(Gly) was obtained.

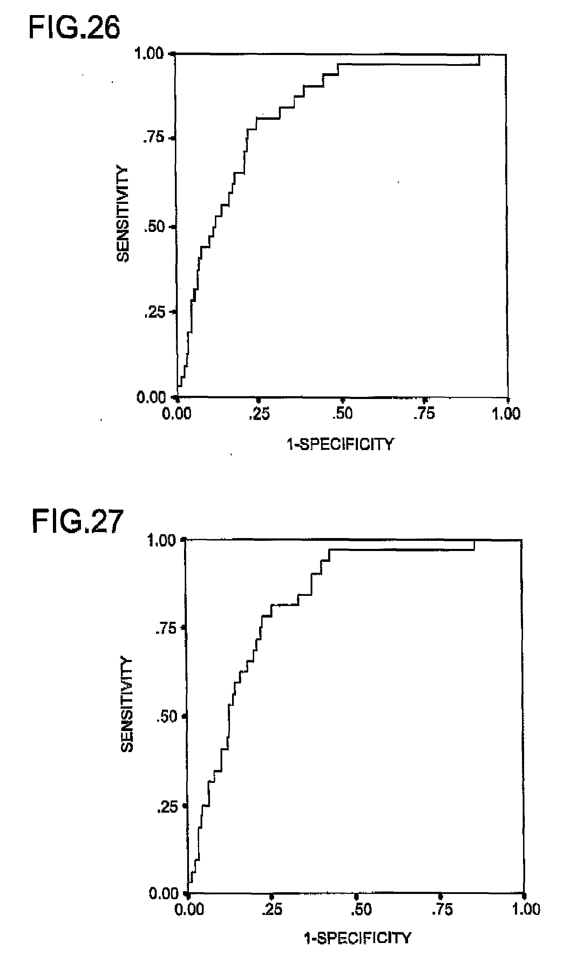

Discrimination of the 2 groups by the index 2 was evaluated by the AUC (area under the curve) of the ROC (receiver operating characteristic) curve (see FIG. 26), to give an AUC of 0.824±0.038 (95% confidence interval: 0.751 to 0.898).

### Example 3

The sample data used in Example 1 were used in Example 3. An index for maximizing the performance of discriminating the 2 groups of non-metabolic syndrome and metabolic syndrome was extensively searched by a method (a method of searching a multivariate discriminant) described in International Publication PCT/JP2006/304398 that is an international application filed by the present applicant. The method that can be used to search a multivariate discriminant includes a logistic regression equation, a linear discriminant, a support vector machine, and a Mahalanobis' generalized distance method.

The multivariate discriminant was extensively searched to give index 4 containing Glu, Gly, Ala, Thr and Ser (for example, at least one of a logistic regression, a linear discriminant, a support vector machine, and a Mahalanobis' generalized distance method) as an example of a plurality of multivariate discriminants having almost the same discrimination performance represented by index 3 (specifically, the index 3 is one including at least one amino acid variable from Val, Leu, Ile, Tyr, Trp, Glu, Ala, Asp, Gly, Ser and Thr (for example, at least one of a logistic regression, a linear discriminant, a support vector machine, and a Mahalanobis' generalized distance method)). As one example, a logistic regression equation containing Glu, Gly, Ala, Thr and ser as index 4 (numerical coefficients of amino acid variables Glu, Gly, Ala, Thr and Ser and the constant term were 0.020±0.013, -0.028±0.009, 0.011±0.004, 0.023±0.012, -0.029±0.017, and -1.043±2.283, respectively) was obtained in the case of logistic analysis.

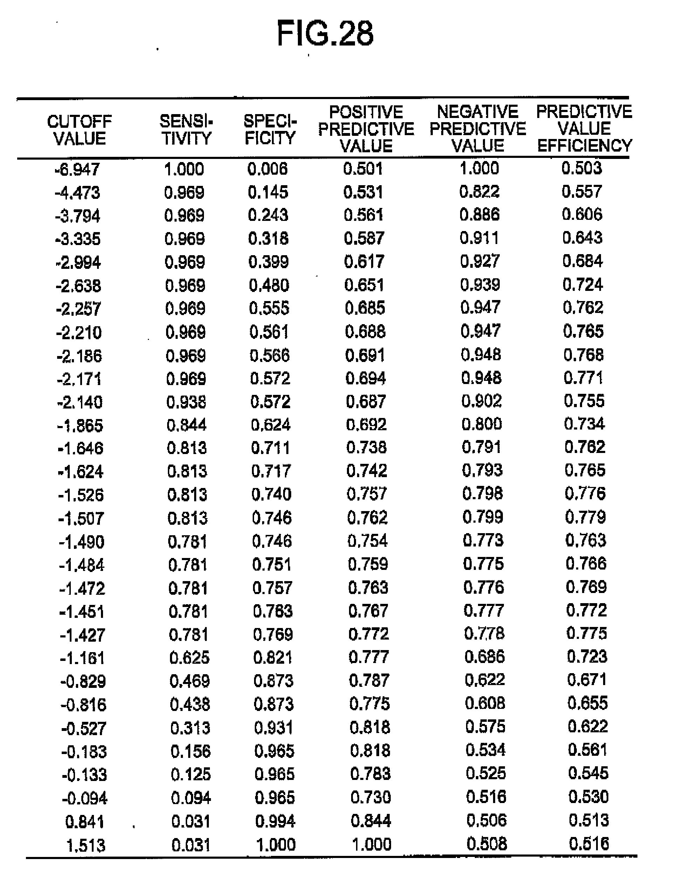

Discrimination of the 2 groups by the index 4 was evaluated by the AUC of the ROC curve (see FIG. 27), to give an AUC of 0.823±0.036 (95% confidence interval: 0.753 to 0.893), and the index 4 was revealed to be a useful index with high diagnostic performance. When the optimum cutoff value for discrimination of the 2 groups by the index 4 (the cutoff value was obtained for a variable obtained by logit transformation of probability obtained from logistic analysis) was determined assuming that the incidence of metabolic syndrome was 0.5, the cutoff value was -1.507, and the sensitivity was 81%; the specificity, 75%; the positive predictive value, 76%; the negative predictive value, 80%, and the correct diagnostic rate, 78% (FIG. 28), and the index 4 was revealed to be an useful index with high diagnostic performance.

When the same data as described above were used, and as another example of the index 4 containing Glu, Gly, Ala, Thr and Ser, the multivariate discriminant by a linear discriminant, a support vector machine and a Mahalanobis' generalized distance method was evaluated, the AUC of the ROC curve in the liner discriminant was 0.819±0.035 (95% confidence interval: 0.750 to 0.889), the error ratio in the support vector machine was 15.6%, the error ratio in the Mahalanobis' generalized distance method was 24.4%, and the index 4 was revealed to be an useful index with high diagnostic performance.

### Example 4

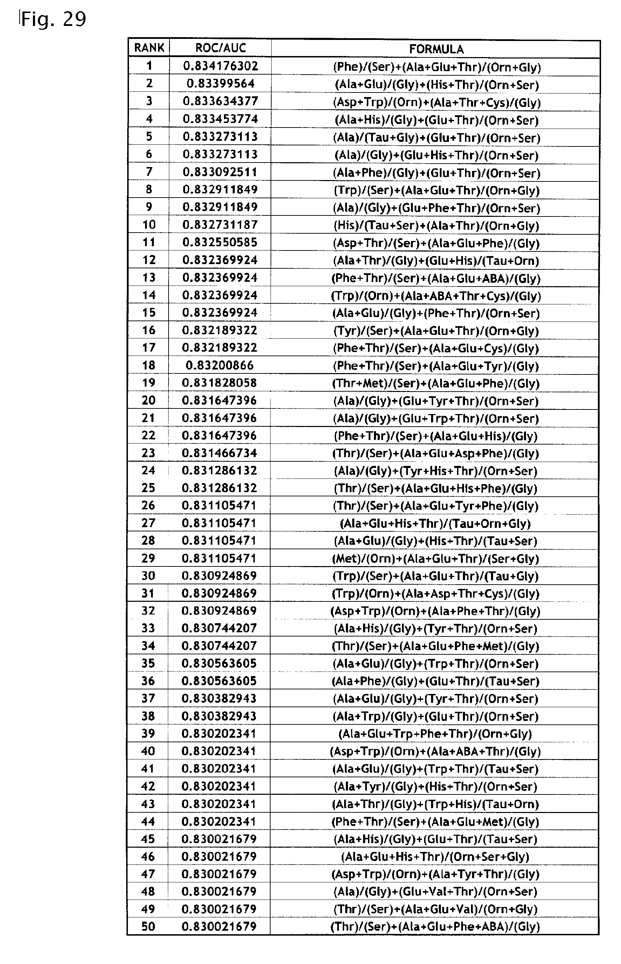

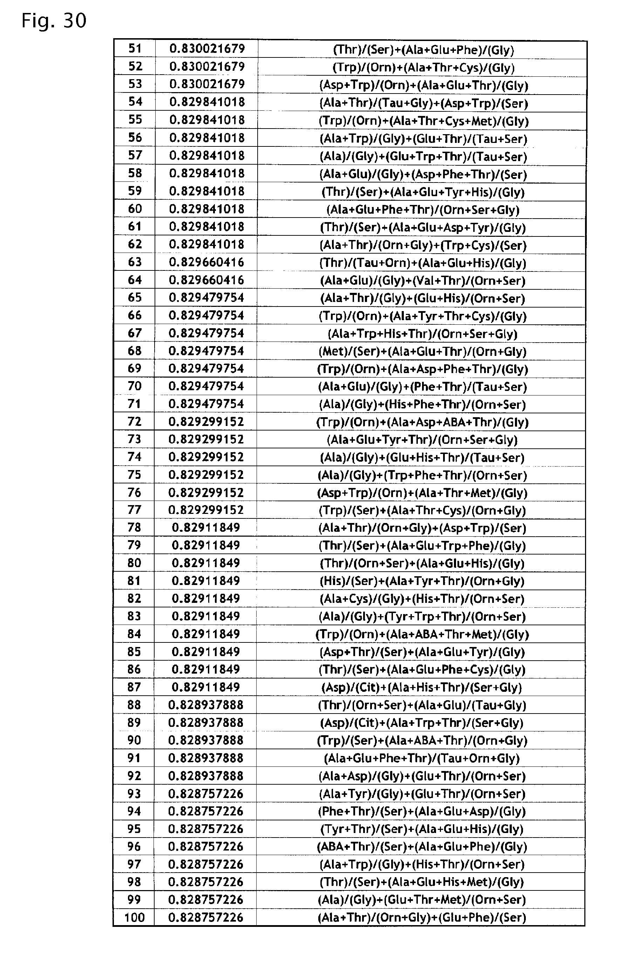

The sample data used in Example 1 were used in Example 4. Using a method described in International Publication WO 2004/052191 that is an international application filed by the present applicant, an index for maximizing the performance of discriminating the 2 groups of non-metabolic syndrome and metabolic syndrome was extensively searched to give a plurality of indices having similar performance. A list of AUCs of ROC curves for diagnostic performance of discriminating the 2 groups by the indices is shown in FIGS. 29 and 30.

### Example 5

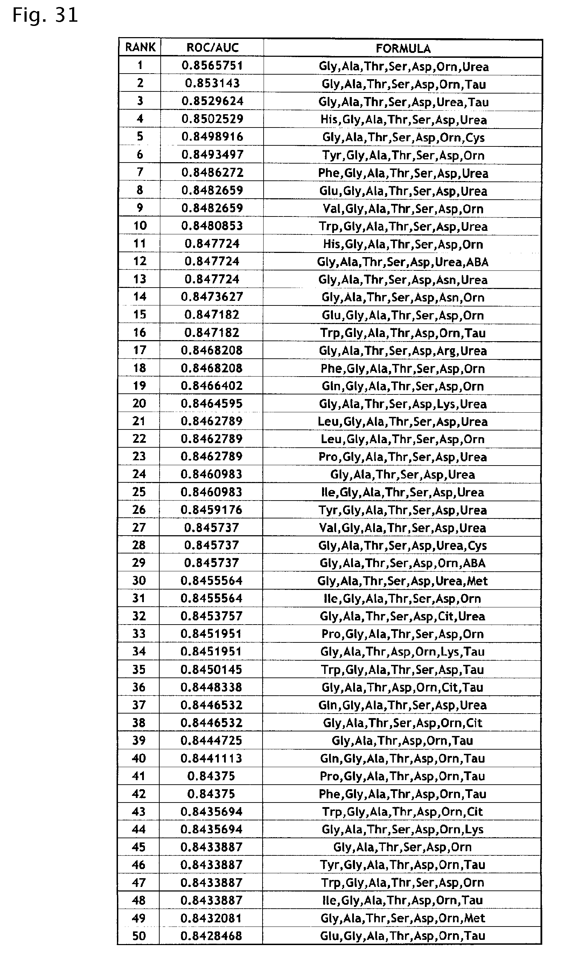

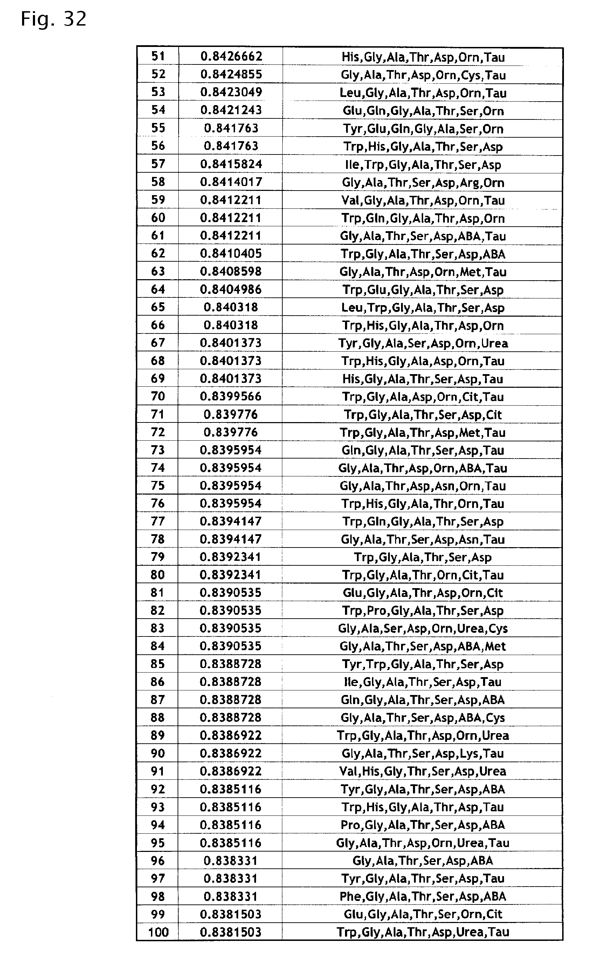

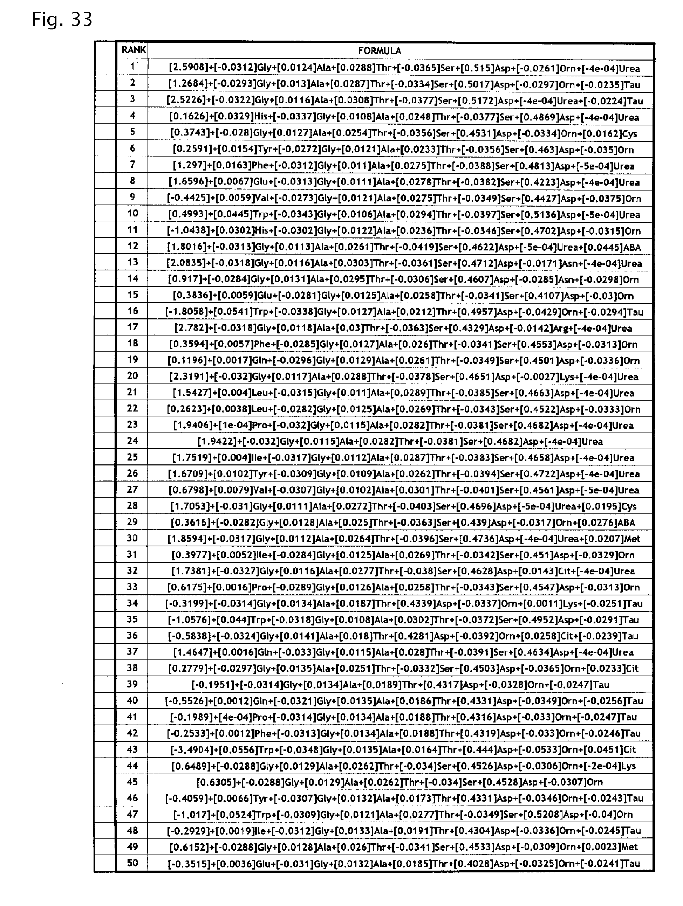

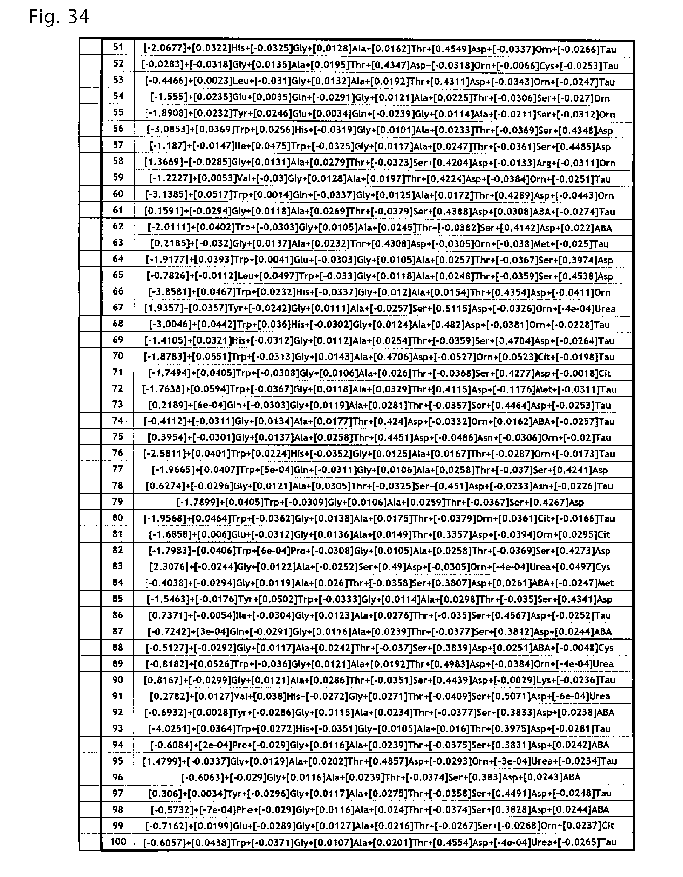

The sample data used in Example 1 were used in Example 5. Using method (a method of searching a multivariate discriminant) described in International Publication PCT/JP2006/304398 that is an international application filed by the present applicant, an index for maximizing the performance of discriminating the 2 groups of non-metabolic syndrome and metabolic syndrome was searched by logistic analysis, to give a plurality of indices having similar performance. A list of AUCs of ROC curves for diagnostic performance of discriminating the 2 groups by the indices is shown in FIGS. 31 and 32. Specific examples of the indices in each rank in FIGS. 31 and 32 are shown in FIGS. 33 and 34.

### Example 6

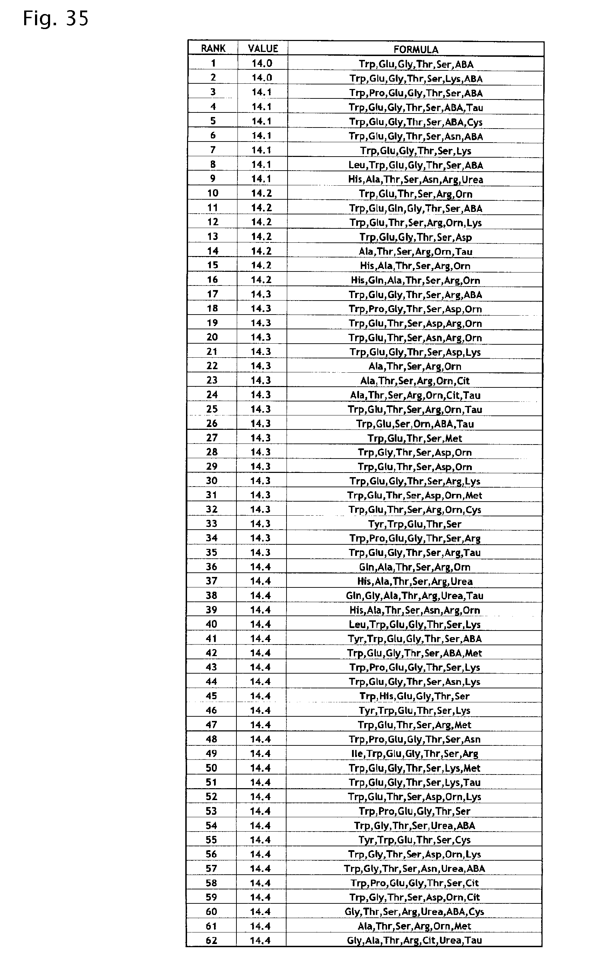

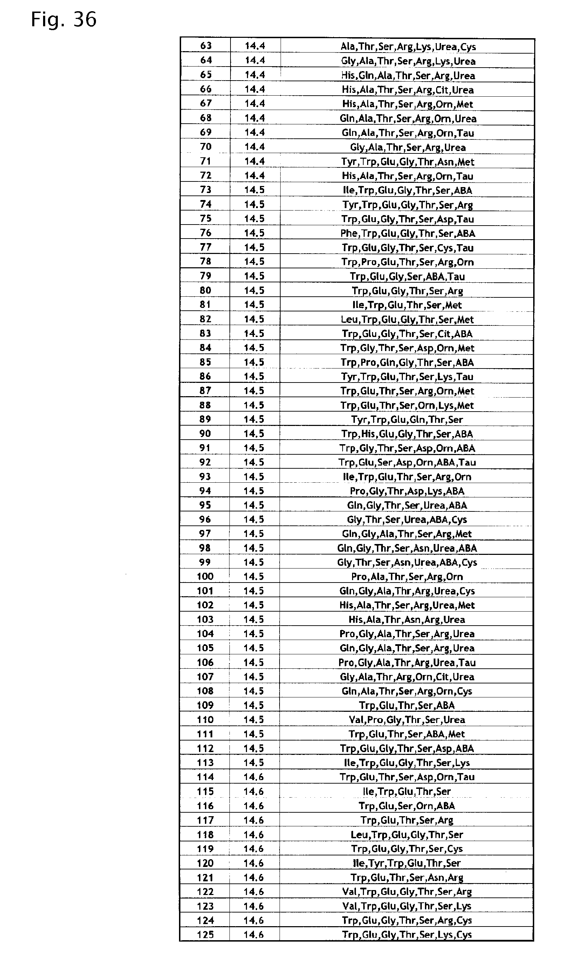

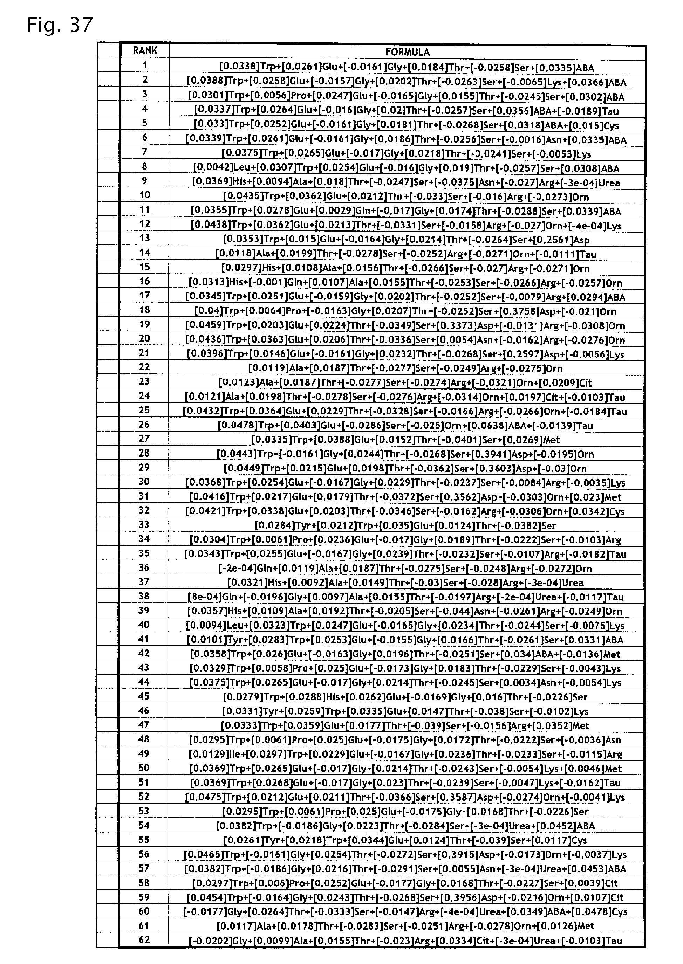

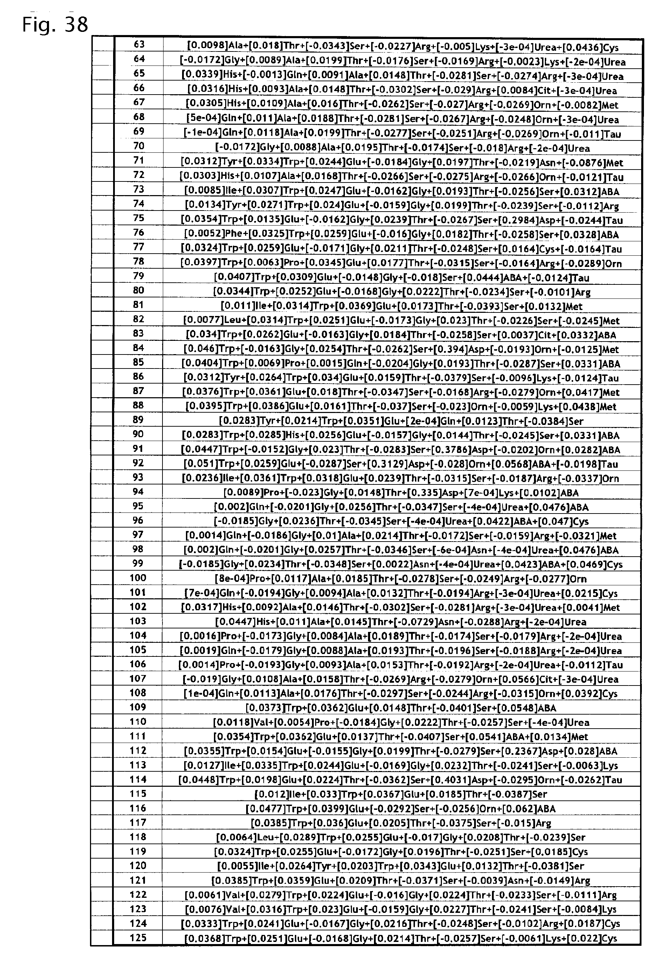

The sample data used in Example 1 were used in Example 6. Using method (a method of searching a (multivariate discriminant) described in International Publication PCT/JP2006/304398 that is an international application filed by the present applicant, an index for maximizing the performance of discriminating the 2 groups of non-metabolic syndrome and metabolic syndrome was searched by linear discrimination, to give a plurality of indices having similar performance. A list of error rates (%) in diagnostic performance of discriminating the 2 groups by the indices is shown in FIGS. 35 and 36. Specific examples of the indices in each rank in FIGS. 35 and 36 are shown in FIGS. 37 and 38.

### INDUSTRIAL APPLICABILITY

As described above, the method of evaluating metabolic syndrome, the metabolic syndrome-evaluating apparatus, metabolic syndrome-evaluating method, the metabolic syndrome-evaluating system, the metabolic syndrome-evaluating program, the recording medium, and the method of searching for prophylactic/ameliorating substance for metabolic syndrome according to the present invention can be practiced widely in many industrial fields, in particular in pharmaceutical, food, and medical fields, and are extremely useful in the field of bioinformatics, for example, performing disease state prediction, disease risk prediction, and proteome or metabolome analysis.

## Claims

1. A method of evaluating metabolic syndrome, comprising:
a measuring step of measuring amino acid concentration data on the concentration value of amino acid in blood collected from a subject to be evaluated, and
a concentration value criterion evaluating step of evaluating the state of metabolic syndrome in the subject, based on the amino acid concentration data of the subject measured at the measuring step.

2. The method of evaluating metabolic syndrome according to claim 1, wherein the concentration value criterion evaluating step includes evaluating the state of metabolic syndrome in the subject, based on the concentration value of at least one of Val, Leu, Ile, Tyr, Trp, Glu, Ala, Asp, Gly, Ser and Thr contained in the amino acid concentration data of the subject measured at the measuring step.

3. The method of evaluating metabolic syndrome according to claim 2, wherein the concentration value criterion evaluating step further includes a concentration value criterion discriminating step of discriminating between metabolic syndrome and non-metabolic syndrome in the subject, based on the concentration value of at least one of Val, Leu, Ile, Tyr, Trp, Glu, Ala, Asp, Gly, Ser and Thr contained in the amino acid concentration data of the subject measured at the measuring step.

4. The method of evaluating metabolic syndrome according to claim 3, wherein the concentration value criterion discriminating step includes discriminating between metabolic syndrome and non-metabolic syndrome in the subject, based on the concentration values of at least two of Val, Leu, Ile, Tyr, Trp, Glu, Ala, Asp, Gly, Ser and Thr contained in the amino acid concentration data of the subject measured at the measuring step.

5. The method of evaluating metabolic syndrome according to claim 1, wherein the concentration value criterion evaluating step further includes:
a discriminant value calculating step of calculating a discriminant value that is a value of multivariate discriminant, based on both the amino acid concentration data of the subject measured at the measuring step and a previously established multivariate discriminant with the concentration of the amino acid as a variable, and
a discriminant value criterion evaluating step of evaluating the state of metabolic syndrome in the subject, based on the discriminant value calculated at the discriminant value calculating step.

6. The method of evaluating metabolic syndrome according to claim 5, wherein the multivariate discriminant contains at least one of Val, Leu, Ile, Tyr, Trp, Glu, Ala, Asp, Gly, Ser and Thr as the variable, and
the discriminant value calculating step includes calculating the discriminant value, based on both the concentration value of at least one of Val, Leu, Ile, Tyr, Trp, Glu, Ala, Asp, Gly, Ser and Thr contained in the amino acid concentration data of the subject measured at the measuring step and the multivariate discriminant.

7. The method of evaluating metabolic syndrome according to claim 6, wherein the discriminant value criterion evaluating step further includes a discriminant value criterion discriminating step of discriminating between metabolic syndrome and non-metabolic syndrome in the subject, based on the discriminant value calculated at the discriminant value calculating step.

8. The method of evaluating metabolic syndrome according to claim 7, wherein the multivariate discriminant includes at least two of Val, Leu, Ile, Tyr, Trp, Glu, Ala, Asp, Gly, Ser and Thr as the variables, and
the discriminant value calculating step includes calculating the discriminant value, based on both the concentration values of at least two of Val, Leu, Ile, Tyr, Trp, Glu, Ala, Asp, Gly, Ser and Thr contained in the amino acid concentration data of the subject measured at the measuring step and the multivariate discriminant.

9. The method of evaluating metabolic syndrome according to claim 8, wherein the multivariate discriminant is expressed by one fractional expression or the sum of a plurality of the fractional expressions and contains either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant.

10. The method of evaluating metabolic syndrome according to claim 9, wherein the multivariate discriminant is formula 1:
Thr/Ser+(Glu+Ala)/Gly (formula 1)

11. The method of evaluating metabolic syndrome according to claim 8, wherein the multivariate discriminant is any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree.

12. The method of evaluating metabolic syndrome according to claim 11, wherein the multivariate discriminant contains Glu, Gly, Ala, Thr and Ser as the variables.

13. A metabolic syndrome-evaluating apparatus comprising a control unit and a memory unit to evaluate the state of metabolic syndrome in a subject to be evaluated, wherein the control unit includes:
a discriminant value-calculating unit that calculates a discriminant value that is a value of multivariate discriminant, based on both previously obtained amino acid concentration data on the concentration value of amino acid in the subject and a multivariate discriminant with the concentration of the amino acid as variable stored in the memory unit; and
a discriminant value criterion-evaluating unit that evaluates the state of metabolic syndrome in the subject, based on the discriminant value calculated by the discriminant value-calculating unit.

14. The metabolic syndrome-evaluating apparatus according to claim 13, wherein the multivariate discriminant contains at least one of Val, Leu, Ile, Tyr, Trp, Glu, Ala, Asp, Gly, Ser and Thr as the variable, and
the discriminant value-calculating unit calculates the discriminant value, based on both the concentration value of at least one of Val, Leu, Ile, Tyr, Trp, Glu, Ala, Asp, Gly, Ser and Thr contained in previously obtained amino acid concentration data of the subject and the multivariate discriminant.

15. The metabolic syndrome-evaluating apparatus according to claim 14, wherein the discriminant value criterion-evaluating unit further includes a discriminant value criterion-discriminating unit that discriminates between metabolic syndrome and non-metabolic syndrome in the subject, based on the discriminant value calculated by the discriminant value-calculating unit.

16. The metabolic syndrome-evaluating apparatus according to claim 15, wherein the multivariate discriminant includes at least two of Val, Leu, Ile, Tyr, Trp, Glu, Ala, Asp, Gly, Ser and Thr as the variables, and
the discriminant value-calculating unit calculates the discriminant value, based on both the concentration values of at least two of Val, Leu, Ile, Tyr, Trp, Glu, Ala, Asp, Gly, Ser and Thr contained in the previously obtained amino acid concentration data of the subject and the multivariate discriminant.

17. The metabolic syndrome-evaluating apparatus according to claim 16, wherein the Multivariate discriminant is expressed by one fractional expression or the sum of a plurality of the fractional expressions and contains either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant.

18. The metabolic syndrome-evaluating apparatus according to claim 17, wherein the multivariate discriminant is formula 1:
Thr/Ser+(Glu+Ala)/Gly (formula 1)

19. The metabolic syndrome-evaluating apparatus according to claim 16, wherein the multivariate discriminant is any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree.

20. The metabolic syndrome-evaluating apparatus according to claim 19, wherein the multivariate discriminant contains Glu, Gly, Ala, Thr and Ser as the variables.

21. The metabolic syndrome-evaluating apparatus according to any one of claims 13 to 20, wherein the control unit further includes a multivariate discriminant-preparing unit that prepares the multivariate discriminant to be stored in the memory unit, based on metabolic syndrome state information containing the amino acid concentration data and metabolic syndrome state index data on an index for indicating the state of metabolic syndrome, stored in the memory unit,
the multivariate discriminant-preparing unit further includes:
a candidate multivariate discriminant-preparing unit that prepares a candidate multivariate discriminant that is a candidate of the multivariate discriminant, based on a predetermined discriminant-preparing method from the metabolic syndrome state information;
a candidate multivariate discriminant-verifying unit that verifies the candidate multivariate discriminant prepared by the candidate multivariate discriminant-preparing unit, based on a predetermined verifying method; and
a variable-selecting unit that selects a variable of the candidate multivariate discriminant based on a predetermined variable-selecting method from the verification result obtained by the candidate multivariate discriminant-verifying unit, thereby selecting a combination of the amino acid concentration data contained in the metabolic syndrome state information used in preparing the candidate multivariate discriminant, and
the multivariate discriminant-preparing unit prepares the multivariate discriminant by selecting the candidate multivariate discriminant used as the multivariate discriminant, from a plurality of the candidate multivariate discriminants, based on the verification results accumulated by repeatedly executing the candidate multivariate discriminant-preparing unit, the candidate multivariate discriminant-verifying unit and the variable-selecting unit.

22. A metabolic syndrome-evaluating method of evaluating the state of metabolic syndrome in a subject to be evaluated which is carried out with an information processing apparatus including a control unit and a memory unit, the method comprising:
a discriminant value calculating step of calculating a discriminant value that is a value of multivariate discriminant, based on both previously obtained amino acid concentration data on the concentration value of amino acid in the subject and a multivariate discriminant with the concentration of the amino acid as variable stored in the memory unit; and
a discriminant value criterion evaluating step of evaluating the state of metabolic syndrome in the subject, based on the discriminant value calculated at the discriminant value calculating step,
that are executed by the control unit.

23. The metabolic syndrome-evaluating method according to claim 22, wherein the multivariate discriminant contains at least one of Val, Leu, Ile, Tyr, Trp, Glu, Ala, Asp, Gly, Ser and Thr as the variable, and
the discriminant value calculating step includes calculating the discriminant value, based on both the concentration value of at least one of Val, Leu, Ile, Tyr, Trp, Glu, Ala, Asp, Gly, Ser and Thr contained in the previously obtained amino acid concentration data of the subject and the multivariate discriminant.

24. The metabolic syndrome-evaluating method according to claim 23, wherein the discriminant value criterion evaluating step further includes a discriminant value criterion discriminating step of discriminating between metabolic syndrome and non-metabolic syndrome in the subject, based on the discriminant value calculated at the discriminant value calculating step.

25. The metabolic syndrome-evaluating method according to claim 24, wherein the multivariate discriminant includes at least two of Val, Leu, Ile, Tyr, Trp, Glu, Ala, Asp, Gly, Ser and Thr as the variables, and
the discriminant value calculating step includes calculating the discriminant value, based on both the concentration values of at least two of Val, Leu, Ile, Tyr, Trp, Glu, Ala, Asp, Gly, Ser and Thr contained in the previously obtained amino acid concentration data of the subject and the multivariate discriminant.

26. The metabolic syndrome-evaluating method according to claim 25, wherein the multivariate discriminant is expressed by one fractional expression or the sum of a plurality of the fractional expressions and contains either at least one of Val, Leu, Ile, Tyr, Trp, Glu, Ala, Asp and Thr as the variable in the numerator and at least one of Gly and Ser as the variable in the denominator or at least one of Gly and Ser as the variable in the numerator and at least one of Val, Leu, Ile, Tyr, Trp, Glu, Ala, Asp and Thr as the variable in the denominator, in the fractional expression constituting the multivariate discriminant.

27. The metabolic syndrome-evaluating method according to claim 26, wherein the multivariate discriminant is formula 1:
Thr/Ser+(Glu+Ala)/Gly (formula 1)

28. The metabolic syndrome-evaluating method according to claim 25, wherein the multivariate discriminant is any one of a logistic regression equation, a linear discriminant, a multiple regression equation, a formula prepared by a support vector machine, a formula prepared by a Mahalanobis' generalized distance method, a formula prepared by canonical discriminant analysis, and a formula prepared by a decision tree.

29. The metabolic syndrome-evaluating method according to claim 28, wherein the multivariate discriminant contains Glu, Gly, Ala, Thr and Ser as the variables.

30. The metabolic syndrome-evaluating method according to any one of claims 22 to 29, wherein the method further includes a multivariate discriminant preparing step of preparing the multivariate discriminant to be stored in the memory unit, based on metabolic syndrome state information containing the amino acid concentration data and metabolic syndrome state index data on an index for indicating the state of metabolic syndrome, stored in the memory unit that is executed by the control unit,
the multivariate discriminant preparing step further includes:
a candidate Multivariate discriminant preparing step of preparing a candidate multivariate discriminant that is a candidate of the multivariate discriminant, based on a predetermined discriminant-preparing method from the metabolic syndrome state information;
a candidate multivariate discriminant verifying step of verifying the candidate multivariate discriminant prepared at the candidate multivariate discriminant preparing step, based on a predetermined verifying method; and
a variable selecting step of selecting variable of the candidate multivariate discriminant based on a predetermined variable-selecting method from the verification result obtained at the candidate multivariate discriminant verifying step, thereby selecting a combination of the amino acid concentration data contained in the metabolic syndrome state information used in preparing the candidate multivariate discriminant, and
at the multivariate discriminant preparing step, the multivariate discriminant is prepared by selecting the candidate multivariate discriminant used as the multivariate discriminant, from a plurality of the candidate multivariate discriminants, based on the verification results accumulated by repeatedly executing the candidate multivariate discriminant preparing step, the candidate multivariate discriminant verifying step and the variable selecting step.

31. A metabolic syndrome-evaluating system comprising a metabolic syndrome-evaluating apparatus including a control unit and a memory unit to evaluate the state of metabolic syndrome in a subject to be evaluated and an information communication terminal apparatus that provides amino acid concentration data on the concentration value of amino acid in the subject that are connected to each other communicatively via a network, wherein
the information communication terminal apparatus includes:
an amino acid concentration data-sending unit that transmits the amino acid concentration data of the subject to the metabolic syndrome-evaluating apparatus; and
an evaluation result-receiving unit that receives the evaluation result of the state of metabolic syndrome of the subject transmitted from the metabolic syndrome-evaluating apparatus, and
the control unit of the metabolic syndrome-evaluating apparatus includes:
an amino acid concentration data-receiving unit that receives the amino acid concentration data of the subject transmitted from the information communication terminal apparatus;
a discriminant value-calculating unit that calculates a discriminant value that is a value of multivariate discriminant, based on both the amino acid concentration data of the subject received by the amino acid concentration data-receiving unit and a multivariate discriminant with the concentration of the amino acid as variable stored in the memory unit;
a discriminant value criterion-evaluating unit that evaluates the state of metabolic syndrome in the subject, based on the discriminant value calculated by the discriminant value-calculating unit; and
an evaluation result-sending unit that transmits the evaluation result of the subject obtained by the discriminant value criterion-evaluating unit to the information communication terminal apparatus.

32. A metabolic syndrome-evaluating program for evaluating the state of metabolic syndrome in a subject to be evaluated, that makes an information processing apparatus including a control unit and a memory unit execute:
a discriminant value calculating step of calculating a discriminant value that is a value of multivariate discriminant, based on both previously obtained amino acid concentration data on the concentration value of amino acid in the subject and a multivariate discriminant with the concentration of the amino acid as variable stored in the memory unit; and
a discriminant value criterion evaluating step of evaluating the state of metabolic syndrome in the subject, based on the discriminant value calculated at the discriminant value calculating step,
that are executed by the control unit.

33. A computer-readable recording medium, comprising the metabolic syndrome-evaluating program according to claim 33 recorded thereon.

34. A method of searching for prophylactic/ameliorating substance for metabolic syndrome, comprising:
a measuring step of measuring amino acid concentration data on the concentration value of amino acid in blood collected from a subject to be evaluated to which a desired substance group consisting of one or more substances has been administered;
a concentration value criterion evaluating step of evaluating the state of metabolic syndrome in the subject, based on the amino acid concentration data measured at the measuring step; and
a judging step of judging whether the desired substance group prevents or ameliorates metabolic syndrome, based on the evaluation result at the concentration value criterion evaluating step.
